# EUROPEAN PATENT SPECIFICATION

(11) **EP 4 605 043 B1**
(45) Date of publication and mention of the grant of the patent: **14.10.2026**
(21) Application number: 24703211.3
(22) Date of filing: 05.02.2024
(51) Int. Cl.: A61M 11/00, A61M 31/00, A61M 3/00

(54) **DISCHARGER WITH IMPROVED PIERCING TIP**
AUSTRAGER MIT VERBESSERTER DURCHSTECHSPITZE
DISPOSITIF D'ÉCOULEMENT À POINTE DE PERFORATION AMÉLIORÉE

(30) Priority: 10.02.2023 EP 23155965
(43) Date of publication of application: 27.08.2025
(73) Proprietor: medmix Switzerland AG, 9469 Haag (Rheintal) (CH)
(72) Inventor: NIEBER, Benjamin, 9469 Haag (Rheintal) (CH)
(74) Representative: Manitz Finsterwald Patent- und Rechtsanwaltspartnerschaft mbB
(86) International application number: PCT/EP2024/052778
(87) International publication number: WO 2024/165494

(56) References cited:
- WO-A1-2021/245605
- US-A1- 2014 060 523

## Description

The present disclosure relates to a discharger for discharging a predetermined amount of fluid and to a discharge system comprising such a discharger, as well as to corresponding methods of using such dischargers. The present disclosure further relates to the use of the discharger and the discharge system for discharging a fluid including at least one medical, dental or veterinary agent.

Dischargers such as expendable syringes for use in the medical, dental or veterinary field are generally known which comprise one or more compartments in which the medical, dental or veterinary agent or a liquid including the medical, dental or veterinary agent is contained before use. The compartments are normally sealed with various kinds of barrier foils in order to isolate its contents from the environment.

Typically these dispensers are used to treat symptoms of a patient.

It is an object of the present invention to make available a dispenser by means of which symptoms can not only be treated, but through whose use an infection can be avoided and preferably be prevented.

It is a further object of the invention to make available a low cost and simple to use dispenser.

Patent documents D1 US2014/0060523 and D2 WO2021/245605 are hereby acknowledged..

These objects are satisfied by a discharger having the features of the respective independent claim. A method for discharging a fluid from a discharger is also claimed.

Such a discharger is configured to apply a fluid at and/or in a nasal cavity of a patient, wherein the discharger is filled with a fluid, with the fluid comprising lipid nanoparticles.

The technology further relates to a use of a discharger, the discharger being configured to apply a fluid at a nasal cavity of a patient, wherein the discharger is filled with a fluid, with the fluid comprising lipid nanoparticles, and the fluid is applied at and/or in the nasal cavity.

Through the use of such a dispenser a radical improvement in prophylactic and therapeutic protection against infections of humans, such as by influenza (flu) or cold viruses is made available.

Thereby a first-in-class highly potent immunotherapy relying on optimised antibodies contained by newly developed lipid nanoparticles (LNPs) and delivered at the intranasal cavity is made possible leading to a reduced number of infections with viral pathogens.

Such a discharger comprises a dispensing element and a housing, the housing having a static piston connected to the dispensing element at least in a fluid conducting manner and with the static piston being arranged within the housing; the discharger further comprising a container containing a fluid, the container being moveable relative to the static piston and the housing, with the static piston comprising a piercing tip adapted to pierce a membrane or seal of the container when the container is moved towards the housing, with the static piston further comprising at least one sealing element provided in the region of the piercing tip, with the at least one sealing element providing a seal between the piercing tip and an inner surface of the container when the moveable container is moved relative to the housing into a position in which the membrane or seal is pierced and in which the at least one sealing element engages the inner surface to prevent the fluid from passing between the at least one sealing element and the inner surface and into a part of the housing.

The at least one sealing element thereby provides a direct seal between the static piston and the container ensuring that no fluid can pass between the at least one sealing element and the inner surface of the container and thereby preferably prevent the fluid from leaking from the container into a part of the housing that is not configured to receive a fluid.

In contrast to designs where the housing is sealed off to the outside the present invention thus directly seals between the parts of the discharger moveable relative to one another where the presence of a leak is most likely.

Moreover, by providing a seal that engages the inner wall of the container, the container is more accurately aligned relative to the housing and hence with respect to the discharger as the seal acts as a further guide for the container. Such an assembly can thus advantageously also compensate an incorrect mounting of the container at the discharger.

Preferably the at least one sealing element is arranged at an end of the piercing tip remote from the other end of the piercing tip comprising an inlet of the dispensing element. On entraining the container towards the static piston the membrane or seal of the container then comes into contact with that end of the piercing tip having an inlet for a fluid to be dispensed from the container via the dispensing element first.

Advantageously the membrane or seal is configured to be pierced by said other end of the piercing tip comprising the inlet. Preferably the membrane or seal is then first pierced in the region of the inlet so that the then pressurized fluid stored in the container can pass directly into the inlet and thereby into the dispensing element.

Particularly preferably the at least one sealing element is a sealing lip. A sealing lip is simple and cost effective to manufacture and ensures a good seal between two components moveable relative to one another.

It is preferred if first, second and preferably third sealing lips are provided that are all configured to engage the inner surface to prevent the fluid from passing into said part of the housing. Advantageously the first, second and preferably third sealing lips are arranged one after the other in parallel to one another along a longitudinal axis of the discharger.

Providing at least two sealing lips that are arranged to, on the one hand, engage the inner surface increases a sealing action in this region of the discharger. On the other hand, such a double seal arrangement that is arranged one after the other and in parallel to one another further improves the guidance of the container relative to the housing, further correcting any initially present misalignment between the housing and the container.

Preferably the first sealing element has a diameter that is smaller than a diameter of a second sealing element, and if a third sealing element is provided then the first sealing element has a diameter that is smaller than a diameter of the third sealing element, with the second and third sealing elements preferably having an at least substantially identical diameter. Forming the first sealing element, such that this has a diameter that is smaller than the diameter of a second or even a third sealing element, means that the first sealing element can push the membrane or seal into the compartment following its piercing and towards the inner surface to clear the way for the subsequent sealing elements that then fully engage the inner surface of the container.

In this connection it is advantageous if an external diameter of the first sealing element is less than or equal to an internal diameter of the container and an external diameter of the second sealing element and optionally of the third sealing element is larger than the internal diameter of the container. This facilitates the introduction of the static piston into the container during the discharging process and ensures an improved seal between the inner surface of the container and the second and third sealing elements.

It is preferred when a diameter of the piercing tip reduces from the at least one sealing element to said other end of the piercing tip comprising the inlet. If the container is misaligned relative to the housing then the shape on the piercing tip can allow for a correction of the misalignment, on the one hand. On the other hand the shape of the piercing tip provides a possibility of exerting a, preferably uniform, pressure on the membrane or seal so that this is pierced, preferably centrally pierced, by the piercing tip. A central piercing of the membrane or seal ensures that the seal is pierced in the region of the inlet. This further avoids fluid from running down the piercing tip and towards the at least one sealing element.

In this connection it should be noted that it is advantageous when the compartment comprises an end, in particular a piercing tip receiving end, that is disposed opposite to the membrane or seal and that is configured complementary or at least substantially complementary to the shape of the piercing tip. Once the piercing tip has travelled completely through a space of the compartment and reaches the end of the compartment it is thereby ensured that as much of the volume as possible of the fluid stored in the compartment has been discharged via the dispensing element in the discharged state of the discharger.

Preferably the container comprises a compartment in its interior in which the fluid is initially stored prior to piercing the membrane or seal, and wherein the inner surface is present at a wall of the compartment. In this way the at least one sealing element provides a direct seal in the region of the components of the discharger that are moveable relative to one another and therefor susceptible to leaks.

Advantageously the container further comprises an outer wall arranged in parallel to the wall of the compartment and a guide groove present between the outer wall and the wall of the compartment, with said guide groove receiving at least some of the housing. Such a, preferably annular, groove ensures that the container can be aligned correctly relative to the housing on assembly of the discharger avoiding misalignments of these parts on assembly of the discharger.

It is preferred if the discharger comprises a child safety lock, in particular wherein the child safety lock is formed between the container and the housing. Such child safety locks prevent the accidental activation of the discharger.

Advantageously the at least one sealing element does not contact the housing in a storage state of the discharger and with the at least one sealing element only contacting the inner surface of the container in a discharging state or discharged state of the discharger. In this way a discharger is formed in which no forces act on the at least one sealing element in the storage state of the discharger. Such forces could lead to a deterioration over time of the at least one sealing element and hence to a reduced sealing action in this region on use of the discharger.

Preferably an inner surface of a cylindrical wall of the housing comprises a further inwardly projecting sealing lip that is configured to engage a surface of the container different from the inner surface. In the event that fluid does pass between the at least one sealing element and the inner surface of the compartment into a part of the housing, this further sealing element can prevent the fluid from exiting between the housing and the container, where a user has his/her hands during discharging. This means that, at least during discharging, a user does not come into direct contact with the fluid present in the discharger.

Advantageously the discharger is filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

In this way, for example, bleaching agents for teeth, fluoride for teeth, disinfectants, adhesives, wound adhesives, topical anesthetics, sunscreen, after sun, skin moisturizers or other drugs and cosmetics can be stored in the discharger and administered using the discharger. Such fluids can e.g. be the aforementioned frontline, eye drops and nose drops used to e.g. decongest eyes and noses of patients suffering from allergies, colds or flues.

In a further aspect the present technology relates to a static piston for a discharger, with the static piston preferably being configured for the discharger discussed in the foregoing, wherein the static piston comprises a piercing tip having an inlet formed at an end thereof and a passage that starts at the inlet and that passes through the piercing tip as well as through the static piston and up to an outlet, with the static piston comprising at least one sealing element formed at an end of the piercing tip remote from the inlet.

The advantages associated with the discharger in accordance with the invention likewise hold true for the static piston described herein.

In vet a further aspect the present technology relates to a method of discharging a fluid from a discharger, in particular in accordance with a discharger discussed in the foregoing, the method comprising the steps of:
- guiding a container in a direction along a longitudinal axis of the discharger towards a piercing tip of a static piston accommodated within a housing of the discharger,
- piercing, preferably uniformly piercing, a membrane or seal present at the container by means of the piercing tip causing the membrane or seal to be pierced, in particular initially at a center of the membrane or seal,
- guiding the container further towards the static piston and bringing an inner surface of the container into engagement with at least one sealing element arranged at the piercing tip of the static piston to prevent the fluid from passing between the at least one sealing element and the inner surface and thereby into a part of the housing, and
- discharging the fluid via an outlet of the discharger.

The advantages associated with the discharger in accordance with the invention likewise hold true for the method described herein.

Such a container for a manually operated discharger comprises a compartment for a fluid and a longitudinal axis, with the compartment being configured to be sealed off, on the one hand, by at least one wall of the compartment, said at least one wall at least regionally peripherally extending around the longitudinal axis and, on the other hand, by a seal at a front end of the container, wherein the container is a double walled container which in addition to the wall of the compartment further comprises an outer wall extending around the wall of the compartment, with a groove being formed between the outer wall and the wall of the compartment and with at least one connection web, preferably fixedly, connecting the outer wall to the wall of the compartment.

The groove is provided at the double walled container as a mounting guide. Thereby a skew mounting of the container at a housing of a discharger can be avoided. Moreover, in use of the discharger the groove acts as a guide groove. This is because the housing can be guided in the groove relative to the container, when the container is mounted at a discharger.

In this way one can ensure that the container is reliably guided relative to a piercing tip arranged therein. It has namely been found that if the container is guided in an as uniform manner as possible with respect to the housing of a discharger, then a significant reduction and possibly also a prevention of liquids leaking from the container into a part of the housing or to the outside of the housing can be achieved.

Such a double walled container is made available in a cost effective manner by providing an outer wall that surrounds the wall of the compartment and connecting these two walls by means of at least one connecting web.

The seal of the container is preferably configured to be pierced in use of the container. Forming the seal such that it can be pierced provides the container with a pre-defined outlet via which fluids stored in the compartment can be removed from the container.

Preferably the wall of the compartment and the outer wall of the, preferably cylindrically shaped, container are injection molded in one piece from the same material. Using an injection molding process makes available a reproducible cost effective mode of manufacture for parts of plastic. As the connection web is preferably also formed during the injection molding process this is automatically integrally formed with and thereby fixedly connected to the wall of the compartment and to the outer wall of the container.

Advantageously a length of the groove is at least 50%, preferably 70% to 95%, of a length of the outer wall in the direction of the longitudinal axis of the container. In this way the groove of the container is configured to receive a substantial portion of the housing, thereby ensuring a guidance of the container with respect to the substantial portion of the housing.

It should be noted that the groove is open at the front end. In this way the components of the container that are configured to interact with the housing are provided at one side of the container.

Preferably the front end coincides with a plane, said plane comprising at least one of an end of the wall of the compartment, an end of the outer wall, the seal and an opening of said groove. This means that the components of the container forming the front end are all arranged at substantially the same height of the container.

Advantageously a rear end of the container comprises a recess formed therein, preferably wherein the recess is configured for placement of a finger or thumb of a user. Thereby the rear end is configured for an improved placement of the thumb or a finger of a user using a discharger having such a container.

It is preferred if an end of the compartment oppositely disposed of the front end is formed by a further wall of the compartment, with the further wall converging from the wall of the compartment to a common point forming the end of the compartment, said common point preferably coinciding with the longitudinal axis.

By forming the end of the compartment such that it converges to the common point means that it is configured complementary or at least substantially complementary to the shape of a piercing tip of a discharger. On use of the discharger the piercing tip travels completely through a space of the compartment and when it reaches the end of the compartment it is ensured that as much of the volume as possible of the fluid stored in the compartment has been discharged from the discharger.

Advantageously the end of the compartment is set back from the rear end of the container by at least 20%, in particular 25 to 45%, of the length of the outer wall. On injection molding the container such a design has advantages with respect to the manufacturing tolerances, as less filigree parts need to be made available at the rear end of the container than at the front end of the container.

It is preferred if a further web is provided that extends in parallel to the outer wall between the end of the compartment and the rear end of the container, with the further web in particular forming a base of the recess. By means of this further web a pressure exerted on the rear end of the container can be translated into a movement in the direction of the longitudinal axis of the container when mounted at a housing of the discharger. In this connection it should be noted that in the mounted state of the container at a housing of a discharger the longitudinal axis of the container coincides with the longitudinal axis of the discharger.

Advantageously the wall of the compartment extends beyond the end of the compartment in parallel to the outer wall. On injection molding the container such a design has advantages with respect to the manufacturing tolerances, as less filigree parts need to be made available at the rear end of the container.

Preferably at least one slot is formed in the outer wall, wherein the slot extends from the groove to an outer surface of the outer wall. If a discharger cooperating with the housing comprises a pin or the like, the guidance of the container relative to the housing can be further improved by means of such complementary parts.

Advantageously the at least one slot comprises two longitudinal sections that are offset in parallel to one another and to the longitudinal axis and that are connected to one another via a connection section. Such a design can then be used to form e.g. a child lock and/or to clearly delimit e.g. a storage state of a discharger comprising such a container relative to a discharging or discharged state of the discharger.

In a preferred embodiment the container is filled with a fluid. In this case substances such as frontline can be readily stored in the container. In this connection it should be noted that the container is advantageously sealed off by the seal that is preferably configured as a membrane. A membrane is an advantageous kind of seal that following its piercing enables the correct interaction of the container with the discharger.

The container is preferably filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

In this way, for example, bleaching agents for teeth, fluoride for teeth, disinfectants, adhesives, wound adhesives, topical anesthetics, sunscreen, after sun, skin moisturizers or other drugs and cosmetics can be stored in the discharger and administered using the discharger. Such fluids can e.g. be the aforementioned frontline, eye drops and nose drops used to e.g. decongest eyes and noses of patients suffering from allergies, colds or flues.

In a further aspect the present technology relates to a discharger comprising the container described herein.

The discharger further comprises a housing and a static piston, wherein the container is configured to be moved relative to the housing and to the static piston between a storage state, a discharging state and a discharged state, wherein at least some of the housing is received in the groove in the storage state, in the discharging state and in the discharged state and wherein the groove is preferably configured to receive and to permit a movement of a cylindrical wall of the housing therein in order to guide the container relative to the housing and in particular wherein the groove is configured to receive more of the housing in the discharging state than in the storage state and especially to receive more of the housing in the discharged state than in the discharging state.

The advantages described in connection with the container likewise hold true with respect to the discharger described herein.

The improved guidance is in particular brought about by forming a width of the guide groove marginally wider than a thickness of the housing in such a way that the housing is moveably received in the guide groove with sufficient clearance to move, but yet not so much clearance that a play is present between the housing and the container allowing these components to become skew with respect to one another.

Preferably the static piston comprises at least one sealing element configured to engage an inner surface of the compartment when the discharger is in one of the discharging state and the discharged state.

The at least one sealing element thereby provides a direct seal between the static piston and the compartment of the container ensuring that no fluid can pass between the at least one sealing element and the inner surface of the compartment and thereby preferably prevent the fluid from leaking from the container into a part of the housing that is not configured to receive a fluid during either the discharging or discharged state.

Advantageously the housing comprises a sealing element at an inner surface thereof, with the sealing element being configured to engage an outer surface of the compartment when the discharger is in one of the discharging state and the discharged state.

In the event that fluid does pass between the at least one sealing element and the inner surface of the compartment into a part of the housing, this further sealing element can prevent the fluid from exiting between the housing and the container, where a user has his/her hands during discharging. This means that, at least during discharging, a user does not come into direct contact with the fluid present in the discharger.

In a further aspect the present technology relates to a method of assembling a discharger as described herein, the method comprising the steps of:
- introducing at least some of the housing into the groove of the container, and
- axially displacing the container in the direction of the longitudinal axis relative to the housing. The advantages associated with the discharger in accordance with the invention likewise hold true for the method described herein.

Advantageously the method comprises the further steps of subsequently rotating the container, and activating a child lock while rotating to arrive in the storage state of the discharger. Such child safety locks prevent the accidental activation of the discharger.

Such a discharger comprises a dispensing element and a housing, the housing having a static piston connected to the dispensing element at least in a fluid conducting manner and with the static piston having a piercing tip and being arranged within the housing; the discharger being configured to receive at least a portion of a container for containing a fluid, with the container then being moveable relative to the static piston and the housing along a longitudinal direction of the discharger, wherein the housing comprises a sealing element arranged at an inner surface of the housing, with the sealing element projecting from the inner surface of the housing in the direction of the longitudinal direction of the discharger.

In use of the discharger, the sealing element arranged at the inner surface of the housing is configured to engage a surface of a container in particular when the discharger is in one of the discharging state and the discharged state. This sealing element is provided to prevent the fluid from exiting between the housing and the container. This is the region of the discharger where a user has his/her hands during discharging. This means that, at least during discharging, a user does not come into direct contact with the fluid present in the discharger, as leaks in this region can be prevented by the sealing element present at the inner surface of the housing.

The present technology thus directly seals between the parts of the discharger moveable relative to one another where the presence of a leak is most likely. This is achieved in a cost effective manner through the provision of a seal at the inner surface of the housing.

Preferably the housing has a cylindrical wall having the inner surface and the sealing element is arranged to circumferentially extend around the inner surface of the cylindrical wall. A cylindrical housing is simple to manufacture e.g. in injection molded processes. Providing a sealing element that extends around the complete inner surface of such a housing means that an effective seal is formed without interruptions.

Moreover, round parts can be aligned accurately relative to one another permitting a simple mounting of the container at the housing of the discharger. Such a design of housing can thus advantageously also facilitate the mounting of the container at the discharger.

It is preferred if the sealing element is arranged at an inner surface of the housing in the region of the piercing tip. On use of the discharger the forces that arise during the piercing action of the seal cause the fluid stored in the container to leak out in the region of the piercing tip. By providing the sealing element in the region of the piercing element thus means that it is placed in the vicinity of the part of the housing where a leak is most likely to arise.

Particularly preferably the sealing element is an inwardly projecting sealing lip. A sealing lip is simple and cost effective to manufacture and ensures a good seal between two components moveable relative to one another.

Advantageously the sealing element projects in the direction of the piercing tip arranged along the longitudinal direction. In this way the sealing element is provided in direct vicinity of the part of the housing where a leak is most likely to arise.

Preferably the sealing element is arranged in a plane perpendicular to the longitudinal direction, with the plane further comprising at least one of the piercing tip, an end of the piercing tip and an inlet of the piercing tip. Such a design has found to yield particularly good sealing results.

It is preferred if the sealing element is either integrally formed at the inner surface of the cylindrical wall or is in contact with the inner surface of the cylindrical wall. An integrally formed sealing element is simple to manufacture e.g. during an injection molding process and ensures an intimate contact of the sealing element at the housing. A separate sealing element, such as an O-ring, can likewise be employed in a simple and cost effective manner.

Advantageously the discharger further comprises the container, wherein the sealing element is only in contact with or connected to the housing in a storage state of the discharger, i.e. the container does not contact the sealing element in the storage state; and wherein the sealing element is configured to engage the container in at least one of a discharging state and a discharged state of the discharger in addition to being in contact with or connected to the housing in both the discharging state and the discharged state of the discharger. In this way a discharger is formed in which forces only act on the sealing element when the discharger has been displacement from the storage state into one of the discharging and discharged state of the discharger. Such forces could lead to a deterioration over time of the at least one sealing element and hence to a reduced sealing action in this region on use of the discharger when the discharger is in one of the discharging state and the discharged state.

Preferably the container comprises a seal, with the piercing tip being configured to pierce the seal of the container when the container is moved out of the storage state and towards the housing, and in particular wherein the sealing element is configured to engage the container, preferably a surface of the container, as soon as the seal of the container is in contact with the piercing tip.

Using a seal the container can be used to store fluids. By permitting the seal to only be opened once the discharger is activated, the fluid stored therein will not be subjected to the surrounding atmosphere prior to activation of the discharger. Moreover, by positioning the sealing element within the housing such that it only comes into contact with the container once the seal is pierced, no forces act on the sealing element prior to activation of the discharger ensuring that the sealing element does not deteriorate during storage of the discharger.

Advantageously the static piston comprises at least one further sealing element arranged at the piercing tip. Such additional sealing elements further improve the leak tightness of the discharger.

Preferably the at least one further sealing element provides a seal between the piercing tip and an inner surface of the container when the moveable container is moved relative to the housing into a position in which the seal is pierced, with said inner surface being different from the surface of the container The at least one further sealing element of the static piston thereby provides a direct seal between the static piston and the container ensuring that no fluid can pass between the at least one sealing element and an inner surface of the container and thereby preferably prevent the fluid from leaking from the container into a part of the housing that is not configured to receive a fluid during either the discharging or discharged state.

In this connection it should be noted that the at least one further sealing element could also be a sealing lip. A sealing lip is simple and cost effective to manufacture and ensures a good seal between two components moveable relative to one another.

It is preferred if the container comprises a compartment in its interior in which the fluid is initially stored prior to piercing the seal, and wherein said surface that is engaged by the sealing element of the housing is an outer surface of a wall of the compartment.

Advantageously the container further comprises an outer wall arranged in parallel to the wall of the compartment and a guide groove present between the outer wall and the wall of the compartment, with said guide groove being configured to receive at least some of the housing.

The guide groove is provided at such a double walled container as a mounting guide. Thereby a skew mounting of the container at a housing of a discharger can be avoided. Moreover, in use of the discharger the groove acts as a guide groove. This is because the housing can be guided in the groove relative to the container, when the container is mounted at a discharger.

Preferably the discharger comprises a child safety lock. Such child safety locks prevent the accidental activation of the discharger.

It is preferred if a fluid is stored in the container in the storage state of the discharger. In this case substances such as frontline can be readily stored in the container. In this connection it should be noted that the container is advantageously sealed off by the seal that is preferably configured as a membrane. A membrane is an advantageous kind of seal that following its piercing enables the correct interaction of the container with the discharger.

Advantageously the discharger is filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

In this way, for example, bleaching agents for teeth, fluoride for teeth, disinfectants, adhesives, wound adhesives, topical anesthetics, sunscreen, after sun, skin moisturizers or other drugs and cosmetics can be stored in the discharger and administered using the discharger. Such fluids can e.g. be the aforementioned frontline, eye drops and nose drops used to e.g. decongest eyes and noses of patients suffering from allergies, colds or flues.

In a further aspect the present technology relates to a method of discharging a fluid from a discharger, in particular a discharger as discussed in the foregoing, the method comprising the steps of:
- guiding a container in a longitudinal direction of the discharger towards a piercing tip of a static piston accommodated within a housing of the discharger,
- piercing a seal present at the container by means of the piercing tip causing the seal to be pierced, in particular initially at a center of the seal,
- bringing an outer surface of the container into engagement with at least one sealing element arranged at an inner surface of the housing to prevent the fluid from passing between the at least one sealing element and the outer surface, and preferably
- guiding the container further towards the static piston to discharge the fluid via an outlet of the discharger.

The advantages associated with the discharger in accordance with the invention likewise hold true for the method described herein.

In accordance with the invention a discharger for discharging a predetermined amount of fluid comprises
- a housing defining a longitudinal first axis and having a proximal end and a distal end,
- a discharge section having a discharge passage for the fluid extending between an inlet opening and an outlet opening of the discharge section,
- a carriage cooperating with the proximal end region of the housing such that the carriage is moveable relative to the housing along the first axis, the carriage comprising a receiving space for the fluid, and
- a piercing tip being separate from the carriage and having a flow channel for establishing a flow connection for the fluid from the receiving space of the carriage to the inlet opening of the discharge section, the piercing tip defining a centrally extending longitudinal second axis and having a shell surface, wherein the shell surface of the piercing tip has an essentially convexly curved, in particular dome-shaped, configuration.

The discharger, in general, may be adapted to be used in the medical, dental or veterinary field or in the health care sector. The fluid to be discharged is in particular a liquid which includes at least one medical, dental or veterinary agent. The present disclosure is, however, not limited to the medical, dental or veterinary sector and can be used in other appliances in which it is desirable to discharge a predetermined amount of fluid in a reliable and uniform manner.

The present disclosure is not limited to the discharge of fluids such as liquids. Rather, the discharger as disclosed herein may alternatively be adapted or used to discharge viscous or gel-like materials.

In a preferred embodiment, the flow channel is centrally located within the piercing tip and extends along the longitudinal second axis defined by the piercing tip, thereby establishing a flow connection to the inlet opening of the discharge section.

The flow channel may also be located at an offset position relative to the longitudinal second axis. In this case, the flow channel, for example, may extend inclined relative to the second axis. The flow channel may also be divided into different axially and radially extending portions. For example, the flow channel may be divided into a first axial portion extending offset from the second axis merging into a radial portion which, in turn, merges into a second axial portion extending offset from the second axis or along the second axis, thereby establishing a flow connection to the inlet opening.

The flow channel may be cylindrical and preferably has a diameter between 1/5 and 1/20, more preferably between 1/7 and 1/15, particularly between 1/9 and 1/11 of a diameter of the piercing tip at its widest point. This widest point, in general, corresponds to the point where the piercing tip starts to taper. However, the flow channel may also have an oval, triangular or rectangular cross-section, for instance.

The flow channel defines an aperture at the shell surface of the piercing tip, i.e. the aperture represents an interruption of the shell surface. Unless otherwise stated, this aperture shall - within the scope of the following discussion of the configuration or shape of the piercing tip - not be considered in detail. This means, the discussion, in general, shall be based on a notional closed shell surface of the piercing tip, unless otherwise stated.

According to the technology, an outer surface of the piercing tip is convexly curved or vaulted to the outside.

In other words, the shell surface of the piercing tip contains at least an outer surface section which has a convexly curved configuration. This means that the piercing tip may also contain at least one surface section which is not convexly curved, but rather is, for example, conical or truncated conical. According to the technology, however, the shell surface of the piercing tip has an essentially convexly curved configuration. More preferably, the piercing tip has an essentially dome-shaped configuration. In this context, the expression "essentially" means that the main configuration of the piercing tip, i.e. the main part of the shell surface of the piercing tip, is convexly curved or dome-shaped, respectively, but that there may be small regions, in particular in an area where the aperture of the flow channel is located, in which the configuration may slightly deviate from a convexly curved or dome-shaped configuration. It is of course also possible that the shell surface of the piercing tip has a complete convexly curved or dome-shaped configuration.

In a preferred embodiment, the piercing tip is essentially rotationally symmetric about the centrally extending longitudinal second axis defined by the piercing tip. It is particularly preferred in this context that the longitudinal second axis corresponds to the longitudinal first axis defined by the housing of the discharger, i.e. the piercing tip is preferably also essentially rotationally symmetric about the longitudinal first axis. Against this background, the expression "essentially" also implies that the piercing tip, in particular in an area where the aperture of the flow channel is located, may slightly deviate from a rotationally symmetric configuration.

Surprisingly, it turned out that a piercing tip having a convexly curved outer surface, in particular being essentially rotationally symmetric, is especially well-suited not only for reliably piercing a wide variety of barrier foils but also for a continuous and uniform discharge process. The reason for this is that the piercing tip according to the technology combines two features. On the one hand, only a relative moderate and user-friendly piercing force is necessary to initially pierce the foil. On the other hand, in particular due to its convexly curved configuration, the force that must be applied during the displacement of the piercing tip, i.e. during the discharge process, remains essentially constant over a wide distance. This ensures a highly homogeneous discharge, as the user during the discharge process only needs to apply a substantially constant and moderate force.

The shape of the piercing tip being essentially rotational symmetrical can be defined by two surface lines of a longitudinal cross-section along the second axis. The point at which the two surface lines intersect may be referred to as apex or vertex of the piercing tip.

In embodiments having a piercing tip being essentially rotationally symmetric, the two surface lines are essentially axially symmetric relating to the longitudinal second axis. In this case, the configuration of the two surface lines is essentially independent of the relative position of the longitudinal cross-section along the second axis. This means that two surface lines resulting from a first longitudinal cross-section along the second axis essentially have the same configuration as two surface lines resulting from a second longitudinal cross-section along the second axis being rotated, for example, by 90° relative to the first longitudinal cross-section. As above, the expression "essentially" implies that the surface lines, in particular in an area where the aperture of the flow channel is located, may slightly deviate from an axially symmetric configuration.

In embodiments having a flow channel being centrally located along the longitudinal second axis, the apex of the piercing tip can be an imaginary point located where the two surface lines would intersect if they were not interrupted by the flow channel.

Each of the two surface lines, in general, can be divided into two or more different sections having different characteristics. For example, a surface line can be divided into a linear section and a curved section, wherein the curved section may be a segment of a circle, an ellipse or a parable. The curved section of the surface line corresponds to the convexly curved outer surface section of the piercing tip. According to another example, a surface line can be divided into a section being a segment of a circle and a section being a segment of a parable or ellipse. According to still another example, a surface line can be divided into different sections, wherein each section represents a segment of a circle with a different diameter.

In an embodiment, the two surface lines of a longitudinal cross-section along the second axis intersect at an angle of between 70° and 110°, preferably between 80° and 100°, more preferably between 85° and 95°, in particular approximately 90°.

According to this embodiment, each of the two surface lines can be divided into at least two different sections. A linear section forming the apex of the piercing tip and at least another curved section representing a convexly curved outer surface section of the piercing tip. This means that the outer surface section of the piecing tip is divided into a conical section forming the apex and into at least another convexly curved surface section. Preferably, the conical section is rather small compared to the convexly curved surface section.

In a further embodiment, the shape of the piercing tip in a longitudinal cross-section along the second axis is defined by two surface lines, each surface line having at least one section defined by a curvature radius being larger than the diameter of the piercing tip at its widest point. According to this embodiment, each of the two surface lines comprises at least one section being a segment of a circle with a radius being larger than the diameter of the piercing tip at its widest point.

Expediently, this section represents the largest portion of the surface line, wherein it is particularly preferred that the surface line comprises only one further section, preferably having a curvature radius of smaller diameter.

The curvature radius being larger than the diameter of the piercing tip at its widest point may have a length of between 1,01 and 1,50, preferably between 1,05 and 1,40, more preferably between 1,10 and 1,30, in particular approximately 1,20, times the diameter of the piercing tip at its widest point.

In still a further embodiment, the piercing tip in a longitudinal cross-section along the second axis is defined by two surface lines, each surface line having at least two sections of different curvature radii. According to this embodiment, each of the two surface lines comprises at least two sections each being a segment of a circle with different radius.

Preferably, the curvature radius of the first section, i.e. the section which starts where the piercing tip begins to taper, is smaller than the curvature radius of the second section, i.e. the section which follows the first section. The length of the curvature radius of the first section lies preferably in the range between one tenth and half of the curvature radius of the second section.

The piercing tip according to another embodiment of the discharger may comprise a ridge at its apex, wherein the ridge preferably is perpendicular to the longitudinal second axis. The ridge, however, can also be inclined or slanted relative to the longitudinal second axis.

The length of the ridge preferably lies between 0,05 and 0,40, preferably between 0,10 and 0,35, more preferably between 0,15 and 0,30, times the diameter of the piercing tip at its widest point.

Even without taking into account the flow channel, the shape of the piercing tip having a ridge at its apex at least slightly deviates from a rotationally symmetric configuration. This, in particular, becomes obvious when considering a first longitudinal cross-section along the second axis and along the ridge as well as a second longitudinal cross-section being rotated by 90° relative to the first longitudinal cross-section.

With regard to the characteristics of the surface lines defining the piercing tip in the first cross-section, largely the same applies as outlined above relating to the essentially rotational symmetrical embodiments. However, instead of an apex the piercing tip has a ridge-like elevation which preferably is located above an imaginary apex of the piercing tip. This imaginary apex is located where the two surface lines would intersect if they were not interrupted by the ridge.

As regards the second longitudinal cross-section being rotated by 90° relative to the first one, i.e. the plane of the second cross-section is perpendicular to the ridge, the shape of the piercing tip preferably can be described by two surface lines intersecting at an angle of between 70° and 110°, preferably between 80° and 100°, more preferably between 85° and 95°, in particular approximately 90°. This means that each of the two surface lines is divided into a linear section corresponding to a lateral edge of the ridge and at least one curved section which may have the characteristics as outlined previously.

Expediently, the ridge is interrupted by the flow channel such that two protrusions adjacent to the flow channel are formed. In other words, the flow channel divides the ridge into two separated ridge-like protrusions which preferably have about the same dimensions.

Advantageously, these protrusions lower the piercing force that has to be initially applied for puncturing a barrier foil. In this way, a wide variety of barrier foils can be reliably punctured and the discharger according to the present technology can be applied substantially independent of the type of barrier foil being used.

Expediently, the ridge or the protrusions adjacent to the flow channel are deformable, particularly upon being pressed against a counterpart, wherein the counterpart preferably represents an end section of the receiving space. Due to their deformability the protrusions are compressed when the piercing tip is forced towards the counterpart. In this way, the space between the piercing tip and the counterpart being available for the fluid to be discharged becomes minimized which, in turn, leads to lower amounts of fluid residues at the end of the discharge process. The compression of the protrusions progresses in such a way that the flow channel does not become clogged by the displaced material which is basically due to the position and shape of the protrusions.

The piercing tip is preferably at least partially positioned inside the housing so as to protrude into the receiving space of the carriage when the carriage is being moved towards the distal end of the housing. By means of the flow channel, the piercing tip thereby establishes a flow connection for the fluid to the inlet opening of the discharge section.

By providing a carriage with a receiving space for the fluid, either a separate container being adapted to be loaded into the receiving space and containing the predetermined amount of fluid can be loaded into the carriage or the predetermined amount of fluid to be discharged can be directly stored inside the receiving space. The amount of the fluid either contained in the container or in the carriage itself may for example range between 0,1 and 10 ml, preferably between 0,5 and 5 ml.

Expediently, the fluid inside the receiving space or container is protected from environmental influences by a breakable seal, in particular a barrier foil.

It is preferred that a separate container is applied to avoid a direct storage of the fluid inside the receiving space of the carriage. The carriage, in this way, can be reusable since it normally does not come into contact with the fluid to be discharged. Even if the carriage or the whole discharger is a disposable product by providing a separate container containing the fluid storage costs for the discharger can be reduced since the discharger and the containers can be stored independently.

In a preferred embodiment, an inner contour of the receiving space or an inner contour of the container is essentially complementary in shape to the piercing tip. The expression "essentially", in this context, implies that the shape of the inner contour can deviate from an exact complementary shape of the piercing tip in an area where the aperture of the flow channel is located and, where applicable, in an area where the protrusions are located.

This design allows for a nearly complete discharge of the fluid since the space available for the fluid can be completely filled by the piercing tip resulting in a nearly complete displacement of the fluid.

In this context, the piercing tip being essentially rotationally symmetric is of particular advantage since a special orientation of the piecing tip relative to the complementary shaped and accordingly also rotationally symmetric inner contour of the receiving space or the container is not necessary to establish a form-fitting connection. This also facilitates the assembly of the discharger since neither the piercing tip nor the complementary designed receiving space or container needs to adopt a preferred orientation direction.

It should be noted in this connection that also the piercing tip comprising two deformable protrusions can establish a form-fitting connection to the inner contour of the receiving space or the container even if this inner contour is rotationally symmetric and has not been specially adapted to the protrusions. Due to the deformability of the protrusions the piercing tip comprising the deformable protrusions is able to adapt to the rotationally symmetric inner contour of the receiving space or the container upon applying a force.

In this way the advantage of a reduced initial force for puncturing a barrier foil being due to the protrusions at the piercing tip and the advantage of a nearly residue-free discharge can be combined.

In a further embodiment the piercing tip is formed integrally with the housing and/or the discharge section. In other words, it is preferable that the piercing tip and the housing and/or the discharge section are formed in one piece.

However, it can also be preferable when the piercing tip is formed as a unitary separate piece which, for example, can be linked to the housing and/or the discharge section for example by a plug connection.

Expediently, the piercing tip is formed by injection molding and preferably consists of an elastically deformable polymer selected from the group comprising polypropylene, cyclic olefin polymer, polyethylene, polyamide, polybutylenterephthalat and polymethyl methacrylat.

The discharger and/or the container, in general, may be fabricated from any suitable material. In one embodiment, the material is plastic, wherein the same polymers as named above are suitable. Alternatively, the material may be glass, metal or an alloy.

In an embodiment, the discharger further comprises securing means which are adapted to prevent the carriage from being unintentionally moved from a starting position along the longitudinal first axis. These securing means facilitate the handling of the discharger by avoiding unintentional operation of the carriage.

In particular, the securing means may comprise a pin/slot arrangement having at least one pin formed at the carriage and at least one slot formed in a wall of the housing. The pin is guided by the slot. The slot may comprise a first portion extending in a circumferential direction and merging into an axial slot portion extending along the longitudinal first axis of the discharger. Such securing means require the user to rotate the carriage with respect to the housing before the carriage can be pushed into the housing and towards a final discharge position.

Means such as wing-like projections can be formed at an outer side of the housing so that the discharger may be used like a common syringe by holding the discharger with one finger at each projection and with the thumb on a proximal end of the carriage or of a container loaded into the carriage, thereby providing for a comfortable single-hand operation of the discharger.

Another aspect of the technology relates to a discharge system comprising a discharger as disclosed herein and at least one container holding an amount of fluid to be discharged, wherein the container is adapted to be loaded into the receiving space of the carriage of the discharger.

A further aspect of the technology relates to the use of a discharger as disclosed herein or of a discharge system as disclosed herein, for discharging a liquid including at least one medical, dental or veterinary agent, wherein in particular the amount of the liquid lies in the range of 0,1 to 10 ml, preferably between in the range of 0,5 to 5 ml.

With respect to one common classification applied in the medical, dental or veterinary field or the health care sector, the discharger and the discharge system as provided by the present disclosure belong to the group consisting of systems without protection cap.

Accordingly, one aspect of the present disclosure relates to a discharger comprising a housing defining a longitudinal axis and having a proximal end and a distal end, comprising a discharge device arranged at least partly inside the housing and having an inlet opening situated inside the housing and an outlet opening, the discharge device further defining a discharge passage for the fluid extending between the inlet opening and the outlet opening, comprising a carriage received in a proximal end region of the housing and being movable relative to the housing along the longitudinal axis between a starting position and a final discharge position, wherein in the starting position the carriage protrudes from the proximal end of the housing so as to be pushable by a user further into the housing and towards the final discharge position, and wherein the carriage defines a receiving space which is adapted to be loaded with a separate container holding an amount of fluid to be discharged, and comprising activation means for establishing a flow connection for the fluid from within the receiving space of the carriage to the inlet opening of the discharge device, the activation means being inoperative when the carriage is in the starting position and being adapted to establish the flow connection when the carriage is in the final discharge position or is being moved towards the final discharge position.

According to another aspect of the present disclosure, a container is provided which includes a predetermined amount of fluid to be discharged by the discharger. According to a further aspect of the present disclosure, a discharge system is provided which comprises a discharger and a container as disclosed herein, the container being loaded into or being adapted to be loaded into the carriage of the discharger.

The discharger may be adapted to be used in the medical, dental or veterinary field or in the health care sector, the fluid included in the container in particular being a liquid which includes at least one medical, dental or veterinary agent. The present disclosure is, however, not limited to the medical, dental or veterinary sector and can be used in other appliances in which it is desirable to discharge a predetermined amount of fluid in an easy and reliable manner with the fluid being well-protected against environmental influences.

The present disclosure is not limited to the discharge of fluids such as liquids. Rather, the discharger as disclosed herein may alternatively be adapted or used to discharge a viscous material
The concept of the present disclosure is to avoid a direct storage of the fluid inside the discharger itself. Consequently, a practically unlimited storage life of the discharger is achieved. By providing a carriage with a receiving space, a separate container can be loaded into the discharger. This concept allows for an easy and yet reliable single-hand operation of the discharger since the flow connection from the container being loaded into the receiving space of the carriage to the inlet opening of the discharge device may be automatically established by the activation means of the discharger.

The amount of the fluid included in the container may range between 0,2 and 2 ml. In an embodiment, the amount of the fluid included in the container is 0,5 ml.

In an embodiment the activation means establish the flow connection when the carriage is on its way between the starting position and the final discharge position so that the fluid can begin to flow through the discharge passage before the carriage reaches its final discharge position. In an alternative embodiment the activation means may require an additional operation step to be performed by the user in order to actually establish the flow connection. In a particularly comfortable and yet reliable embodiment, however, the flow connection is established automatically by moving the carriage towards the final discharge position.

As already mentioned before, in one embodiment the flow connection is established before the carriage reaches the final discharge position. In an alternative embodiment the carriage has to be in its final discharge position for the flow connection to be established, either automatically or through an additional operation step to be performed by the user.

In an embodiment, the activation means are positioned inside the housing so as to protrude into the receiving space when the carriage is being moved from the starting position towards the final discharge position. By protruding into the receiving space, the activation means are able to interact with a container loaded into the receiving space, for example by breaking a seal of the container.

The activation means may be provided with means for breaking a seal. In particular, these means are adapted to provide a piercing, penetrating, puncturing and/or perforating action for the seal.

This embodiment has the advantage that a container loaded into the receiving space can be activated automatically in an easy and reliable manner simply by pushing the carriage, including the container, into the housing of the discharger.

Means such as wing-like projections can be formed at an outer side of the housing so that the discharger may be used like a common syringe by holding the discharger with one finger at each projection and with the thumb on a proximal end of the carriage or of a container loaded into the carriage, thereby providing for a comfortable single-hand operation of the discharger.

The discharge device including the inlet opening, the outlet opening and the fluid passage extending between these openings may be, at least partially, formed integrally with the housing of the discharger. In an embodiment, the discharge device may comprise a portion formed integrally with the housing and one or more separate parts which are connected to the integral portion. In such an embodiment, the activation means may be formed by the portion being integral with the housing or by a separate component of the discharge device, or both.

The discharge device may have a proximal end portion generally facing towards the receiving space, the activation means and the inlet opening being formed at or being integrated into the proximal end region. The proximal end region may be, at least partially, formed integrally with the housing.

In order to enable an easy loading of the container into the carriage, the carriage may have a proximal end having an insertion opening, the receiving space being accessible through the insertion opening, wherein the carriage is adapted to be loaded with the container by sliding the container through the insertion opening into the receiving space. The receiving space may be adapted or matched to the container such that a proximal end face of the container, when loaded into the carriage, is flush with the proximal end of the carriage. In this way, the carriage and the loaded container form a common proximal end face to be acted upon by a user like a push button.

In a further embodiment, the discharge device is adapted to guide the carriage along the longitudinal axis when the carriage is being moved from the starting position towards the final discharge position. Guiding the carriage is an additional function which is imparted to the discharge device in this embodiment. The mechanical stability and reliability are improved by guiding the carriage.

The discharge device may have a proximal end portion including the inlet opening and the carriage may have a distal opening, wherein the discharge device may extend with its proximal end portion through the distal opening into the carriage. In this embodiment, by extending into the carriage, the discharge device may be used to guide the axial movement of the carriage and at the same time may interact with a container loaded into the carriage in order to establish the flow connection for the fluid in the container.

In particular, the proximal end portion of the discharge device is sealingly fitted into the carriage so as to prevent fluid leakage through the distal opening of the carriage. Fitting the proximal end portion of the discharge device into the carriage eliminates the need for separate sealing elements, thereby providing a simple and effective concept in order to ensure that the fluid from the container flows at least substantially only into the inlet opening and through the discharge passage of the discharge device.

In an embodiment, the proximal end portion of the discharge device has an outer diameter which is smaller than an inner diameter of the carriage in the area of the sealing space. Hereby, a reception space for a wall portion of the container is created, this reception space being disposed around the proximal end portion of the discharge device when the carriage is being moved towards the final discharge position. In addition, this concept leads to an efficient usage of the space available within the housing so as to minimize the outer dimensions of the housing and thus of the discharger as a whole.

In an embodiment, the carriage generally has a multiple-cylinder shape at least comprising a proximal cylindrical portion and a distal cylindrical portion. While the proximal cylindrical portion may define the receiving space for the container, the distal cylindrical portion may be used to guide the carriage within the housing or at least to assist guidance of the carriage while at the same time receiving a proximal end portion of the discharge device.

The proximal cylindrical portion of the carriage may have an outer diameter which is greater than an outer diameter of the distal cylindrical portion of the carriage.

In a further embodiment, the proximal cylindrical portion of the carriage has an inner diameter which is greater than an inner diameter of the distal cylindrical portion of the carriage.

Different inner diameters of the cylindrical portions of the carriage may be used to provide for an additional function of the carriage. In particular, inside the carriage a transition portion may be provided between the proximal cylindrical portion and the distal cylindrical portion. The transition portion may define a distal end of the receiving space so as to serve as a stop for the container being loaded in the receiving space.

In an embodiment, the discharger further comprises securing means which are adapted to prevent the carriage from being unintentionally moved from the starting position along the longitudinal axis. These securing means facilitate the handling of the discharger by avoiding unintentional operation of the carriage.

In particular, the securing means may comprise a pin/slot arrangement having at least one pin formed at the carriage and at least one slot formed in a wall of the housing, the pin being guided by the slot. The slot may comprise a first portion extending in a circumferential direction and merging into an axial slot portion extending along the longitudinal axis of the discharger. Such securing means require the user to rotate the carriage with respect to the housing before the carriage can be pushed into the housing and towards the final discharge position.

Generally, the volume for the fluid within the container may be dimensioned as desired in accordance with the respective intended use. The outer shape and the outer dimensions of the container may be the same for different volumes and thus for different quantities of fluid included within the container.

Before this background, the present disclosure also relates to a set of a plurality of containers having at least substantially identical outer shapes and outer dimensions but being different from each other regarding the volume or quantity of contained fluid.

The outer shape of the container may be generally cylindrical.

In an embodiment, the fluid inside the container is protected from environmental influences by a breakable seal.

The container may have a distal end face at least part of which being formed by a breakable seal.

The seal may be formed by a portion of an outer wall of the container, the seal portion having a thickness being smaller than that of other portions of the wall. In an alternative embodiment, the outer wall of the container defines an opening which, after filling the fluid into the container, is closed by a separate sealing element. This sealing element may be in the form of a foil or a film.

The discharger of the present disclosure may be provided with a set of different carriages which differ from each other regarding the size and/or the shape of the receiving space. This enables the discharger to be loaded with different containers so as to enhance the discharger's range of use. Alternatively, a set of inserts may be provided, each insert being matched to a specific type of container so as to adapt the carriage to a specific type of container by inserting the insert into the carriage. In other words, a single carriage may be matched to different containers by using specific adapters to be inserted into the carriage.

In a further embodiment, the discharge device of the discharger has a proximal end portion including the inlet opening, the proximal end portion being sealingly fitted into the container when the carriage is moved towards the final discharge position, so as to prevent at least substantially fluid leakage out of the container past the discharge device. Fitting the proximal end portion into the container also assists in guiding the carriage and thus the container when the carriage is pushed into the housing.

The container may have a cylindrical wall portion which has a thickness being at least approximately the same as the difference between the inner diameters of a proximal cylindrical portion and a distal cylindrical portion of the carriage. In this embodiment, when the container is loaded into the carriage, the distal cylindrical portion of the carriage and the container together may define a cylindrical space having a constant inner diameter which may be at least substantially the same as the outer diameter of a proximal end portion of the discharge device. Thus, when the carriage, including the loaded container, is pushed into the housing, the proximal end portion of the discharge device, starting from the distal cylindrical portion of the carriage, gets into the container, thereby permanently guiding the carriage being moved into its final discharge position.

In an embodiment, the discharge device has a proximal end portion including the inlet opening and the container has an inner wall confining a fluid space of the container, wherein the inner wall comprises a proximal end section, and wherein the proximal end portion of the discharge device and the proximal end section of the inner wall being shaped at least approximately complementary to each other. In this embodiment, the fluid space of the container is more or less completely filled by the discharge device so that the residual amount of fluid contained within the fluid space is minimized when the carriage is in its final discharge position.

In another aspect, the present disclosure also relates to the use of a discharger as disclosed herein or of a discharge system as disclosed herein, for discharging a liquid including at least one medical, dental or veterinary agent, wherein in particular the amount of the liquid lies in the range of 0,2 to 2 ml and preferably is approximately 0,5 ml.

In accordance with the invention, a syringe is proposed which comprises a hollow body, a piston and an intermediate piece. The hollow body has a surrounding hollow body surface and forms a passage therein. An axis is formed in an axial longitudinal direction of the hollow body. The passage has a rear body opening and a front body opening which are arranged opposite to one another in the longitudinal direction. A first needle seat is formed at the front body opening, in which needle seat a needle cannula is arranged and relative to which needle seat the needle cannula is movable along the axis. The piston is arranged in the hollow body and is movable in the passage along the axis, wherein the piston comprises a front piston opening, a rear piston end and a surrounding intermediate wall. The intermediate wall is arranged between the front piston opening and the rear piston end and forms a receiving chamber for receiving at least one fluid. The intermediate piece is arranged in the receiving chamber and is movable along the axis, wherein a first sealing region, which seals the receiving chamber in a storage position, acts between the intermediate wall and the intermediate piece. A second needle seat and a supply space, which is in particular formed as a channel or in the manner of a channel, are formed in the intermediate piece, with the second needle seat and the supply space being in flow communication with one another. The piston, the hollow body and the intermediate piece are movable into a release position by means of a coaxial relative movement starting from the storage position. A seal is provided in the storage position between the supply space and the receiving chamber, said seal being able to be cancelled by the coaxial relative movement such that the receiving chamber and the supply space are in flow communication in the release position.

The syringe can, for example, be a disposable syringe for single use. Plastic, in particular PP or COC, can, for example, be a material from which the syringe is manufactured. Possible materials are also PE, PA, PBT and PMMA. The material can, however, also be glass, a metal alloy or metal. The material can advantageously be a pressure-resistant material.

The hollow body can be a tubular hollow body, preferably, but not necessarily, having a circular, ellipsoidal or polygonal base surface. The hollow body has a surrounding hollow body surface which forms a passage therein. An axis is formed in an axial longitudinal direction of the hollow body, preferably through the centroid of the two base surfaces of the hollow body. The hollow body can be configured as a mainly hollow cylindrical tube, with the passage having a rear body opening and a front body opening which are arranged opposite one another in the longitudinal direction.

A first needle seat is formed at the front body opening, with a needle cannula being arranged in the first needle seat and the needle cannula being movable along the axis. The front body opening can be of nozzle shape, for example. The first needle seat can be configured with a tubular form into the hollow body with a needle seat opening. The first needle seat can preferably, but not necessarily, have a circular, ellipsoidal or polygonal base surface. The first needle seat can be aligned along the axis. A fluid can flow out of the receiving chamber in the needle cannula, in particular a first fluid and a second fluid can flow out of the receiving chamber after one another. In the storage position, the needle cannula can be arranged in a first needle seat such that the needle cannula is completely moved into the hollow body. In the release position, the needle cannula can be moved out of the first needle seat, that is out of the hollow body, with the needle cannula being able to be held in the intermediate piece. In a drawn in position, the needle cannula can be moved completely into the hollow body and can no longer be arranged in the first needle seat.

The piston is arranged in the hollow body, in particular in the passage, and is movable along the axis. The piston comprises a front piston opening, which is arranged in the direction of the front body opening, and a rear, in particular closed, piston end. The front piston opening and the rear piston end are arranged opposite one another in the longitudinal direction. The piston can be moved from the storage position into the release position and vice versa. The piston can additionally in particular be moved into a flushing position and into a drawn in position, with the flushing position being located between the release position and the drawn in position. A movement of the piston from the storage position into the release position can take place, for example, as with a conventional syringe, by pressing onto the rear piston end which can be configured as a push button, for example. So that the piston cannot be pressed further into the hollow body in the release position, the rear piston end can be configured as an abutment device, for example as a projection. In the storage position, the piston can be held in the hollow body by means of a holding device, i.e. a movement of the piston can be prevented, and indeed such that a movement of the piston is only possible from a specific force action onto the rear piston opening onward. The holding device can, for example, comprise a dimple in the piston and a groove in the hollow body, or vice versa. The dimple can be latched in the groove, for example. The piston can also be guided in the axial direction by means of a guide element in the passage.

The piston equally comprises a surrounding intermediate wall which is arranged between the front piston opening and the rear piston end and which forms a receiving chamber for receiving fluid, in particular a first fluid and a second fluid. The receiving chamber can be configured as a U-shaped tube, with the base surface of the receiving chamber being able to have a circular, ellipsoidal or polygonal base surface. The receiving chamber can in particular have a volume of 0.1 to 100 ml, preferably a volume of 0.5 to 2 ml.

A second needle seat is formed in the intermediate piece which is arranged in the receiving chamber and which is movable along the axis, with the needle cannula being arranged in the second needle seat and being movable along the axis. The needle cannula can, however, also be held in the intermediate piece, in particular in an inner element or in the second needle seat, such that a coaxial movement of the needle cannula independently of the intermediate piece is not possible. The second needle seat can be aligned along the axis. In the storage position, the needle cannula can be arranged in the first and second needle seats so that the needle cannula is moved at least approximately completely into the hollow body. In the release position, the needle cannula can be moved out of the hollow body, with the needle cannula being held firmly in the axial direction, for example, by means of a needle holder in the intermediate piece, in particular by means of a needle holder in the second needle seat. The needle cannula can e.g. be latched to the needle holder or can be bonded or clamped into the needle holder. The intermediate piece can be moved in the passage along the axis by means of a force transmission from the piston onto the intermediate piece.

In accordance with the invention, a supply space is formed in the intermediate piece and the supply space is in flow communication with the second needle seat. The supply space can be provided in the form of one or more bores in the intermediate piece. The bore can e.g. have a largest diameter of 3 mm and can, for example, be cylindrical. A cross-sectional shape of the bore perpendicular to or in parallel with the axis can e.g. be rectangular or generally polygonal. The supply space can be arranged with a longitudinal axis at an angle of e.g. 50 to 90 degrees, preferably of at least approximately 90 degrees, to the axis. Flow communication is in particular to be understood such that a fluid can flow or stream from the supply space into the second needle seat and into the needle cannula.

The piston, the hollow body and the intermediate piece are movable relative to one another by means of a coaxial movement starting from a storage position into a release position. In the storage position, the receiving chamber is sealed with respect to the supply space. The storage position is in particular to be understood such that the piston can be pulled out of the hollow body, such that the front piston opening is therefore located in the vicinity of the rear body opening, and such that it is prevented by sealing that a fluid flows from the receiving chamber into the intermediate piece and from there into the needle cannula. The release position is to be understood such that the piston has been moved by means of a coaxial movement into the hollow body, for example by pressing at the rear piston end, and such that the seal is thereby cancelled so that the fluid can flow from the receiving chamber into the intermediate piece and from there into the needle cannula. In this respect, starting from the storage position, the needle cannula can first be moved out, with the seal still remaining activated. The seal is deactivated on the transition into the release position. In a subsequent drawn in position with a piston pulled back again, the needle cannula can moreover be drawn into the hollow body.

The advantages of the syringe in accordance with the invention are that the syringe serves as a functional, primary packaging means and an active ingredient is already present in the syringe and does not first have to be drawn in, whereby, for example, contamination problems can be precluded. In addition, the syringe is easier to operate, for example with one hand, because a complex plugging on or screwing on of the needle cannula is dispensed with. The optionally provided draw-in position has the advantage that a risk of injury or infection by the needle cannula can be precluded after the use of the syringe.

In a possible embodiment of the invention, the intermediate piece comprises an outer element and an inner element. The inner element in this respect comprises the second needle seat and an abutment for the outer element. The inner element is in particular arranged with the abutment such that an axial movement of the inner element relative to the outer element is bounded by the abutment in the direction of the rear body end. The supply space can be of conical design. A seal can be formed between the inner element and the outer element.

The intermediate piece can be in one part or in multiple parts. The intermediate piece can comprise an outer element and an inner element, with the second needle seat being able to be arranged either at the outer element or at the inner element. The inner element can be arranged partly, or also completely, in the outer element. The inner element can in particular be arranged in a bore which can have a circular or polygonal cross-section. The supply space can be arranged at the inner element or at the outer element. The inner element further comprises an abutment for the outer element. The abutment can be configured, for example, as a peripheral edge, in the form of one or more webs or as a step. In the storage position, a first sealing region can be formed between the intermediate wall and the outer element of the intermediate piece and a second sealing region can be formed between the inner element and the outer element. The first sealing region can be formed as a seal, for example as a groove at the intermediate wall of the receiving chamber and as a dimple at the intermediate piece, in particular at the outer element, or vice versa. The second sealing region can likewise be formed as a seal, for example as a groove at the outer element and as a dimple at the inner element. The first sealing region and/or the second sealing region can alternatively be formed as an O-ring seal. The first sealing region and/or the second sealing region can comprise one or more seals. The active ingredient can thus advantageously already be stored in the syringe and a simplified release principle of the active ingredient can be achieved.

In a possible embodiment of the invention, the receiving chamber is divided by means of a separating element into a first chamber and a second chamber. In a flushing position, the second chamber and the supply space can be in flow communication. In the storage position, a sealing region is formed and arranged between the intermediate wall and the separating element such that the second chamber and the supply space have no flow communication. The separating element can be configured as a film or as a plug which is arranged movably along the axis in the receiving chamber. The intermediate piece, on a multi-part embodiment of the intermediate piece e.g. an inner element of the intermediate piece, can be formed as a mandrel in the direction of the rear body end to break open the separating element.

The receiving chamber can be divided by means of a separating element into exactly two chambers, namely a first chamber and a second chamber. The receiving chamber can, however, also be divided into more than two chambers which are separated by means of a plurality of separating elements and which can receive more than two fluids in total. Each separating element can be configured as a film or as a plug.

In general, the or each separating element can be formed from plastic or from a metal material. The separating element is respectively arranged in the receiving chamber such that the respective chambers are separated in a fluid-tight manner. If a first chamber and a second chamber are provided, they can receive two fluids, with a first fluid being able to be arranged in the first chamber and a second fluid being able to be arranged in the second chamber. In addition, in the storage position or in the release position, a sealing region can be formed and arranged between the intermediate wall and the separating element such that the second chamber and the supply space as well as the second chamber and the first chamber do not have any flow communication. This sealing region can be configured as a seal, for example as a groove in the intermediate wall and as a dimple complementary thereto in the separating element, or vice versa. The sealing region can, however, also be an O ring. The sealing region can comprise one or more seals. A flushing position is to be understood such that the second chamber and the supply space are in flow communication with one another. The separating element or its position can be mechanically changed to establish this flow communication. For example, the film can be pierced by the mandrel or the sealing region between the separating element and the intermediate wall can be deactivated so that the second chamber and the supply space are in flow communication. In the storage position and/or in the release position, in contrast, the separating element separates the second chamber and the first chamber as well as the second chamber and the supply space such that they have no flow communication. The first fluid can in this respect, for example, be a medicine to be dispensed and the second fluid can be a flushing solution. It is of advantage in this respect that the first fluid and the second fluid, but also more than two fluids, can thus be functionally separated. In addition, the handling and the operation of the syringe is simpler since the active ingredient is already contained in the syringe and no longer has to be drawn up into the syringe.

In a possible embodiment of the invention, the inner element of the intermediate piece is configured as a further hollow body, with the piston being arranged movably in the further hollow body. The further hollow body can be a tubular hollow body, preferably, but not necessarily, having a circular, ellipsoidal or polygonal base surface. The shape of the further hollow body can correspond to that of the hollow body, with the further hollow body being able to be arranged in the passage of the hollow body. The further hollow body can comprise a further passage and extend along the axis. The further hollow body can be configured as a hollow cylindrical tube. The piston can furthermore be arranged in the further hollow body. The coaxial movement of the piston can take place along the axis in the further hollow body which in turn moves along the axis in the hollow body. A better support and guide stability of the intermediate piece is thus advantageously established.

In a possible embodiment of the invention, a spring is arranged in the passage against whose restoring force the piston is movable in the direction of the front body opening of the hollow body. The needle cannula can advantageously be more easily drawn into the hollow body by means of the spring, i.e. the spring can ensure that the syringe moves into the draw-in position or the spring can assist the transition into the draw-in position.

In a further possible embodiment of the invention, means are provided for an automatic lateral pivoting or deflecting of the intermediate piece in an end position and/or draw-in position. The means are in particular active between a rear end of the receiving chamber and a rear end of the intermediate piece and in particular comprise a sloping surface. It is possible by such means to act on the needle cannula via the intermediate piece and the second needle seat by a pivot force or deflection force which endeavors to move the needle cannula from a working alignment in parallel with the longitudinal axis of the hollow body into a safety alignment inclined with respect to the longitudinal axis. The needle cannula can in particular adopt the inclined position as soon as it moves out of engagement with the first needle seat which is formed at the front body opening and which otherwise also ensures the working alignment of the needle cannula against the action of the pivot force or deflection force.

The technology further relates to a syringe as described herein, wherein the receiving chamber already contains at least one fluid, in particular a liquid containing at least one medicine to be dispensed, wherein in particular the receiving chamber contains a liquid containing at least one medicine to be dispensed in a first chamber of the receiving chamber and a flushing solution in a second chamber of the receiving chamber.

The technology furthermore relates to a use of a syringe as described herein for discharging a fluid already contained in the receiving chamber without drawing up the fluid, in particular for injecting a liquid containing at least one medicine to be dispensed (administered), wherein a liquid containing at least one medicine to be dispensed is discharged from a first chamber of the receiving chamber in a first phase and subsequently a flushing solution is discharged from a second chamber of the receiving chamber in a second phase.

In accordance with the technology, a discharger, preferably expendable syringe, for discharging a predetermined amount of fluid, preferably from a separate container, in particular a liquid including at least one medical, dental or veterinary agent, comprises a discharge section defining a longitudinal axis and having a proximal end and a distal end and having a discharge passage for the fluid extending between an inlet opening and an outlet opening of the discharge section. The discharger further comprises an intermediate section comprising a distal portion cooperating with the proximal end of the discharge section and a proximal portion defining a receiving space which is adapted to be loaded with the separate container holding an amount of fluid to be discharged, preferably by the discharger. The distal portion and the proximal portion are formed in one piece and are interconnected by a breakable section.

The present disclosure is not limited to the medical, dental or veterinary sector and can be used in other appliances in which it is desirable to discharge a predetermined amount of fluid in a reliable and uniform manner. The present disclosure is also not limited to the discharge of fluids such as liquids. The discharger as disclosed herein may alternatively be adapted or used to discharge viscous or gel-like or even powdery or fine granular materials.

The breakable section of the intermediate section preferably can comprise a predetermined breaking area or breaking point adapted to break upon applying an axial force to the intermediate section, in particular along the longitudinal axis, especially towards the distal end of the discharge section.

Advantageously, the force upon which the predetermined breaking area or point is intended to break approximately can lie in the range of a force that can be exerted by an adult person by pressing together a thumb and index finger and/or middle finger.

It is also conceivable that the predetermined breaking area or point can be adapted to break upon applying a rotary motion to the proximal portion of the intermediate section relative to the distal portion of the intermediate section or vice versa.

The breakable section preferably can have the shape of a lip, a web, a rim, a shoulder, a film or the like. Expediently, the breakable section can comprise a tearable film, in particular being made of the same material as the distal and/or the proximal portion of the intermediate section. When applying a force the tearable film can stretch to a limited extent until it finally breaks.

The intermediate section may be formed by injection molding and preferably may consist of an elastically deformable polymer selected from the group comprising polypropylene, cyclic olefin polymer, polyethylene, polyamide, polybutylene terephthalate and polymethyl methacrylate.

It can be preferred if the breakable section can comprise or can be made of a different material than the distal portion and/or the proximal portion of the intermediate section. By choosing a suitable material for the breakable section, the predetermined breaking area or point can be adjusted and adapted to the type of usage of the discharger.

In a preferred embodiment, after breakage of the breakable section, the proximal portion of the intermediate section can be movable relative to the distal portion of the intermediate section along the longitudinal axis between a starting position and a final discharge position. The direction of movement of the proximal portion preferably can be along the longitudinal axis towards the distal end of the discharge section.

A position of the proximal portion of the intermediate section before breakage of the breakable section can be referred to as "initial position". The "starting position" can be defined as a position of the proximal portion of the intermediate section being reached immediately after breakage of the breakable section, i.e. when the proximal portion of the intermediate section can be movable relative to the distal portion. The "final discharge position" can be defined as a position of the proximal portion of the intermediate section in which - once reached by an initial motion of the proximal portion of the intermediate section - no further movement of the proximal portion in its initial direction is possible.

Against this background, the breakable section advantageously also may act in favour of child safety, since it can prevent the proximal portion of the intermediate section from being unintentionally moved along the longitudinal axis in a manner allowing for a discharge of the fluid. At least concerning young children, the provided discharger thus can be regarded as being child-resistant.

However, the discharger can further comprise securing means which can be adapted to prevent the proximal portion of the intermediate section from being unintentionally moved from the starting position along the longitudinal axis.

It is preferred if in the starting position the proximal portion of the intermediate section at least partially can protrude from the distal portion of the intermediate section and preferably can receive the distal portion to be guided through the proximal portion when the proximal portion is being moved towards the distal end of the discharge section and towards the final discharge position.

According to this embodiment, the distal portion of the intermediate section preferably can have an outer diameter essentially equal to or slightly greater than an inner diameter of the proximal portion of the intermediate section, such that the distal portion after breakage of the breakable section can be guided through the proximal portion. For example, the distal portion can have an outer diameter that is between 3 mm and 15 mm, preferably between 6 and 7 mm. The inner diameter of the proximal portion can be up to 0,1 mm or 0,2 mm smaller than the outer diameter of the distal portion. The inner diameter of the separate container can be equal to the inner diameter of the proximal portion or of a part of the proximal portion. The receiving space of the proximal portion can have an inner diameter that is equal to the outer diameter of the separate container, which can be e.g. in the range of 5 mm to 20 mm and is preferably between 8 and 9 mm.

Advantageously, in the initial position of the proximal portion of the intermediate section, i.e. before breakage of the breakable section, the distal portion of the intermediate section can be at least partially enclosed by the proximal portion. However, the distal portion can also be entirely enclosed by the proximal portion.

After the breakage of the breakable section, the distal portion and/or the proximal portion preferably maintain their structure. In other words, the breakage of the breakable section advantageously only "destroys" the breakable section itself but does not change the form or structure of the distal portion and the proximal portion. The breakable section can have a, preferably round or circular, shape that encircles the longitudinal axis.

If the structure of the distal portion and/or the proximal portion is maintained, the distal portion can continue cooperating with the proximal end of the discharge section, after the breakage of the breakable section.

In an embodiment of the discharger, the distal portion of the intermediate section and the proximal end of the discharge section can cooperate by means of a plug connection. The proximal end of the discharge section preferably can be simply inserted into an opening located at a receiving end of the distal portion and can be maintained in position particularly in a force-locking manner. An additional interconnection such as gluing or welding, especially laser welding, is not necessarily required, however, can nevertheless be performed.

The cooperation between the proximal end of the discharge section and the distal portion of the intermediate section can also be based on a snap-in connection. In this context, for instance, a locking element, such as a locking pin or the like, may be provided at the distal portion and a counter locking element may be provided at the proximal end. The proximal end of the discharge section then, as it is inserted through the opening into the distal portion of the intermediate section, can slide over the locking element at the distal portion which in cooperation with the counter locking element provided at the proximal end can prevent a sliding off or dropout of the discharge section. Additional interconnection methods like welding or gluing can be omitted.

Another possible type of cooperation between the distal portion of the intermediate section and the proximal end of the discharge section can be a screw connection, wherein the distal portion of the intermediate section preferably may be provided with an internal thread and the proximal end of the discharge section preferably may be provided with an external thread.

Preferably, the distal portion of the intermediate section further can comprise a stop portion serving as a stop for the proximal end of the discharge section when being inserted into the distal portion. In other words, the stop portion can define to what extent the distal end of the discharge section can be inserted into the proximal portion of the intermediate section.

It is particularly preferred if only between one tenth and one twentieth of the entire length of the discharge section can be received into the distal portion of the intermediate section. In this way, the dimensions of the distal portion of the intermediate section can be reduced to a necessary minimum level that allows for holding the proximal end of the discharge section in place. The material consumption thus can be kept down to a minimum.

In a further embodiment of the discharger, the distal portion of the intermediate section can comprise activation means for establishing a flow connection for the fluid from within the receiving space of the proximal portion of the intermediate section to the inlet opening of the discharge section.

One concept of the present disclosure is to avoid a direct storage of the fluid inside the discharger itself. By providing an intermediate section comprising a proximal portion having a receiving space, a separate container containing an amount of fluid to be discharged can be loaded into the discharger. Consequently, a practically unlimited storage life of the discharger itself can be achieved. Moreover, this concept allows for an easy and reliable operation of the discharger since the flow connection for the fluid from the container being loaded into the receiving space of the proximal portion of the intermediate section to the inlet opening of the discharge device can be automatically established by the activation means.

Expediently, the flow connection can be established automatically after breakage of the breakable section by moving the proximal portion of the intermediate section relative to the distal portion of the intermediate section along the longitudinal axis from the starting position towards the distal end of the discharge section and towards the final discharge position.

In the initial position, i.e. before breakage of the breakable section, and/or in the starting position of the proximal portion of the intermediate section, the activation means preferably can be inoperative, meaning that no connection to the fluid to be discharged can be established.

Advantageously, the activation means can establish the flow connection when the proximal portion of the intermediate section is on its way between the starting position and the final discharge position so that the fluid can begin to flow through the discharge passage of the discharge section before the proximal portion can reach the final discharge position.

In another embodiment, the activation means can establish the flow connection when the proximal portion of the intermediate section is brought from its initial position to its starting position, i.e. the fluid connection can be established during the breakage of the breakable section.

It is also conceivable if the activation means may require an additional operation step to be performed by a user in order to actually establish the flow connection. However, it is particularly preferred if the flow connection can be established automatically by moving the proximal portion of the intermediate section towards the final discharge position.

In still a further embodiment, the activation means can be positioned at least partially within the proximal portion of the intermediate section so as to protrude into the receiving space and beyond the receiving space when the proximal portion is being moved from the starting position towards the final discharge position. By protruding into the receiving space defined by the proximal portion and further beyond this receiving space, i.e. beyond a receiving end of the proximal portion, the activation means can be able to interact with the container loaded into the receiving space, for instance by breaking a seal of the container.

In this context, it is preferred if the activation means may be provided with means for breaking a seal, in particular for piecing, penetrating, puncturing and/or perforating the seal.

The activation means preferably may comprise a piercing tip having a flow channel for establishing the flow connection for the fluid from the receiving space to the inlet opening of the discharge section. Advantageously, the outer surface of the piercing tip can have a convexly curved, in particular a dome-shaped, configuration and preferably can be rotationally symmetric. The flow channel preferably can be centrally located within the piercing tip, in particular along a rotary axis of the piercing tip. However, the flow channel can also be located at an offset position relative to the rotary axis of the piercing tip. It is particularly preferred if the piercing tip can be rotationally symmetric about the longitudinal axis defined by the discharge section.

According to another embodiment, the piercing tip may comprise a ridge at an apex, wherein the ridge preferably can be perpendicular to the longitudinal axis. The ridge, however, can also be inclined or slanted relative to the longitudinal axis. Expediently, the ridge can be interrupted by the flow channel such that two protrusions adjacent to the flow channel are formed. In other words, the flow channel can divide the ridge into two separated ridge-like protrusions which preferably may have about the same dimensions.

In an embodiment, the activation means can be sealingly fitted within the proximal portion of the intermediate section so as to prevent at least substantially fluid leakage through the receiving end of the distal portion of the intermediate section. In this context, it is particularly preferable if the activation means or the distal portion of the intermediate section may comprises a sealing lip or the like providing a seal against an inner wall of the proximal portion of the intermediate section.

Advantageously, the distal portion is adapted to advance inside (i.e. into) the proximal portion after the breakage of the breakable section, wherein a sealing connection is created between the distal portion and the proximal portion. When advancing inside the proximal portion (after the breakage of the breakable section), the distal portion can act as a piston that displaces the fluid stored in the separate container. Due to the sealing connection, an undesired leakage of the container and/or the proximal portion is prevented.

Expediently, the sealing lip, the sealing connection or the like may be provided at the activation means, in particular at the piercing tip, preferably at an outer surface region before the piercing tip starts tapering. The sealing lip may me integrally formed with the activation means which advantageously eliminates the need for separate sealing elements, such as gaskets or O-rings. Thus, an effective concept is provided which ensures that the fluid from the container flows at least substantially only into the inlet opening and through the discharge passage of the discharge device.

In a preferred embodiment, the distal portion of the intermediate section may consist of a first essentially cylindrical portion protruding from the proximal portion of the intermediate section and a second portion functioning as the activation means. According to this embodiment, the activation means preferably may be entirely enclosed by the proximal portion of the intermediate section. Further, the first cylindrical portion protruding from the proximal portion may comprise the sealing lip. The breakable portion according to this embodiment preferably can have the shape of a web and may be located at a distal end facing away from the receiving space of the proximal portion where the first cylindrical portion of the distal portion starts to protrude.

In a further embodiment, the activation means can be shaped at least approximately complementary to an inner wall portion of the container adapted to be loaded within the receiving space so as to minimize the residual amount of fluid contained within the container when the proximal portion of the intermediate section is in its final discharge position. In other words, an inner contour of the container essentially may be complementary in shape to the activation means being preferably in the shape of a piercing tip.

The expression "essentially", in this context, implies that the shape of the inner wall portion of the container can deviate from an exact complementary shape of the activation means, especially in an area where the activation means may comprise a ridge or ridge-like protrusions, if applicable.

This design allows for a nearly complete discharge of the fluid since the space available for the fluid can be completely filled by the activation means resulting in a nearly complete displacement of the fluid.

The proximal portion of the intermediate section preferably can have a receiving end with an insertion opening, wherein the receiving space can be accessible through the insertion opening. Further, the proximal portion preferably can be adapted to be loaded with the container by sliding the container through the insertion opening into the receiving space. The receiving space preferably may be dimensioned such that the container at least partially protrudes from the proximal portion.

By keeping the part of the container being inserted into the receiving space as small as possible, the use of material can be kept down to a minimum since according to the present invention the container needs no further encasement. In this context, preferably only one third, more preferably only one fourth, in particular only one tenth of the total length of the container may be received into the receiving space defined by the proximal portion of the intermediate section.

Expediently, inside the proximal portion of the intermediate section a transition portion may be provided which serves as a stop for the container being loaded into the receiving space. This transition portion may be in the shape of a shoulder or an abutment and can determine the length with which the container is received into the receiving space.

The container preferably can cooperate with the receiving space of the proximal portion of the intermediate section by means of a plug connection or a snap-in connection as has previously been described with regard to the discharge section cooperating with the distal portion of the intermediate section.

In an embodiment, the discharger further may comprise the container, in particular in the form of a cartridge or a capsule, wherein the container can include the predetermined amount of fluid to be discharged by the discharger.

The outer shape of the container may be generally cylindrical and the fluid to be discharged preferably can be contained in a fluid reservoir within the container.

Expediently, the fluid inside the container can be protected from environmental influences by a breakable seal, in particular, a barrier foil. The container may have a distal end face at least part of which being formed by a breakable seal.

The seal may be formed by a portion of an outer wall of the container, wherein preferably the seal portion may have a thickness smaller than that of other portions of the wall. Alternatively, the outer wall of the container can define an opening which, after filling the fluid into the container, may be closed by a separate sealing element. The sealing element may be in the form of a foil or a film.

In an embodiment, the discharge section can comprise projections, such as finger flanges, finger rests or finger grips, in particular being moulded on or being integrally formed with the discharge section.

The discharger thus can be used like a common syringe by holding the discharger with one finger at each projection and with the thumb on a proximal end of the container being loaded into the receiving space of the proximal portion of the intermediate section, thereby providing for a comfortable single-hand operation of the discharger. The container in this way can be activated like a push button which is to be pressed by a user with her or his thumb while counteracting this pressing actuation by holding the discharge section with two fingers behind the projections.

In this way, an axial force towards the distal end of the discharge section can be applied to the proximal portion of the intermediate section. As a consequence, the breakable section can break and the proximal portion can be transferred into the starting position. By further applying the axial force, the proximal portion together with the container being loaded in the receiving space of the proximal portion can be moved further towards the distal end of the discharge section. Thereby, the activation means of the distal portion of the intermediate section can establish a flow connection for the fluid from within the receiving space to the inlet opening of the discharge section, preferably by breaking the seal located at a distal end face of the container. As the activation means enters into the fluid reservoir of the container, the fluid can be urged out of the container through the discharge passage of the discharge section.

By directly providing the projections on the discharge section, the provision of additional housing parts can be omitted. The rate of material consumption in the production thus is correspondingly low. Moreover, since during operation of the discharger, on the one hand, the discharge section can be pressed against the distal portion of the intermediate section and, on the other hand, the container can be pressed against the proximal portion of the intermediate section, a slip-tight connection between the above-named separate components - discharge section, intermediate section, container - is not necessarily required. This particularly facilitates on-site assembly and handling of the discharger.

In a further embodiment, the discharge section may comprise a distal end portion defining the outlet opening. Preferably, the distal end portion can be formed integrally or can be made as a separate component, such as a spray head, a needle, a brush, a sponge or a pipette, being connectable to the distal end of the discharge section. For large area applications, for instance, a spray head or a sponge can be of advantage, whereas selective applications might require a needle or a plain tube of small diameter.

In another aspect, the present disclosure also relates to the use of a discharger as disclosed herein for discharging a liquid including at least one medical, dental or veterinary agent, wherein in particular the amount of the liquid can lie in the range of 0.1 to 5 ml and preferably can be approximately 0.5 ml.

In general, the volume for the fluid within the container may be dimensioned as desired in according with the respective intended used. The outer shape and the outer dimensions of the container may be the same for different volumes and thus for different quantities of fluid included within the container.

Such an applicator nasal decolonization using a fluid comprises an application tip adapted for insertion into a nasal cavity, the application tip being configured to store and release said fluid, and a support to which the application tip is connected, wherein the application tip is formed from at least one of an open-cell foam, a plastic, a flock (a material having fibers and that is often interwoven), cotton, polyester, rayon, calcium alginate, foam, knitted polyester, a medical grade plastic, a thermoplastic elastomer, low density polyethylene, acrylonitrile butadiene styrene, polypropylene, polyethylene, polycarbonate, polyurethane, polyetheretherketone, and polyvinyl alcohol.

In this connection it should be noted that the application tip has to be shaped complementary to the shape of a nasal cavity such that on insertion of the application tip into the nasal cavity the tissue of the nasal cavity is not damaged simply due to a poorly shaped design of the application tip which could possibly lead to further undesired infections.

It should further be noted that the application tip is configured to store and release said fluid. This means that the application tip must have a capacity for being wetted in order to transfer a fluid from e.g. a container to the nasal cavity and then be able to release the fluid inside the nasal capacity.

In this connection it should be noted that the application tip may have a surface absorbability selected in the range of 10 to 30 mm², in particular in the range of 15 to 26 mm².

Such an absorption permits storage of the fluid at the application tip in such a way that this acts as a foam.

It should further be noted that an ethanol permeation time of at least a part of the application tip is in the range of 10 to 200 s, and a maximum diameter is 50 µm or more within the range of 1.0 mm × 1.0 mm of the cross section. In particular wherein the application tip comprises a polyurethane elastic body containing 20 or more pores of 300 µm or less, especially wherein the pores of the continuous pore elastic body have a substantially circular or elliptical cross-sectional shape. Such permeation times permit the release of a fluid stored at the application tip. Moreover, they are best achieved using a polyurethane elastic body having the above-mentioned properties.

The measurement of the permeation rate of alcohols is defined in ASTM F739 and EN 374. Moreover, the water vapor transmission of materials and the water vapor permeability are defined in the following standards ASTM International (2016) ASTM E96/E96M-16-Standard test methods for water vapor transmission of materials. In: ASTM International West Conshohocken, PA and for water vapor permeability *DIN* 53122; the European standard, EN 1015-19:1998/A1:2004
Similarly, ASTM D570 - defines the Standard *Test Method* for *Water Absorption* of *Plastics, as does* ISO 62:2008 that specifies the plastics - Determination of water absorption. ISO 175:2010(en) Plastics - defines methods of test for the determination of the effects of immersion in liquid chemicals.

In this connection it should be noted that a material of the application tip when present in the shape of a cylinder or at least generally cylindrical in shape with a size selected with a diameter of 3 mm and a length of 5 mm can absorb between 6 and 16 mg of a fluid, in particular between 8 and 12 mg of a fluid depending on the viscosity of the fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 135 mm² and a surface tension selected in the range of 28 to 35 mN/m at 20°C can absorb between 9 and 35 mg of a fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 135 mm² and a surface tension selected in the range of 28 to 35 mN/m at 20°C can release between 6 and 13 mg of said fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 57 mm² and a surface tension selected in the range of 28 to 35 mN/m at 20°C can absorb between 2 and 35 mg of a fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 57 mm² and a surface tension selected in the range of 28 to 35 mN/m at 20°C can release between 0.3 and 22 mg of said fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

Such materials can be successfully used to provide an applicator that avoids damage to the nasal cavity.

In this connection it should be noted that e.g. the use of an open-cell foam, provides a foam that consists mainly of interconnected cells which share some of their structure with other cells. This type of foam is softer and more flexible than closed-cell foam, and allows gas and vapor to move freely through the cells, and absorbs liquid when immersed in it.

Other kinds of plastic that can be used for the application tip, such as HDPE and PP, make available a medical grade plastic as an application tip that is known to be safe to use with such applicators and which also make available an application tip having the desired softness and flexibility which avoids damage to the nasal cavity.

In this connection it should be noted that the application tip may have an average value of Young's modulus selected 0.5 to 500 kPa, preferably in the range of 1 to 300 kPa, especially of 5 to 250 kPa, especially of 8 to 10 kPa and most preferably of 9.3 kPa.

Methods of measuring the softness of plastics, such as e.g. open-cell polyurethane foams, are well known and are defined in ISO 527-1:2019(en)Plastics - Determination of tensile properties.

A hardness of a material of the application tip may be selected in the range of 25 to 95, in particular of 40 to 60, measured on the Shore hardness scale A, e.g. for thermoplastic elastomers.

A hardness of a material of the application tip may be selected in the range of 35 to 50, in particular of 40 to 48, especially of 44 to 46, measured on the Shore hardness scale D, e.g. LDPE.

An end of the application tip at least partly may have at least one of a conical outer shape, an at least substantially conical outer shape, a truncated cone shape, an at least substantially truncated cone shape, a rounded shape, an at least substantially rounded shape, a spherical shape, an at least substantially spherical shape, an oval shape, an at least substantially oval shape, rounded edges and combinations of the foregoing, i.e. a shape which increases in size between the end and a middle portion of the application tip.

By means of such shapes an application tip may be provided that is formed complementary to the shape of a nasal cavity and hence reduce the risk of injury to the nasal cavity.

The applicator may be a 2K applicator, with the application tip being formed from a first material and the support being formed from a second material, optionally with the application tip being formed directly at the support in a two-step manufacturing process. In this way the support can be formed harder than the application tip with the application tip optionally being formed integrally thereat.

The application tip may be formed from a high-density polyethylene, optionally wherein the high-density polyethylene is sintered and has a porous volume selected in the range of 40 to 60 %, preferably in the range of 45 to 55 %, with a pore size ranging in the range of 80 to 300 µm, in particular in the range of 100 to 250 µm. Such sintered porous plastic materials can reliably store and release fluids and are soft enough to avoid damage to the nasal cavity.

The applicator may further comprise a container filled with a fluid, such as a liquid, wherein the application tip is configured to be wetted with the liquid stored in the container, preferably the container being configured to store between 0.2 and 200 ml of a fluid. In this way the fluid to be applied can be stored in a container that is preferably sealed with regard to the environment to provide an applicator that can be stored for longer periods of time and that avoids spillage and contamination of the fluids stored therein. Moreover, the container provides a volume that can be filled to a high precision. Nasal applications typically require between 0.2 and 10 ml of fluid for an effective treatment, thus providing a single use container with a filling volume of 0.2 to 10 ml is desirable as this enables such amounts of liquids to be dispensed is benefifical. Similarly, for multi-use applications it is desirable to make available containers having a filling volume selected in the range of 10 to 200 ml.

Hydrophilic properties can be considered during the production of applicator foams. In this connection the application tip may comprise a foam having a density ranging from 30kg/m³ to 150kg/m³, and a porosity in the range of 60ppi to 150ppi. Such foams are suited for application in the treatment of nasal decolonization.

The application tip may comprise a polyurethane elastic body containing 20 or more pores of 300 µm or less. Such a foam can be used to reliable store fluids and to release the fluids therefrom.

The applicator may further comprise a dispenser, wherein the support is a rod comprising a passage and the passage is connected to or integrally formed with an outlet of the dispenser for dispensing liquids from the container of the dispenser to the application tip for a supply of the fluid to the application tip. The provision of a dispenser at the applicator enables a pre-determined amount of fluid to be dispensed via the application tip in a well-defined manner.

The dispenser may comprise a housing defining a longitudinal axis and having a proximal end and a distal end, a discharge device arranged at least partly inside the housing and having an inlet opening situated inside the housing and an application tip, the discharge device further defining a discharge passage for the fluid extending between the inlet opening and the application tip, a carriage as the container received in a proximal end region of the housing and being moveable relative to the housing along the longitudinal axis between a starting position and a final discharge position, and wherein the carriage defines a receiving space which is adapted to be loaded with a separate container holding an amount of fluid to be discharged, and activation means for establishing a flow connection for the fluid from within the receiving space of the carriage to the inlet opening of the discharge device, wherein the activation means are adapted to establish the flow connection when the carriage is in the final discharge position or is being moved towards the final discharge position. Such a dispenser can be used to disperse the fluid stored in a separate container in a simple and sterile manner. Moreover, the container insertable into the carriage provides a volume that can be filled to a high precision. The dispenser permits this pre-defined volume to be dispensed in one go such that the pre-defined amount of material can be dispensed in one go, particularly if the dispenser is of single-use design.

In the starting position, the carriage may protrude from the proximal end of the housing so as to be pushable by a user further into the housing and towards the final discharge position, and/or wherein the activation means are inoperative when the carriage is in the starting position.

The flow connection may be established automatically by moving the carriage towards the final discharge position and/or before the carriage reaches the final discharge position. In this way a dispenser that is simple to handle und use can be realized.

The dispenser may be a syringe. Such a dispenser is simple to realize, cost effective in manufacture and easy to handle.

The syringe may comprise a plunger and a barrel in which the fluid can be stored, with the plunger acting on the fluid during a dispensing of the fluid from the barrel via the application tip. Such a design of the syringe is simple and easy to use.

The dispenser may be an applicator device comprising: a carrier body provided with the application tip at one end and a pin at an opposing end; a transverse channel penetrating the pin; an axial channel branching off from the transverse channel and leading to the applicator device; and a reservoir device having a ring-shaped edge seal sealingly engaging the pin, whereby the applicator device is activated by displacing the reservoir device on the pin toward the application device to open a flow connection between the reservoir device and the transverse channel, the reservoir device having a receptacle segment that is elastically deformable at least in portions so that, when the applicator device is activated and a flow connection between the transverse channel and the receptacle segment exists, the fluid is discharged via the application tip by manual compression of the elastically deformable area of the receptacle segment. Providing a dispenser with an elastically deformable part which can be used to urge the fluid from the dispenser avoids spillage of the fluid and permits the dispensing of a pre-defined amount of fluid.

The rod may be a solid rod and the solid rod may be connected to a handle of the applicator. Thereby a versatile and simple to use applicator can be provided that utilizes a well-known and hence simple to manufacture closure.

The applicator may further comprise a container, optionally filed with said fluid, with the application tip being stored in the container and optionally in said fluid prior to use of the application tip. In this way the fluid can be stored with the applicator in a safe and reliable way.

The handle is optionally a part of a closure for the container and the application tip and the rod may be insertable into and removable from the container. Thereby a versatile and simple to use applicator can be provided that utilizes a well-known and hence simple to manufacture closure.

The handle may be releasably screwed to the container. Thereby a versatile and simple to use applicator can be provided that utilizes a well-known and hence simple to manufacture closure.

The handle may be a part of the support. Such a simplistic design is simple to manufacture and leads to a cost-effective applicator.

The container may comprise a membrane with the fluid being stored directly adjacent to the membrane and the application tip being stored at the other side of the membrane.

The support may comprise one or more seals configured to seal against an inner surface of the container. Such seals can avoid contamination from entering the applicator and/or can prevent fluids from exiting the applicator.

The container may be a multi-part container having an inner container arranged within an outer container. Materials stored in such a container can be protected from external contamination leading to a prolonged shelf-life and a sterile packaging of the applicator.

Such an applicator nasal decolonization using a fluid comprises an application tip adapted for insertion into a nasal cavity, the application tip being configured to store and release said fluid, and a support to which the application tip is connected, wherein the application tip is formed from at least one of an open-cell foam, a plastic, a flock (a material having fibers and that is often interwoven), cotton, polyester, rayon, calcium alginate, foam, knitted polyester, a medical grade plastic, a thermoplastic elastomer, low density polyethylene, acrylonitrile butadiene styrene, polypropylene, polyethylene, polycarbonate, polyurethane, polyetheretherketone, and polyvinyl alcohol.

In this connection it should be noted that the application tip has to be shaped complementary to the shape of a nasal cavity such that on insertion of the application tip into the nasal cavity the tissue of the nasal cavity is not damaged simply due to a poorly shaped design of the application tip which could possibly lead to further undesired infections.

It should further be noted that the application tip is configured to store and release said fluid. This means that the application tip must have a capacity for being wetted in order to transfer a fluid from e.g. a container to the nasal cavity and then be able to release the fluid inside the nasal capacity.

In this connection it should be noted that the application tip may have a surface absorbability selected in the range of 10 to 30 mm², in particular in the range of 15 to 26 mm².

Such an absorption permits storage of the fluid at the application tip in such a way that this acts as a foam.

It should further be noted that an ethanol permeation time of at least a part of the application tip is in the range of 10 to 200 s, and a maximum diameter is 50 µm or more within the range of 1.0 mm × 1.0 mm of the cross section. In particular wherein the application tip comprises a polyurethane elastic body containing 20 or more pores of 300 µm or less, especially wherein the pores of the continuous pore elastic body have a substantially circular or elliptical cross-sectional shape. Such permeation times permit the release of a fluid stored at the application tip. Moreover, they are best achieved using a polyurethane elastic body having the above-mentioned properties.

The measurement of the permeation rate of alcohols is defined in ASTM F739 and EN 374. Moreover, the water vapor transmission of materials and the water vapor permeability are defined in the following standards ASTM International (2016) ASTM E96/E96M-16-Standard test methods for water vapor transmission of materials. In: ASTM International West Conshohocken, PA and for water vapor permeability *DIN* 53122; the European standard, EN 1015-19:1998/A1:2004
Similarly, ASTM D570 - defines the Standard *Test Method* for *Water Absorption* of *Plastics, as does* ISO 62:2008 that specifies the plastics - Determination of water absorption. ISO 175:2010(en) Plastics - defines methods of test for the determination of the effects of immersion in liquid chemicals.

In this connection it should be noted that a material of the application tip when present in the shape of a cylinder or at least generally cylindrical in shape with a size selected with a diameter of 3 mm and a length of 5 mm can absorb between 6 and 16 mg of a fluid, in particular between 8 and 12 mg of a fluid depending on the viscosity of the fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 135 mm² and a surface tension selected in the range of 28 to 35 mN/m at 20°C can absorb between 9 and 35 mg of a fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 135 mm² and a surface tension selected in the range of 28 to 35 mN/m at 20°C can release between 6 and 13 mg of said fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 57 mm² and a surface tension selected in the range of 28 to 35 mN/m at 20°C can absorb between 2 and 35 mg of a fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

In this connection it should further be noted that an application tip having a surface area of 57 mm² and a surface tension selected in the range of 28 to 35 mN/m at 20°C can release between 0.3 and 22 mg of said fluid, with the fluid optionally being selected from the group of members comprising nail varnish remover, water, oil, alcohol, e.g. ethanol, and salt water and/or the fluid having a viscosity selected in the range of 0.5 to 200000 mPas at 20°C.

Such materials can be successfully used to provide an applicator that avoids damage to the nasal cavity.

In this connection it should be noted that e.g. the use of an open-cell foam, provides a foam that consists mainly of interconnected cells which share some of their structure with other cells. This type of foam is softer and more flexible than closed-cell foam, and allows gas and vapor to move freely through the cells, and absorbs liquid when immersed in it.

Other kinds of plastic that can be used for the application tip, such as HDPE and PP, make available a medical grade plastic as an application tip that is known to be safe to use with such applicators and which also make available an application tip having the desired softness and flexibility which avoids damage to the nasal cavity.

In this connection it should be noted that the application tip may have an average value of Young's modulus selected 0.5 to 500 kPa, preferably in the range of 1 to 300 kPa, especially of 5 to 250 kPa, especially of 8 to 10 kPa and most preferably of 9.3 kPa.

Methods of measuring the softness of plastics, such as e.g. open-cell polyurethane foams, are well known and are defined in ISO 527-1:2019(en)Plastics - Determination of tensile properties.

A hardness of a material of the application tip may be selected in the range of 25 to 95, in particular of 40 to 60, measured on the Shore hardness scale A, e.g. for thermoplastic elastomers.

A hardness of a material of the application tip may be selected in the range of 35 to 50, in particular of 40 to 48, especially of 44 to 46, measured on the Shore hardness scale D, e.g. LDPE.

An end of the application tip at least partly may have at least one of a conical outer shape, an at least substantially conical outer shape, a truncated cone shape, an at least substantially truncated cone shape, a rounded shape, an at least substantially rounded shape, a spherical shape, an at least substantially spherical shape, an oval shape, an at least substantially oval shape, rounded edges and combinations of the foregoing, i.e. a shape which increases in size between the end and a middle portion of the application tip.

By means of such shapes an application tip may be provided that is formed complementary to the shape of a nasal cavity and hence reduce the risk of injury to the nasal cavity.

The applicator may be a 2K applicator, with the application tip being formed from a first material and the support being formed from a second material, optionally with the application tip being formed directly at the support in a two-step manufacturing process. In this way the support can be formed harder than the application tip with the application tip optionally being formed integrally thereat.

The application tip may be formed from a high-density polyethylene, optionally wherein the high-density polyethylene is sintered and has a porous volume selected in the range of 40 to 60 %, preferably in the range of 45 to 55 %, with a pore size ranging in the range of 80 to 300 µm, in particular in the range of 100 to 250 µm. Such sintered porous plastic materials can reliably store and release fluids and are soft enough to avoid damage to the nasal cavity.

The applicator may further comprise a container filled with a fluid, such as a liquid, wherein the application tip is configured to be wetted with the liquid stored in the container, preferably the container being configured to store between 0.2 and 200 ml of a fluid. In this way the fluid to be applied can be stored in a container that is preferably sealed with regard to the environment to provide an applicator that can be stored for longer periods of time and that avoids spillage and contamination of the fluids stored therein. Moreover, the container provides a volume that can be filled to a high precision. Nasal applications typically require between 0.2 and 10 ml of fluid for an effective treatment, thus providing a single use container with a filling volume of 0.2 to 10 ml is desirable as this enables such amounts of liquids to be dispensed is benefifical. Similarly, for multi-use applications it is desirable to make available containers having a filling volume selected in the range of 10 to 200 ml.

Hydrophilic properties can be considered during the production of applicator foams. In this connection the application tip may comprise a foam having a density ranging from 30kg/m³ to 150kg/m³, and a porosity in the range of 60ppi to 150ppi. Such foams are suited for application in the treatment of nasal decolonization.

The application tip may comprise a polyurethane elastic body containing 20 or more pores of 300 µm or less. Such a foam can be used to reliable store fluids and to release the fluids therefrom.

The applicator may further comprise a dispenser, wherein the support is a rod comprising a passage and the passage is connected to or integrally formed with an outlet of the dispenser for dispensing liquids from the container of the dispenser to the application tip for a supply of the fluid to the application tip. The provision of a dispenser at the applicator enables a pre-determined amount of fluid to be dispensed via the application tip in a well-defined manner.

The dispenser may comprise a housing defining a longitudinal axis and having a proximal end and a distal end, a discharge device arranged at least partly inside the housing and having an inlet opening situated inside the housing and an application tip, the discharge device further defining a discharge passage for the fluid extending between the inlet opening and the application tip, a carriage as the container received in a proximal end region of the housing and being moveable relative to the housing along the longitudinal axis between a starting position and a final discharge position, and wherein the carriage defines a receiving space which is adapted to be loaded with a separate container holding an amount of fluid to be discharged, and activation means for establishing a flow connection for the fluid from within the receiving space of the carriage to the inlet opening of the discharge device, wherein the activation means are adapted to establish the flow connection when the carriage is in the final discharge position or is being moved towards the final discharge position. Such a dispenser can be used to disperse the fluid stored in a separate container in a simple and sterile manner. Moreover, the container insertable into the carriage provides a volume that can be filled to a high precision. The dispenser permits this pre-defined volume to be dispensed in one go such that the pre-defined amount of material can be dispensed in one go, particularly if the dispenser is of single-use design.

In the starting position, the carriage may protrude from the proximal end of the housing so as to be pushable by a user further into the housing and towards the final discharge position, and/or wherein the activation means are inoperative when the carriage is in the starting position.

The flow connection may be established automatically by moving the carriage towards the final discharge position and/or before the carriage reaches the final discharge position. In this way a dispenser that is simple to handle und use can be realized.

The dispenser may be a syringe. Such a dispenser is simple to realize, cost effective in manufacture and easy to handle.

The syringe may comprise a plunger and a barrel in which the fluid can be stored, with the plunger acting on the fluid during a dispensing of the fluid from the barrel via the application tip. Such a design of the syringe is simple and easy to use.

The dispenser may be an applicator device comprising: a carrier body provided with the application tip at one end and a pin at an opposing end; a transverse channel penetrating the pin; an axial channel branching off from the transverse channel and leading to the applicator device; and a reservoir device having a ring-shaped edge seal sealingly engaging the pin, whereby the applicator device is activated by displacing the reservoir device on the pin toward the application device to open a flow connection between the reservoir device and the transverse channel, the reservoir device having a receptacle segment that is elastically deformable at least in portions so that, when the applicator device is activated and a flow connection between the transverse channel and the receptacle segment exists, the fluid is discharged via the application tip by manual compression of the elastically deformable area of the receptacle segment. Providing a dispenser with an elastically deformable part which can be used to urge the fluid from the dispenser avoids spillage of the fluid and permits the dispensing of a pre-defined amount of fluid.

The rod may be a solid rod and the solid rod may be connected to a handle of the applicator. Thereby a versatile and simple to use applicator can be provided that utilizes a well-known and hence simple to manufacture closure.

The applicator may further comprise a container, optionally filed with said fluid, with the application tip being stored in the container and optionally in said fluid prior to use of the application tip. In this way the fluid can be stored with the applicator in a safe and reliable way.

The handle is optionally a part of a closure for the container and the application tip and the rod may be insertable into and removable from the container. Thereby a versatile and simple to use applicator can be provided that utilizes a well-known and hence simple to manufacture closure.

The handle may be releasably screwed to the container. Thereby a versatile and simple to use applicator can be provided that utilizes a well-known and hence simple to manufacture closure.

The handle may be a part of the support. Such a simplistic design is simple to manufacture and leads to a cost-effective applicator.

The container may comprise a membrane with the fluid being stored directly adjacent to the membrane and the application tip being stored at the other side of the membrane.

The support may comprise one or more seals configured to seal against an inner surface of the container. Such seals can avoid contamination from entering the applicator and/or can prevent fluids from exiting the applicator.

The container may be a multi-part container having an inner container arranged within an outer container. Materials stored in such a container can be protected from external contamination leading to a prolonged shelf-life and a sterile packaging of the applicator.

The fluid may be stored in the applicator and comprises at least one of the following substances: a medical fluid, a dental fluid, a veterinary fluid, an antiseptic substance, an antihistamine, an glucocorticoid, epinephrine, a mast cell stabilizer, an antileukotriene agent, povidone-iodine, mupirocin, alcohol, jojoba, water, orange oil, lauric acid, benzalkonium chloride, vitamin E, hypothiocyanite, lactoferrin, N-chlorotaurine, interferon-alpha, povidone-iodine, quaternary ammonium compounds, alcohol-based nasal antiseptics, hydroxychloroquine, galphimia glauca, luffa operculata, sabadilla and combinations thereof.

Fluids containing such substances can be successfully used in applications for treatment of the nose of a human or animal and in particular in relation to nasal decolonization.

The following drawings illustrate the various embodiments of the present disclosure and together with the description serve to explain the principles and operations of the claimed subject matter:
- Fig. 1: a perspective view of a discharger in a storage state;
- Fig. 2: a perspective view of the discharger of Fig. 1 in a discharged state;
- Fig. 3: a part sectional view of the discharger in the state of Fig. 1;
- Fig. 4: a part sectional view of the discharger in the state of Fig. 2;
- Fig. 5: a sectional view of a further type of static piston of the discharger;
- Figs. 6 to 8: show a discharge system comprising a discharger according to the present disclosure in different states of use;
- Fig. 9: shows a partial cross-section along the longitudinal first axis of the discharge system of Fig. 6;
- Fig. 10: shows an enlarged portion of Fig. 9;
- Fig. 11: shows a partial cross-section along the longitudinal first axis of a proximal portion of the discharge system of Fig. 8;
- Figs. 12 to 14: respectively show a first cross-section of a piercing tip ac cording to the present technology and a side view of the piercing after a rotation by 90°;
- Fig. 15: shows a perspective view of a piercing tip having a ridge;
- Figs. 16 to 18: show a discharge system comprising a discharger and a container according to the present disclosure in different states of use;
- Fig. 19: shows a sectional view along the longitudinal axis of the discharge system of Fig. 16;
- Fig. 20: shows an enlarged portion of Fig. 19;
- Fig. 21: shows a sectional view along the longitudinal axis of a proximal portion of the discharge system of Fig. 18;
- Fig. 22: a schematic representation of a first embodiment of a syringe in accordance with the invention in a storage position;
- Fig. 23: a schematic representation of the first embodiment in the release position;
- Fig. 24: a schematic representation of the first embodiment in an end position after discharging the first fluid and the second fluid;
- Fig. 25: a schematic representation of the first embodiment in a draw-in position;
- Fig. 26: a schematic representation of a second embodiment in the storage position;
- Fig. 27: a schematic representation of a third embodiment in the storage position;
- Fig. 28: a schematic representation of a fourth embodiment in the storage position;
- Fig. 29: a schematic representation of a fifth embodiment in the storage position;
- Fig. 30: shows a perspective view of a discharger comprising a container according to the present disclosure;
- Fig. 31: shows a cross-sectional view along the longitudinal axis of the discharger of Fig. 1;
- Fig. 32: shows the discharger of Fig. 31 with the proximal portion of the intermediate section in the final discharge position.
- Fig.33: shows a first exemplary embodiment of a dispensing device according to the technology in a perspective view;
- Fig. 34: shows an enlarged detail of Fig. 33;
- Fig. 35: shows the mixing and spray head of Fig. 34 in the assembled condition;
- Fig. 36: shows a section according to line IV - IV in Fig. 35,
- Fig. 36A: shows a partial view of an embodiment variant of the mixing and spray piece of Fig. 36 with a compressed air inlet,
- Fig. 37: shows a section according to line V - V in Fig. 36,
- Fig. 38: shows a section according to line VI - VI in Fig. 36,
- Fig. 39: shows a section according to line VII - VII in Fig. 38,
- Fig. 40: shows a second exemplary embodiment of a dispensing device according to the technology in a perspective view,
- Fig. 41: shows a section through the assembled mixing and spray head of Fig. 40,
- Fig. 42: shows a section according to line X - X in Fig. 41;
- Figs. 43 to 45: show an applicator formed as a discharge system comprising a dispenser and a container in different states of use;
- Fig. 46: shows a sectional view along the longitudinal axis of the discharge system of Fig. 43;
- Fig. 47: shows an enlarged portion of Fig. 46;
- Fig. 48: shows a sectional view along the longitudinal axis of a proximal portion of the discharge system of Fig. 45;
- FIG. 49: is a longitudinal section through a first embodiment of an applicator device as an applicator for a single-component system in the deactivation position;
- FIG. 50: shows the applicator device illustrated in FIG. 49 in the activation position,
- FIG. 51: is a longitudinal section through a second embodiment of an applicator device as an applicator for a single-component system in the deactivation position;
- FIG. 52: shows the application position illustrated in FIG. 51 in the activation position;
- Fig. 53: shows a syringe having an application tip;
- Fig. 54: shows a further type of applicator;
- Fig. 55: shows yet a further type of applicator;
- Fig. 56: shows yet a further perspective view of a further kind of discharger;
- Fig. 57: shows a schematic sectional drawing through the discharger of Fig. 56 along the sectional line A:A;
- Fig. 58: shows a detailed view of the second end of the outer housing component of the discharger shown in Fig. 59; and
- Fig. 59: shows a detailed view of a first end of the outer housing component of the discharger shown in Fig. 56.

Further embodiments of the invention are described in the following description of the Figures. The invention will be explained in the following in detail by means of embodiments and with reference to the drawing in which is shown:
In the following the same reference numerals will be used for parts having the same or equivalent function. Any statements made having regard to the direction of a component are made relative to the position shown in the drawing and can naturally vary in the actual position of application.

The various devices shown in the following can each be filled with a fluid F. The Fluid F is a medical fluid, especially a medical fluid comprising lipid nanoparticles. Such fluids F are generally used to prevent viral infections of being ingested by the human body by being applied at or in the intra nasal cavity. Thereby the mucous membrane is coated with a protective layer which degrades over time and provides persons treated with such a medical fluid with protection for said period of time against one or more viral infections.

In this connection it should be noted that a size of the lipid nanoparticles is typically selected in the range of 10 nm to 400 nm, especially in the range of 15 nm to 200 nm. Such a lipid nanoparticle may be constructed as those discussed in the article "Lipid Nanoparticles Potentiate CpG-Oligodeoxynucleotide-Based Vaccine for Influenza Virus" published in Front. Immunol., 09 January 2020 Sec. Vaccines and Molecular Therapeutics Volume 10 - 2019 | https://doi.org/10.3389/fimmu.2019.03018.

It should further be noted that the lipid nanoparticles are present in a solution comprising a polyethylene glycol (PEG), the concentration thereof being selectable in dependence on the desired antibodies and the desired duration of application.

It should further be noted that the lipid nanoparticles are configured to contain antibodies for one or more viral infections, such as cold and/or flu antibodies. The composition may also comprise one or more antibodies for other viral infections.

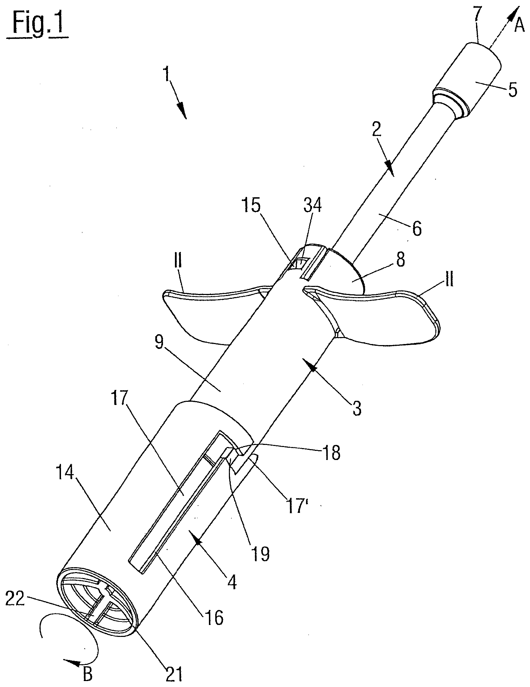

Fig. 1 shows a discharger 1 in a storage state. The discharger 1 comprises a dispensing element 2, a housing 3 and a container 4. The dispensing element 2 extends between a distal end 5 and a proximal end 6 and thereby defines a longitudinal axis A of the discharger 1, with an outlet 7 of the discharger 1 being configured at the distal end 5. The outlet 7 can either be a simple nozzle (not shown) or comprise a specifically designed nozzle (also not shown) that enables specific spraying actions from being undertaken with the discharger 1.

The housing 3 has a distal region 8 and a proximal region 9, with the distal region 8 of the housing 3 adjoining the proximal end 6 of the dispensing element 2. The housing 3 comprises a static piston 10 (see e.g.

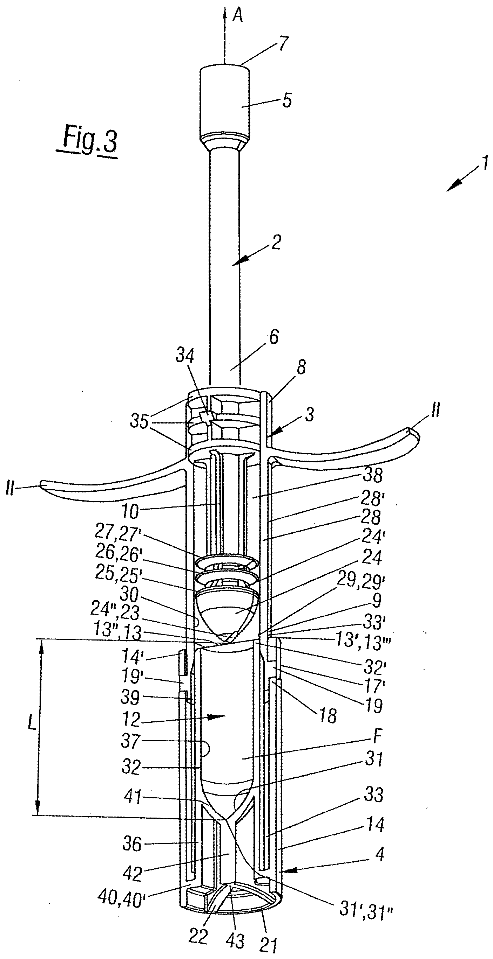

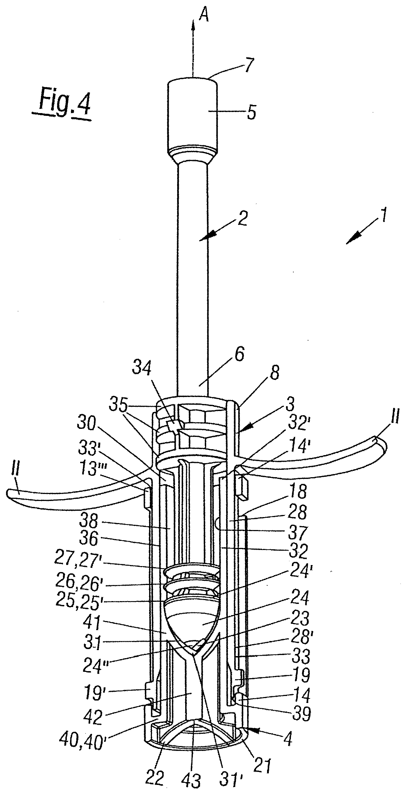

Figs. 3 and 4) arranged within it and has wing-like projections 11 projecting away from the generally cylindrical shaped housing 3 in the distal region 8 of the housing 3.

The housing 3 further comprises a cut-out 15 in the distal region 8. Moreover, a nose 34 can be seen that cooperates with this cut-out. The cut-out 15 and nose 34 form parts of a snap-in connection as will be explained in the following.

The container 4 is arranged at the proximal region 9 of the housing 3. The container 4 comprises a compartment 12 in which a fluid F is stored and sealed off with respect to the static piston 10 by means of a seal 13" that is configured as a membrane 13 (see Fig. 3).

In the present example the outer wall 14 of the container 4 comprises a slot 16 having two longitudinal sections 17, 17' offset in parallel to one another and to the longitudinal axis A and connected to one another via a connection section 18. In the storage state of the discharger 1 shown in Fig. 1, a pin 19 projecting from the proximal region 9 of the housing 3 is present in the connection section 18.

In the present example, the pin 19 is placed in the connection section 18 of the slot 16. By placing the pin 19 in the connection section 18 that does not extend in the direction of the longitudinal axis A an accidental linear displacement of the container 4 relative to the housing 3 and thereby either an accidental removal of the container 4 from the housing 3 or an accidental movement of the container 4 towards the wing-like projections 11 can be prevented. In this way the discharger 1 comprises a child safety lock, with the child safety lock being formed between the container 4 and the housing 3.

On a desired activation of the discharger 1 the container 4 is rotated in the direction of the arrow B so that the pin 19 is then guided into the longitudinal section 17. Once the pin 19 is present in the longitudinal section 17, the container 4 can be pressed towards the wing-like projections 11 in the direction of the longitudinal axis A. A user can carry out the pressing of the container 4 towards the wing-like projections 11 by placing a thumb or a different finger at an end 21 of the housing and two further fingers at each of the wing-like projections 11 and then move these fingers and/or the thumb towards one another in a clamping like manner. The end 21 has a recess 22 formed therein for an improved placement of the finger/thumb at the end 21 of the container 4.

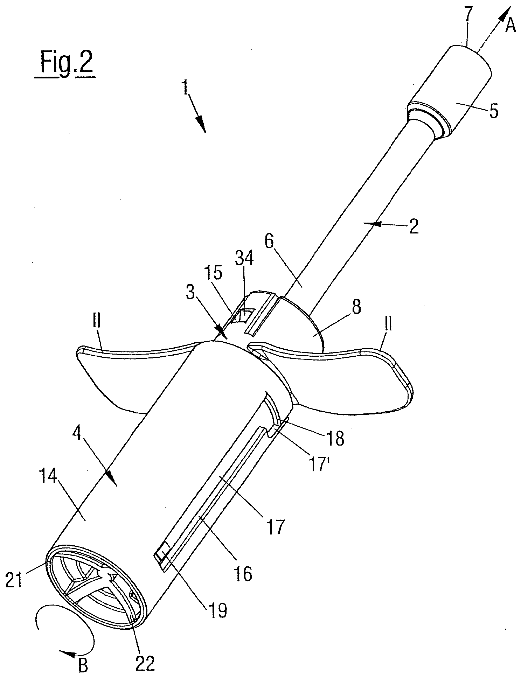

Fig. 2 shows the discharger of Fig. 1 in the discharged state, i.e. the state in which the user has pressed the container 4 from the proximal region 9 of the housing 3 up to the wing-like projections 11 formed at the housing 3. In the discharged state of the discharger 1, the fluid F stored in the compartment 12 has been discharged from the discharger 1 via the outlet 7 (see Fig. 4).

Fig. 3 shows a part sectional view of the discharger 1 shown in the state shown in Fig. 1. This view shows how the static piston 10 is arranged within the housing 3 of the discharger 1. The static piston 10 is connected to the dispensing element 2 in a fluid conducting manner.

For this purpose the static piston 10 has an inlet 23 that leads into a passage (not shown) that connects the inlet 23 with the outlet 7 of the dispensing element 2. The passage thereby extends through the static piston 10 and the dispensing element 2.

The discharger 1 is preferably designed such that the only way a fluid present in the container 4 can pass to the outside via the distal region 8 is via the inlet 23 and the passage, otherwise the housing 3 comprises sealing elements 25', 26', 27', 29' to seal off the housing 3 towards the outside.

The static piston 10 comprises a piercing tip 24 having the inlet 23 formed at one end 24" of the piercing tip 24. The other end 24' of the piercing tip 24 comprises a first sealing lip 25 as a first sealing element 25'. A second sealing lip 26 and a third sealing lip 27 are likewise formed at the static piston 10, as second and third sealing elements 26', 27'. The first, second and third sealing lips 25, 26, 27 are arranged one after the other in parallel to one another in the direction of the longitudinal axis A of the discharger 1. In the storage state of the discharger 1 shown in Fig. 3 the first, second and third sealing lips 25, 26, 27 do not contact a cylindrical wall 28 of the housing 3.

The cylindrical wall 28 of the housing 3 comprises the sealing element 29'. In this instance the sealing element 29' is a sealing lip 29 that circumferentially extends around an inner surface 30 of the cylindrical wall 28 in the proximal region 9 of the housing 3. In the storage state shown in Fig. 3 the sealing lip 29 is an inwardly projecting sealing lip 29. The sealing lip 29 projects, on the one hand, in the direction of the piercing tip 24 arranged around the longitudinal axis A and, on the other hand, into the interior of the housing 3 without engaging a further component. This means that the sealing lip 29 faces the longitudinal axis A of the discharger 1, preferably such that it does not contact nor connect with any further components other than the housing 3 in the storage state of the discharger 1.

It should be noted that a plane comprising the piercing tip 24, the inlet 23 and the end 24" also comprises the sealing element 29'. This plane is arranged perpendicular to the longitudinal axis A.

Fig. 3 also shows a section through the container 4 of the discharger 1 in a plane perpendicular to the longitudinal axis A. As mentioned in connection with Fig. 1, the container 4 comprises the compartment 12 for containing the fluid F in the storage state. The compartment 12 extends from a seal formed by the membrane 13 to a piercing tip receiving end 31, also referred to as an end 31' of the compartment 12, in the direction towards the end 21 of the container 4 comprising the recess 22.

The end 31' of the compartment 12 that is oppositely disposed of the membrane 13 is formed by a wall 41 of the compartment 12. The wall 41 converges from the wall 32 to a common point 31" at the end 31' coinciding with the longitudinal axis A to form the end 31' of the compartment 12. This means that a diameter of the compartment 12 reduces from the wall 32 to the longitudinal axis A along the wall 41.

As also shown the end 31' of the compartment 12 is set back from the rear end 21 of the container 4 by approximately 30% of a length of the outer wall 14. In this connection it should be noted that the end 31' can be set back from the rear end 21 by at least 20%, in particular by 25 to 45%, of the length of the outer wall 14.

A further web 42 is provided in the region of the rear end 21 of the container. This further web 42 extends in parallel to the outer wall 14 between the end 31' of the compartment 12 and the rear end 21 of the container 4. This further web 42 forms a base 43 of the recess 22 at the rear end 21 and hence a base 43 where a finger or thumb can beneficially be placed.

A size of the compartment 12 defines the volume of fluid F that can be stored in the container 4. This means that if a lesser volume of fluid F is to be stored within the compartment 12, then the length L of the compartment 12 can be selected shorter. Consequently, if a greater volume of fluid F is to be stored in the container 4 then a length L of the compartment 12 can be selected longer than in the present case. Hence the volume of fluid F stored in the container 4 corresponds to a space of the container 4.

In this connection it should be noted that typical filling volumes of the compartment 12 of the container 4 are 0.1 to 10 ml, preferably 0.2 to 5 ml.

It should further be noted that a thickness of the wall 32 of the compartment 12 is typically selected in the range of 0.7 to 1.5 mm, preferably in the range of 0.9 to 1.1 mm and especially of around 1 mm. Likewise a thickness of the outer wall 14 of the container 4 is typically selected in the range of 0.7 to 1.5 mm, preferably in the range of 0.9 to 1.1 mm and especially of around 1 mm.

In this connection it should be noted that a thickness of the wall 28 of the housing 3 is typically selected in the range of 0.7 to 1.5 mm, preferably in the range of 0.9 to 1.1 mm and especially of around 1 mm.

It should be noted in this connection that if a compartment 12 of greater volume is selected then a length of the static piston 10 can also be increased in order to ensure that as much as possible of the fluid F initially stored in the compartment 12 is discharged from the discharger 1.

In the embodiment previously described the compartment 12 is integrally formed with the container 4, i.e. the container is a single-part container 4. In a further embodiment a separate compartment in the form of carriage (not shown) having a pre-defined volume could be used to form a two-part or multi-part container (also not shown). The sealing elements 25', 26', 27' are then configured to interact with an inner surface of this multi-part container, i.e. with an inner surface of the carriage.

It should further be noted that the length L of the compartment 12 is defined as the distance between the membrane 13 and the piercing tip receiving end 31.

The shape of the piercing tip receiving end 31 is selected to be complementary to the shape of the piercing tip 24. This is because on discharging the fluid F stored in the compartment 12 of the container 4 as little residue of the fluid F as possible is desired, preferably such that all of the fluid F stored in the container 4 is discharged once the discharger is in the discharged state shown in Fig. 4.

As also shown in Fig. 3, the container 4 is a double walled container comprising an inner wall 32 and the outer wall 14 separated by a, preferably annular, guide groove 33. The inner wall 32 forms a wall of the compartment 12. The guide groove 33 can also be referred to as a ring-like gap that is formed between the inner wall 32 and the outer wall 14 of the double walled container. It should be noted in this connection that the at least one slot 16 extends from the guide groove 33 to an outer surface 14" of the outer wall 14. Preferably the inner wall 32 and the outer wall 14 are injection molded in one piece during the manufacture of the container 4, with a connecting web 40 also being formed during the injection molding process connecting the inner wall 32 to the outer wall 14 at an end 40' of the guide groove 33.

In this connection it should be noted that a length or depth of the guide groove 33 in the longitudinal axis A of the discharger 1 is approximately 90% of a length of the outer wall 14 of the discharger 1 of Fig. 3. It should further be noted that the length of the guide groove 33 can be selected to correspond to 50% to 95% of a length of the outer wall 14.

In the present example the membrane 13 also forms a front end 13‴ of the compartment 12 and thereby of the inner wall 32. The membrane 13 not only forms the front end of the compartment 12, but also defines the front end of the double barreled container 4, as the plane 13' comprising the membrane 13 defines said front end. The plane 13' thereby in addition to the membrane 13 also comprises an end 14' of the outer wall 14 and an end 32' of the wall 32. Thus, the double barreled container 4 extends from the membrane 13 to the end 21, i.e. the rear end 21, having the recess 22 formed therein.

It should be noted in this connection that an opening 33' of the groove is arranged at the front end 13‴ of the container, i.e. the groove 33 is open towards the housing 3 in order to receive the cylindrical wall 28 at the front end 13‴ of the container.

In the storage state of the discharger 1 shown in Fig. 3, the front end of the compartment 12 is received within the housing 3, whereas the outer wall 14 of the double barreled container 4 surrounds the cylindrical wall 28 of the housing 3 in the proximal region 9 thereof. This means that the front end of the container 4 is configured to receive at least the proximal region 9 of the housing 3.

The section through the container 4 of Fig. 3 also shows the presence of a second pin 19' at the other side of the housing 3. This second pin 19' also cooperates with a second slot (not shown) similar to slot 16 present in the double walled container 4.

In the present case the first pin 19 and the second pin 19' are arranged at 180° with respect to one another at an outer surface 28' of the housing 3. In practice dischargers are possible that utilize only one pin and slot arrangement or also more than two pin and slot arrangements. It is also conceivable that the pins are not arranged at 180° with respect to one another at the outer surface 28' of the housing 3, but at a different angle to thereby ensure a correct alignment of the container 4 relative to the housing 3. As can further be seen from Fig. 3 the pins 19, 19' are set back from an end 39 of the housing 3 in the proximal region 9.

On assembly of the discharger 1 the different components are connected to one another. It should be noted in this connection that the static piston 10 and the dispensing element 2 are preferably injection molded in one piece in a common mold in a preferred design of the discharger 1. Alternatively, an over-molding process could also be employed.

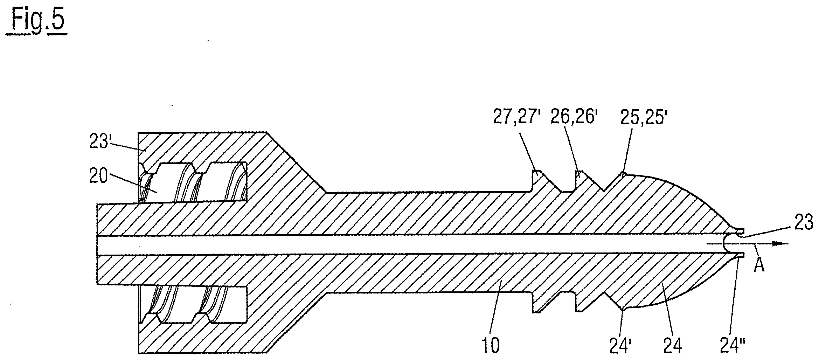

Alternatively the individual components of the discharger 1, e.g. the dispensing element 2, the housing 3, the container 4, the static piston 10, can also be formed separately and then assembled, for example, the static piston 10 and the dispensing element 2 can be connected to one another via a Luer lock connection (see e.g. Fig. 5 in this connection).

In this connection it should be noted that the components of the discharger can be formed from polymeric materials, such as PE (polyethylene), PP (polypropylene) and COC (cyclic olefin copolymers).

For example, the container 4 can be formed by a polymeric material such as COC.

In this connection it should also be noted that like the assembly comprising the static piston 10 and the dispensing element 2, the housing 3 and the container 4 can also be formed in specifically designed molds (not shown) in an injection molding process as separate parts.

The assembly comprising the static piston 10 and the dispensing element 2 is then inserted into the housing 3 via the distal region of the housing 3. The assembly comprising the static piston 10 and the dispensing element 2 is fixed to the inner surface 30 of the housing 3, on the one hand, via snap-in connections, in this respect the snap-in connection is formed by the nose 34 that engages the cut-out 15 (see Fig. 1) present in the cut away section of the housing 3. A second snap-in connection is also present at the other side of the housing 3 that cannot be seen in the depicted section.

On the other hand, the assembly comprising the static piston 10 and the dispensing element 2 is fixed to the inner surface 30 of the housing 3 by means of a press fit. For this purpose three rings 35 are provided at the assembly comprising the static piston 10 and the dispensing element 2. In addition to ensuring a press fit, these three rings 35 also ensure the correct parallel orientation of the static piston 10 within the housing 3, i.e. that the static piston 10 is not inserted at a skew angle into the housing 3.

It should be noted in this connection, that the three rings 35, that provide the correct orientation of the static piston 10 relative to the housing 3 could also formed by only one or two larger rings (not shown).

On assembly of the container 4 at the housing 3, the cylindrical wall 28 of the proximal end region 9 of the housing 3 is inserted into the groove 33 of the container 4. The guidance of the container 4 relative to the housing 3 is further enhanced by aligning the pin 19 of the housing 3 with the longitudinal section 17' of the container 4.

The container 4 is then moved first in the direction of the longitudinal axis A in the direction of the wing-like projections 11 by guiding the cylindrical wall 28 of the proximal region 9 of the housing 3 in the groove 33 in a linear manner. Once the pins 19, 19' reach the end of the respective longitudinal section 17', the container is then rotated in the direction of the arrow B (see Fig. 1) such that the pin 19 is received in the connection section 18 and is stored there, i.e. the child safety lock of the discharger 1 is activated. This means that following the rotation of the discharger 1, the child lock is activated by means of the radial displacement and the discharger 1 arrives in its storage state.

On displacing the discharger 1 into the discharging state and subsequently arriving in the position shown in the part sectional drawing of Fig. 4, the discharged state, the piercing tip 24 exerts a substantially uniform pressure on the membrane 13 causing this to initially deflect and then be uniformly pierced starting from the center of the membrane 13 radially outwardly and then permitting the fluid F stored in the compartment 12 to arrive directly at the inlet 23 present in the piercing tip 24 in the region of the start of the piercing of the membrane 13. Due to the pressure increased in the compartment 12 on pressing the container 4 towards the wing-like projections 11, the fluid F is discharged via the inlet 23, the passage and the outlet 7 from the dispensing element 2.

As the container 4 is moved in the direction of the longitudinal axis A towards the wing-like projections 11, the container 4 is linearly guided relative to the housing 3. This guidance is brought about by means of the interaction taking place between the cylindrical wall 28, the guide groove 33 and the inner and outer walls 32, 14 of the container 4.

Moreover, following the piercing of the membrane 13 and during the further guidance of the cylindrical wall 28, relative to the guide groove 33 and the inner and outer walls 32, 14 of the container 4 towards the wing-like projections 11, the sealing lip 29 present at the inner surface 30 of the housing 3 is brought into engagement with an outer surface 36 of the inner wall 32 of the double walled container 4. By means of the engagement of the sealing lip 29 at the outer surface 36 a seal is formed between the proximal region 9 of the housing and the container 4. The seal thereby forms a barrier to the fluid F escaping from the housing 3 in the proximal region 9, namely via the guide groove 33 and the slot 16.

The sealing lip 29 is thereby configured to only engage the outer surface 36 of the inner wall 32 once the piercing tip 24 comes into contact with the membrane 13. The sealing lip 29 maintains contact with the outer surface 36 of the inner wall 32 even after the piercing tip 14 has moved past the original position of the membrane 13 towards the piercing tip receiving end 31.

In order to ensure that a sealing element 29' of the housing 3 engages the wall 32 of the container 4, a portion of the container 4, preferably a portion of the wall 32 of the container 4 is received within the housing 3.

As the container 4 is moved further in the direction of the longitudinal axis A, an inner surface 37 of the compartment 12 of the double walled container 4 is initially brought into contact with the first sealing lip 25. As the container 4 is moved further in the direction of the longitudinal axis A, the inner surface 37 comes into contact with the second sealing lip 26 and then with the third sealing lip 27. The sealing lips 25, 26, 27 thereby provide a seal between the static mixer 10 and the inner wall 32 of the compartment 12 of the container 4. This seal prevents a fluid F from arriving in the space 38 provided between the piercing tip 24 and the first of the three rings 35 and thereby from passing into a part of the housing 3 at the distal region 8 of the housing 3.

A side effect of the pressure increase of the fluid F prior to discharging is, in particular with regard to viscous fluids, such as frontline, that the fluid F can leak between the inlet 23 and the membrane 13 such that it runs along the piercing tip 24 and towards the distal region 8 of the housing 3. By providing the sealing elements 25', 26', 27' between the piercing tip 24 and the inner surface 37 of the compartment this movement of the fluid F into the distal region 8 of the housing 3 is avoided.

As can be seen from the part sectional drawing of Fig. 4, the first, second and third sealing lips 25, 26, 27 are in contact with the inner surface 37 of the compartment 12, thereby ensuring that no fluid F can pass from the compartment 12 via the piercing tip 24 and the first, second and third sealing lips 25, 26, 27.

Fig. 5 shows a section through a further kind of static piston 10 having the piercing tip 24. In this section one can see that the first sealing element 25' has a diameter that is smaller than a diameter of the second sealing element 26' and of the third sealing element 27'. The diameter of the second and third sealing elements 26', 27' is at least substantially identical.

In this connection it should be noted that an external diameter of the second and third sealing elements 26', 27' is larger than an internal diameter of the compartment 12. By way of example, the external diameter of the second and third sealing elements 26', 27' is selected to be 0.01 mm to 0.2 mm larger than the internal diameter of the compartment 12 of the container 4, preferably the external diameter of the second and third sealing elements 26', 27' is selected to be 0.05 to 0.15 mm larger than the internal diameter of the compartment 12 of the container 4.

It should further be noted that the external diameter of the first sealing element 25' is smaller than or equal to the internal diameter of the compartment 12. By way of example, the external diameter of the first sealing element 25' is selected to be 0.00 mm to 0.2 mm smaller than the internal diameter of the compartment 12 of the container 4, preferably the external diameter of the first sealing element 25' is selected to be 0.02 to 0.10 mm smaller than the internal diameter of the compartment 12 of the container 4.

In use of the static piston 10 the first sealing element 25' then pushes the pierced membrane 13 into the compartment 12 and a seal is initially effected between the membrane 13 and the first sealing element 25'. In this way the first sealing element clears the way for the second and third sealing elements 26', 27' in order to ensure an as good as possible seal between the inner surface 37 of the wall 32 of the compartment 12 and the piercing tip 24.

By forming the second and third sealing elements 26', 27' slightly larger than the internal diameter of the compartment 12, the second and third sealing elements 26', 27' are compressed on introduction into the compartment 12 in order to ensure an as good as possible seal between the inner surface 37 of the wall 32 of the compartment 12 and the piercing tip 24.

An axial distance (clearance) between the second and third sealing elements 26', 27' is selected to be in the range of 0.7 to 2 mm, in the present instance the axial distance amounts to 1 mm.

In the plane of the section shown in Fig. 5, a cross-section of the second and third sealing elements 26', 27' shows that these are formed as circumferentially extending sealing lips 26, 27 at a substantially triangular structure projecting from a central section 10' of the static piston 10. The central section 10' extends around the longitudinal axis A.

In contrast to this the first sealing element 25' is formed as a circumferentially extending sealing lip 25 at the end 24' of the piercing tip 24 remote from the end 24" comprising the inlet 23. The end 24' tapers from the sealing lip 25 towards the central section 10'

A Luer lock type connection 20 is formed at an end 23' of the static piston 10 remote from the inlet 23. This Luer lock type connection 20 can be used to connect various kinds (not shown) of dispensing elements 2 to the static piston 10 in a simple manner.

In this connection it should be noted that the connection formed between the dispensing element 2 and the static piston 10 can also be formed using a different form of connection other than a Luer taper, such as the Luer lock of Fig. 5, or the integral connection of Figs. 3 and 4 discussed in the foregoing. By way of example a bayonet type connection or a simple threaded connection etc. could also be provided as forms of connection between the dispensing element 2 and the static piston 10.

It should further be noted that the dispensing element 2 can be formed by a variety of components, for example, a spray head as shown in Figs. 3 and 4; a syringe type dispensing element, e.g. a needle; a dropper type dispensing element, e.g. a simple pipe having an aperture formed at its distal end (not shown), the aperture optionally being surrounded by a thickened portion to e.g. prevent stitching; and the like can be used as a dispensing element 2.

The dimensions, sizes and shapes of the first, second and third sealing elements 25', 26', 27' discussed in connection with Fig. 5 can naturally be employed at the static piston 10 shown and discussed in connection with Figs. 3 and 4.

It should be noted in this connection that the first, second and third sealing lips 25, 26, 27 are forms of sealing elements 25', 26', 27' and different kinds of sealing elements 25', 26', 27' other than the first, second and third sealing lips 25, 26, 27 could also be employed provided they provide the desired sealing function in this region of the discharger 1.

As can further be seen from Fig. 4, the fourth sealing lip 29 that is designed to engage the outer surface 36 of the inner wall 32 of the double walled container 4, to form a seal for the fluid F in this region, is compressed to such an extent that it is no longer visible. This level of compression is required, on the one hand, to ensure a seal in this region and, on the other hand, such that the cylindrical wall 28 can be reliably guided within the groove 33. The fourth sealing lip 29 is provided in order to ensure that if a fluid F were to pass the first, second and third sealing lips 25, 26, 27 that no fluid can escape from the discharger 1 between the housing 3 and the container 4.

The guide groove 33 is provided at the double walled container 4 in order to receive at least some of the housing 3, preferably some of the cylindrical wall 28 of the housing 3. In this way one can ensure that the container 4 is reliably guided relative to the housing 3 and more specifically with respect to the piercing tip 24 in order, to safeguard that the various sealing elements 25', 26', 27', 29', e.g. the sealing lips 25, 26, 27 and 29 etc., are engaged in a uniform manner in order to prevent liquids from leaking from the container 4 into a part of the housing 3 or to the outside in a non-desired manner.

In this connection it should be noted that the sealing elements 25', 26', 27', 29' can either be integrally formed and/or fixedly connected to the respective parts of the housing 3. Alternatively, they could be formed by separate sealing elements (not shown), such as an O-ring, that are then arranged at the respective position e.g. in a specifically provided groove (also not shown).

The improved guidance is brought about by forming a width of the guide groove 33 marginally wider than a thickness of the cylindrical wall 28 of the housing 3 in such a way that the cylindrical wall 28 is moveably received in the guide groove 33 with sufficient clearance to move, but yet not so much clearance that a play is present between the housing 3 and the container 4 allowing these components to become skew with respect to one another.

In this connection it should be noted that the clearance between the guide groove 33 and the cylindrical wall of the housing 3 is preferably selected to be 0.1 mm on both sides of the cylindrical wall 28 of the housing 3.

The clearance can however be selected to be 0 mm on one side of the cylindrical wall 28 of the housing 3 and 0.1 mm on the other side of the cylindrical wall 28. Such a varying clearance at either side of the cylindrical wall may be due to the selection of the material of the cylindrical wall 28 of the housing 3 or of one of the walls 32, 14 of the container 4 that interact with the cylindrical wall 28 of the housing 3.

A length of the guide groove 33 in the shown example corresponds to a length of cylindrical wall 28 from the wing-like projections 11 to the end 39 in the proximal region 9 of the housing 3. Moreover, a length of the guide groove 33 corresponds to approximately a length of the inner wall 32 of the container 4. The outer wall 14 of the container 4 is longer than the inner wall 32.

The length L of the compartment 12 formed within the inner wall 32 is shorter than the length of the inner wall 32. A depth of the piercing tip receiving end 31 of the compartment 12 corresponds to approximately 20 to 30% of the length L of the compartment 12.

As also shown in Fig. 4 the wing-like projections 11 of the housing 3 do not contact the outer wall 14 of the container 4. This is because the wing-like projections 11 are arranged at a height of the housing 3 relative to the longitudinal axis A such that the static piston 10 can come to a stop at the end of the compartment 12 of the container 4 and hence the point of interaction between the static piston 10 and the piercing tip receiving end 31 defines a stop for the container 4 and not the wing-like projections 11.

Generally speaking the discharger 1 and the container 4 described herein are manually operable dischargers and containers that are held in the hand of a user. They are typically designed as suitable for storing and/or administering a single dose of a fluid F, such as a drug, a medication or other kind of preparation used in the care of humans or animals. This single dose can be administered in one, two or several steps, e.g. if a fluid F is to be administered to the eyes or nostrils of a patient or animal.

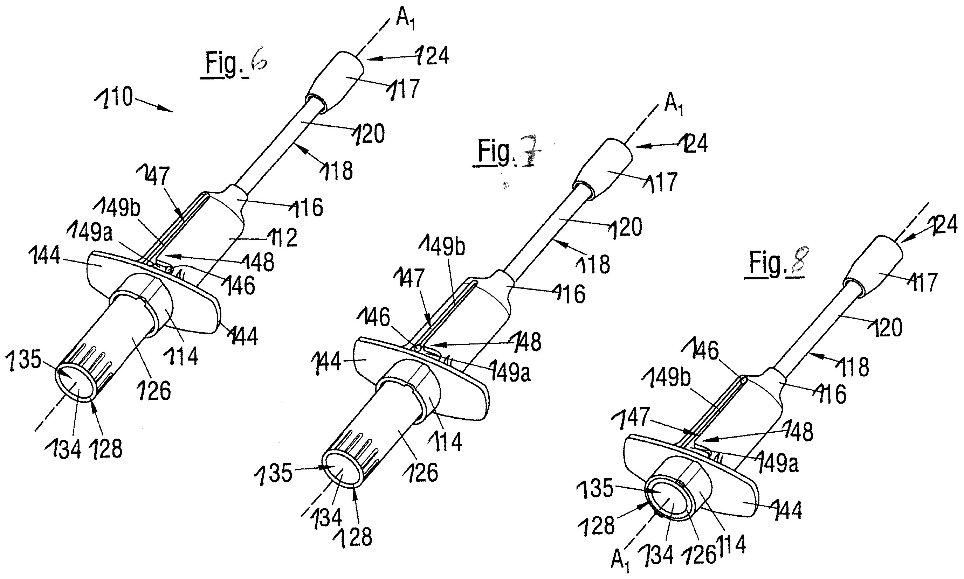

Fig. 6 shows a discharger 110 comprising a housing 112, a carriage 126 and a discharge section 118. The housing 112 defines a longitudinal first axis A₁ and is provided with opposing wing-like protrusions 144 similar to a common disposable syringe. The carriage 126 is received in a proximal end region 114 of the housing 112. The carriage 126 defines a receiving space 128 into which a container 134 has been loaded through an opening 35 of the carriage 126. The discharger 110 and the container 134 form a discharge system and are generally designed as disposable articles intended for one-time use only.

The discharge section 118 of the discharger 110 comprises a discharge passage 120 and extends from within the housing 112 through a tapered distal end 116 towards a thickened distal end portion 117. The distal end portion 117 can be formed integrally with the discharge passage 120 or can be made as a separate component connected in a suitable manner to the discharge passage 120. The distal end portion 117 moreover defines an outlet opening 124.

The distal end portion 117, in general, can be adapted to the type and site of application and can be in the form of or comprise a spray head, a needle (cannula), a brush, a sponge or a pipette. For large area applications, for example, a spray head or a sponge can be of advantage, whereas selective applications might require a needle or a plain tube of small diameters.

The discharger 110 has securing means 148 comprising a slot 147 being formed in the housing 112 and cooperating with a pin 146 being formed at the carriage 126. The slot 147 is divided into a circumferential portion 149a and into a longitudinal portion 149b extending in parallel to the first axis A₁.

The discharger 110 in Fig. 6 is shown in an initial state of use with the pin 146 being situated at the end of the circumferential portion 149a of the slot 147. In this initial state of use a movement of the carriage 126 along the longitudinal axis A₁ towards the distal end 116 is prevented.

Fig. 7 shows the discharger 110 in a starting position with the pin 146 being situated at the intersection point of the circumferential portion 149a and the longitudinal portion 149b of the slot 147. In this state the carriage 126 is movable along the longitudinal axis A₁ towards the distal end 116.

Fig. 8 shows the discharger 110 in a final discharge position with the pin 146 being situated at the end of the longitudinal portion 149b of the slot 147. In this state the carriage 126 is completely situated inside the housing 112.

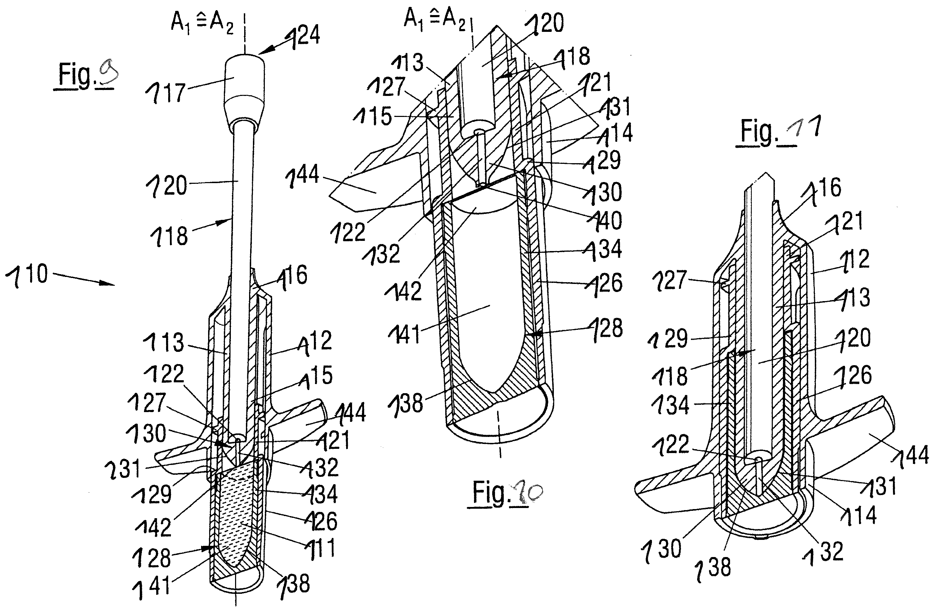

Fig. 9 shows the discharger 110 of Fig. 6 in a partial cross-section along the longitudinal first axis A₁. The discharge section 118, however, is not shown in cross-section.

Fig. 9 reveals the container 134 being located inside the receiving space 128 of the carriage 126. The container 134 is made as a capsule which has a cylindrical outer shape and is filled with a fluid 111 to be discharged. The fluid 111 inside the container 134 is located in a fluid reservoir 141 which is protected from the environment by a breakable seal 142 in the form of a foil. This becomes even more obvious in the enlarged portion of Fig. 9 depicted in Fig. 60. The amount of the fluid 111 inside the fluid reservoir 141 is approximately 0,5 ml. A circumferential shoulder 129 of the carriage 126 acts as an axial stop for the container 134 when being loaded into the receiving space 128 of the carriage 126.

Moreover, Fig. 9 reveals a sleeve portion 113 being integrally formed with the housing 112. The discharge passage 120 is fitted into the sleeve portion 113. A proximal end portion 115 of the sleeve portion 113 is fitted into a distal cylindrical portion 121 of the carriage 126 through a distal opening thereof. The housing 112 generally has a cylindrical outer shape with a constant inner diameter. In order to conform the distal cylindrical portion 121, the outer diameter of which is smaller than the inner diameter of the housing 112, to the inner diameter of the housing 112, a flange 127 is formed integrally with the distal cylindrical portion 121.

The proximal end portion 115 of the sleeve portion 113 comprises a piercing tip 130 having a flow channel 132 being connected to an inlet opening 122 of the discharge passage 120. The piercing tip 130 is formed integrally with the sleeve portion 113 of the housing 112 and defines a centrally extending longitudinal second axis A₂ which in this embodiment corresponds to the first axis A₁.

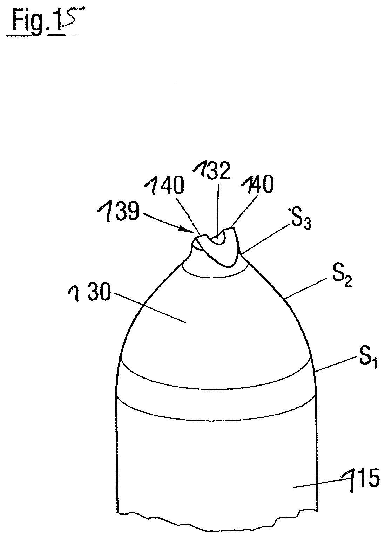

From Fig. 5 it becomes apparent that the flow channel 132 extends along the longitudinal first or second axis A₁, A₂. Also the configuration of the piercing tip 130 becomes clear. The piercing tip 130 has a convexly curved outer surface 131 and moreover comprises two protrusions 140 being adjacent to the flow channel 132, respectively. The protrusions originate from a ridge 139 that has been interrupted by the flow channel 132 during the manufacturing process (see Fig. 15).

Fig. 10 further reveals that the fluid reservoir 141 has a rotationally symmetric inner contour 138. Apart from the protrusions 140, the inner contour 138 is complementary to the outer shape of the piercing tip 130.

Fig. 11 shows the discharger 110 in its final discharge position, i.e. the carriage 126 has been fully moved towards the distal end 116 into the housing 112. In this position, the piercing tip 130 and at least the proximal end portion 115 of the sleeve portion 113 are located in the fluid reservoir 141 of the container 134. The seal 142 has been pierced and the fluid 111 has been displaced. The protrusions 140 have been compressed such that the piercing tip 130 and the inner contour 138 of the fluid reservoir 141 establish a form-fitting connection.

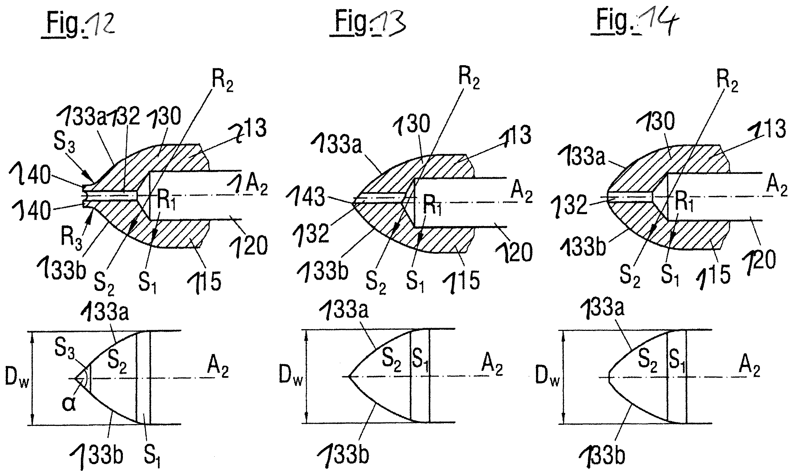

Fig. 12 shows detailed views of the piercing tip 130 of Figs. 9 to 11. The upper pane contains a cross-sectional view along the longitudinal second axis A₂ and along the two protrusions 140. The two protrusions 140 are located adjacent to a proximal entry opening of the centrally located flow channel 132. The shape of the piercing tip 130 is defined by two surface lines 133a, 133b which are axially symmetric. Each surface line 133a, 133b consists of three sections S₁, S₂, S₃, wherein each section S₁, S₂, S₃ is defined by a curvature radius R₁, R₂, R₃. This means each section S₁, S₂, S₃ is a segment of a circle. The length of the curvature radii R₁, R₂, R₃ is indicated by respective arrows. The radius R₁ of the first section S₁ is roughly one fifth of the radius R₂ of the second section S₂. The length of the radius R₂ of the second section S₂ is more than half the length of a diameter D_{w} of the piercing tip 130 at its widest point (see lower pane). The third section S₃ particularly defines the shape of the protrusions 140. The length of the corresponding radius R₃ is approximately half of the radius R₁. Thus, the convex shape of the piercing tip 130 is mainly defined by sections S₁ and S₂.

The lower pane of Fig. 12 shows a side view of the piercing tip 130 shown in the upper pane after a rotation by 90°. The sections S₁, S₂, S₃ are indicated by lines perpendicular to the second axis A₂. It is clearly visible that the surface lines defining section S₃ in this view run linear and intersect at an angle α of approximately 90°. Thus, the protrusions 140 in this view have a triangular profile.

A perspective view clearly indicating the convex shape of the outer surface of the piecing tip 130 according to Figs. 4 to 7 is also shown in Fig. 15.

Fig. 13 in its upper pane shows a cross-section along the longitudinal second axis A₂ of another embodiment of the piercing tip 130 according to the present technology. Each surface line 133a, 133b consists of two sections S₁, S₂ each being a segment of a circle having a corresponding radius R₁, R₂. Regarding the length of the radii R₁, R₂ the same applies as for Fig. 12.

The flow channel 132 is not centrally located within the piercing tip 130 but is located at an offset position relative to the second axis A₂. This means the piercing tip 130 slightly deviates from a rotationally symmetric configuration. Due to this arrangement the piercing tip 130 comprises a thorn-like protrusion 143 which, however, in the side view after a rotation by 90° depicted in the lower pane of Fig. 62 is not visible anymore. It turned out that this configuration lowers the piercing force that has to be initially applied for puncturing the seal 142, however, not to the same extend as for the embodiment comprising two protrusions 140.

Fig. 14 in its upper pane shows a cross-section along the longitudinal second axis A₂ of a further embodiment of the piercing tip 130 according to the present technology. The piercing tip 130 is rotationally symmetric about the second axis A₂ since the flow channel 132 is centrally located within the piercing tip 130. Each surface line 133a, 133b consists of two sections S₁, S₂ each being a segment of a circle having a corresponding radius R₁, R₂. Regarding the length of the radii R₁, R₂ the same applies as for Figs. 12 and 13.

Both representations, the cross-section in the upper pane of Fig. 14 and the side view after a rotation by 90° in the lower pane, indicate that the piercing tip 130 is slightly cut due to the arrangement of the flow channel 132. Thus, the apex of the piercing tip 130 is an imaginary point located where the two surface lines 133a, 133b would intersect if they were not interrupted by the flow channel 132.

Any of the three piercing tips according to Figs. 12 to 13 are able to initially punctuate a foil with a moderate and user-friendly piercing force, wherein the piercing tip comprising two protrusions 140 has shown the best results in this regard. The convexly curved configuration, however, is mainly responsible that the force which needs to be applied during the movement or displacement of the piercing tip remains essentially constant over a wide distance. This constant force distribution was shown by all of the three piercing tips according to Figs. 12 to 14 and can therefore be regarded as independent of the shape in the rather small region of the apex of the piercing tip. With particular benefit it is therefore possible to ensure a highly homogeneous discharge by merely adapting the shape in the apex region of the piercing tip to the characteristics of the applied foil.

For operating the discharger 110, i.e. to discharge the fluid 111 inside the container 134 through the discharge passage 120 out of the outlet opening 124, the carriage 126 including the container 134 needs to be pushed towards the distal end 116 into the housing 112. However, the securing means 148 prevent the carriage 126 from being unintentionally pushed into the housing 112 when the discharger is in an initial state of use. In this initial state of use shown in Figs. 6, 9 and 10, the piercing tip 130 is still positioned spaced apart from the seal 142 of the container 134, i.e. the piercing tip 130 does not yet protrude into the fluid reservoir 141 of the container 134.

In order to discharge the fluid 111, the user, firstly, has to deliberately rotate the carriage 126 counterclockwise with respect to the housing 112 until the pin 146 is aligned with the axial portion 149b of the slot 147. For facilitating the rotation of the carriage 126, the outer surface of the carriage 126 may be roughened or provided with longitudinal grooves providing a non-slip surface.

The carriage 126 now being in the starting position is movable along the longitudinal axis A₁. From the starting position the carriage 126 and the container 134 together can be actuated like a push button which can be pressed by the user with a thumb while counteracting this actuation by holding the housing 112 with two fingers behind the projections 144. Thus, to discharge the fluid 111 the user, secondly, has to deliberately push the carriage 126 towards the distal end 116. While the carriage 126 moves towards the distal end 116, the piercing tip 130 initially punctuates and then pierces the seal 142. The piercing tip 130 enters into the fluid reservoir 141 of the container 134, thereby urging the fluid 111 out of the container 134 through the flow channel 132 being connected to the discharge passage 120. The complementary shapes of the piercing tip 130 and the inner contour 138 of the fluid reservoir 141 ensure that essentially no residual amounts of fluid 111 remain within the container 134.

Since in many countries pertinent regulations prohibit a simple throwing away of medical, dental or veterinary substances, the lack of substantial residual amounts of fluid as provided by the discharge system as described herein simplifies the disposal of used containers and dischargers in accordance with the respective national regulations.

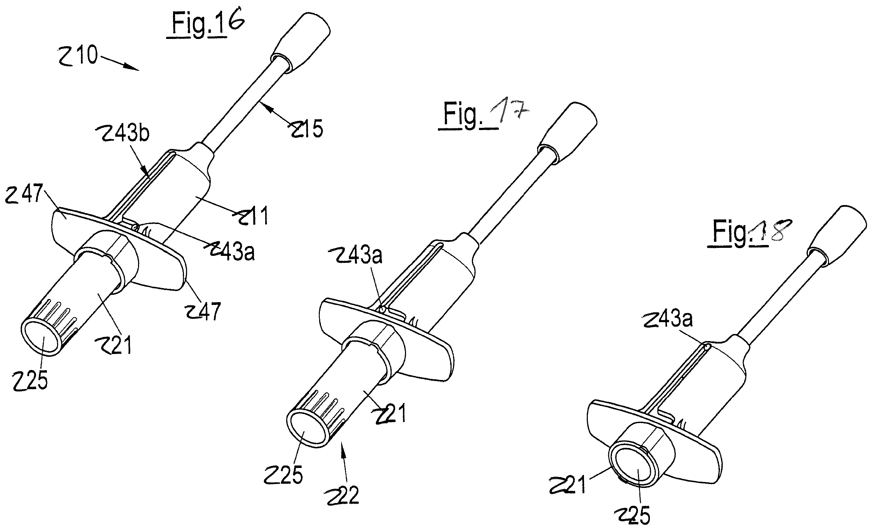

In the embodiment shown in Figs. 16 to 21, the discharge system comprises a discharger 210 into which a separate container 225 having a generally cylindrical outer shape can be loaded.

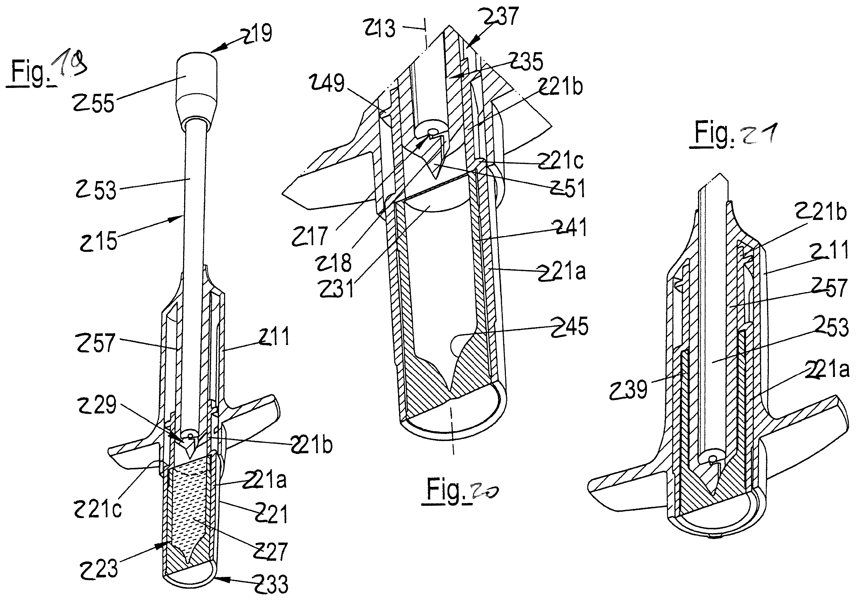

Although other appliances are possible, in the embodiment shown the discharger 210 is intended for use in the medical, dental or veterinary field, and indeed for discharging a predetermined amount of fluid 227 (Fig. 19) which is contained inside the container 225. The fluid to be discharged is a liquid which includes one or more medical, dental or veterinary agents.

The discharger and the container are designed as disposable articles intended for one-time use only.

The container 225 is made as a capsule which has a generally cylindrical outer shape. The container 225 comprises a wall portion 241 (Fig. 20) with an integral proximal end portion providing a proximal end face, and with an opening at the distal end, this opening being closed by a breakable seal 231 in the form of a foil. The wall portion 241 and the seal 231 confine a fluid space of the container which is at least partly filled with the fluid 227. The amount of the fluid is for example 0,5 ml.

In a ready-to-use state as shown in Fig. 16, the container 225 has been loaded into a carriage 221 of the discharger 210. To be loaded into the carriage 225, the container 225 is slid through a proximal insertion opening 233 of the carriage 221.

The carriage 221 is received in a proximal end region of a housing 211 which is provided with wing-like protrusions 227 extending in opposite directions like in a common disposable syringe. Moreover, a slot 243b is formed in the housing 211 which cooperates with a pin 243a formed at the carriage 221 so as to form securing means which are explained later in more detail.

The discharger 210 also comprises a discharge device 215 from which Figs. 16 to 18 show a tube 253 (Fig. 19) extending from within the housing 211 towards a thickened distal end portion 255 (Fig. 19). The end portion 255 can be integral with the tube 253 or made as a separate component connected in a suitable manner to the tube 253.

The discharger 210 is adapted to discharge the fluid 227 inside the container 225 through the discharge device 215 simply by pushing the carriage 221 including the container 225 into the housing 211. This process is described elsewhere in connection with Figs. 4 to 6.

The securing means 243a, 243b prevent the carriage 221 from being pushed into the housing 211 unintentionally. In an initial condition shown in Fig. 16, the pin 243a of the carriage 221 is situated at the end of a circumferential portion of the slot 243b (Fig. 17). This condition prohibits a movement of the carriage 221 along the longitudinal axis 213 (Fig. 20) of the discharger 210.

In order to discharge the fluid, the user must deliberately rotate the carriage 221 with respect to the housing 211 so as to align the pin 243a with an axial portion of the slot 243b (Fig. 17). In order to facilitate the rotation of the carriage 221, the outer wall of the carriage 221 is provided with longitudinal grooves 222.

In Fig. 17, the carriage 221 is in a starting position and ready to be pushed into the housing 211.

Fig. 18 shows a condition in which the carriage 221 is in a final discharge position in which the carriage 221 and thus the loaded container 225 are fully received within the housing 211, the proximal end faces of the carriage 221 and the container 225 being flush with a proximal end face of the housing 211.

From Figs. 16 to 18 it can be seen that the carriage 221 and the container 225 together can be actuated like a push button which is to be pressed by a user with her or his thumb while counteracting this pressing actuation by holding the housing with two fingers behind the projections 247.

As shown in Fig. 19, the discharge device 215 comprises a sleeve portion 257 formed integrally with the housing 211 and being arranged inside the housing 211. Further, the discharge device 215 comprises the tube 253 mentioned above which is fitted into the sleeve portion 257.

A proximal end portion 235 (Fig. 20) of the discharge device 215 is fitted into a distal cylindrical portion 221b of the carriage 221 through a distal opening 237 thereof. The proximal end portion 235 comprises an inlet opening 217 at the proximal end of the tube 253, an inlet passage 218 formed at the proximal end of the sleeve portion 257 and activation means 229 (Fig. 19) comprising a tip 251 (Fig. 20) formed by a correspondingly tapering of the proximal end of the sleeve portion 257. In the initial condition shown in Fig. 19, the tip 251 is still positioned spaced apart from the seal 231 of the container 225, i.e. the activation means 229 comprising the tip 251 do not yet protrude into a proximal cylindrical portion 221a of the carriage 221 which defines a receiving space 223 of the carriage 221 for the container 225.

An axial portion of the inlet passage 218 extends offset from the longitudinal axis 213 and leads to a radial portion of the inlet passage 218 which leads into the inlet opening 217 of the tube 253.

A discharge passage extending through the tube 253 connects the inlet opening 217 with an outlet opening 219 (Fig. 19) provided at the distal end of the discharge device 215.

As can be seen especially in Figs. 19 and 21, the housing 211 generally has a cylindrical outer shape with a constant inner diameter which is equal to the outer diameter of the proximal cylindrical portion 221a of the carriage 221. In order to conform also the distal cylindrical portion 221b, the outer diameter of which being smaller than that of the proximal cylindrical portion 221a, to the inner diameter of the housing 211, a flange 249 is formed integrally with the distal cylindrical portion 221b.

Moreover, the thickness of the wall portion 241 of the container 225 corresponds to the difference between the inner diameters of the cylindrical portions 221a, 221b of the carriage 221. Consequently, the container 225 reduces the inner diameter of the proximal cylindrical portion 221a to that of the distal cylindrical portion 221b, thereby establishing an inner space of the carriage 221 having a constant inner diameter which corresponds to the outer diameter of the proximal end portion 235 of the discharge device 215.

This dimensioning of the individual components of the discharge system provides for a mechanically reliable mutual guiding of the individual components when the carriage 221 including the container 225 is pushed into the housing. Specifically, the carriage 221 is guided by the inner wall of the housing 211 through its flange 249 and its proximal cylindrical portion 221a. In addition, the carriage 221 is guided by the discharge device 215.

Moreover, the design of the individual components as explained above minimizes the required outer dimensions of the discharger 210, in particular the outer diameter of the housing 211. As can be seen in Fig. 21, there is no unused space inside the housing 211. When the carriage 221 including the container 225 is in the final discharge position, all components are concentrically packed within the housing 211.

A transition portion 221c between the proximal cylindrical portion 221a and the distal cylindrical portion 221b of the carriage 221 is formed as a circumferential shoulder which acts as an axial stop for the container 225 when being loaded into the carriage 221. The axial distance between the proximal end face of the proximal cylindrical portion 221a and the transition portion 221c corresponds to the axial length of the container 225.

The outer shape of the tapered proximal end of the discharge device 215 corresponds to a proximal end section 245 of the inner wall of the container 225 so that almost no free space remains inside the container 225 when the carriage 221 including the container 225 is in its final discharged position as shown in Fig. 21. The complementary shapes of the discharge device 215 and the container 225 thus ensure that virtually no residual amounts of fluid remain within the container 225.

Since in many countries pertinent regulations prohibit a simple throwing away of medical, dental or veterinary substances, the lack of substantial residual amounts of fluid as provided by the discharge system as described herein simplifies the disposal of used containers and dischargers in accordance with the respective national regulations.

In operation, in the initial state as shown in Fig. 16 the securing means 243a, 243b prevent the carriage 221 from being unintentionally pushed into the housing 211.

For discharging the fluid included in the container 225, which has previously been loaded into the carriage 221, the user has to rotate the carriage 221 in order to bring the pin 243a into alignment with the axial portion of the slot 243b (Fig. 17).

The carriage 221 including the container 225 can now be pushed into the housing 211, and indeed from the starting position shown in Fig. 17 into the final discharge position shown in Fig. 18.

When the carriage 221 is being moved along the longitudinal axis 213 into the housing 211, the seal 231 of the container 225 is broken by the tip 251 of the activation means 229 provided at the proximal end of the discharge device 215. While the carriage 221 continues its movement into the housing 211, the proximal end portion 235 of the discharge device 215 enters into the fluid space of the container 225, thereby urging the fluid 227 out of the container 225. Since the proximal end portion 235 is sealingly fitted into the distal cylindrical portion 221b of the carriage 221, the fluid can only escape through the inlet passage 218 and thus through the discharge passage of the tube 253 of the discharge device 215.

Consequently, while the carriage 221 is still on its way into the final discharge position, the fluid begins to flow through the inlet passage 218 and the inlet opening 217 and through the discharge passage so as to be ejected from the outlet opening 219.

Since the proximal end portion 235 of the discharge device 215 sealingly fits into the fluid space of the container 225 as well, leakage of fluid past the discharge device 215 is prohibited at any time.

Generally, the discharger and/or the container may be fabricated from any suitable material. In one embodiment, the material is plastic. The material may be selected from the group comprising PP, COC, PE, PA, PBT and PMMA. Alternatively, the material may be glass, metal or an alloy.

From the above description, the skilled person will easily appreciate that the discharge system as described herein ensures a simple and reliable single-hand operation for discharging a predetermined amount of fluid such as a liquid containing at least one medical, dental or veterinary agent. Moreover, this discharge system allows for a long-term storage due to the usage of a separate fluid container so that it is no longer necessary to keep the fluid within the discharger itself. Rather, the fluid can be stored over a long period of time within the container which can be closed by a breakable seal in a manner such that the fluid is very well protected from environmental influences.

With respect to one common classification applied in the medical, dental or veterinary field or the health care sector, the discharger and the discharge system as provided for by the present disclosure belong to the group consisting of systems without protection cap.

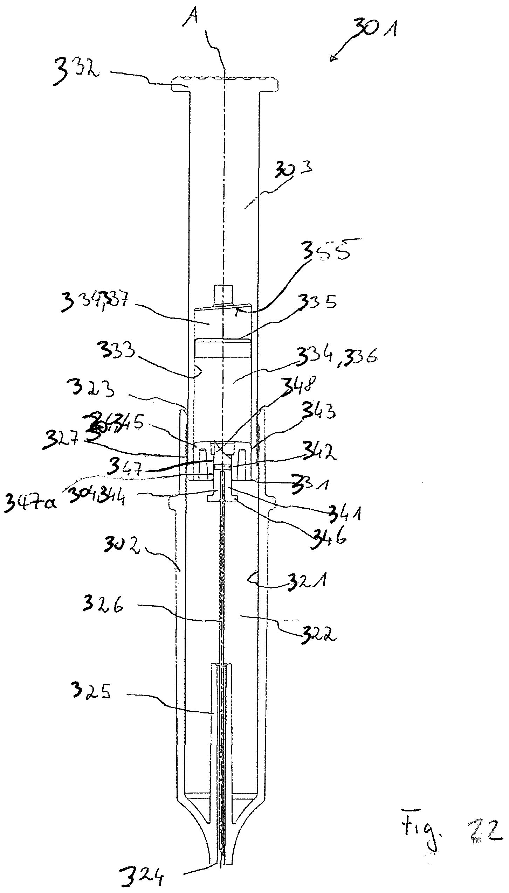

Fig. 22 shows a schematic representation of a first embodiment of a syringe 301 in accordance with the invention in a storage position. The syringe 301 comprises a hollow body 302, a piston 303 and an intermediate piece 304. The hollow body has a surrounding hollow body surface 321 and a passage 322 therein. The hollow body 321 has a circular base surface and forms a cylindrical passage 322. An axis A is formed in an axial longitudinal direction of the hollow body 302. The passage 322 moreover has a rear body opening 323 and a front body opening 324 which are arranged opposite one another in the longitudinal direction.

A first tubular needle seat 325 is formed at the front body opening 324 so that the front body opening 324 is closed except for a needle seat opening in the first needle seat 325. A needle cannula 326 is arranged in the first needle seat 325 and is movable relative to the first needle seat 325 along the axis A.

The piston 303 is arranged in the hollow body 302 and is movable in the passage 322 along the axis A. The piston 303 further comprises a front piston opening 331, which is arranged in the direction of the front body opening 324, and a rear, closed piston end 332. The front piston opening 331 and the rear piston end 332 are arranged opposite one another along the axis A in the longitudinal direction.

A surrounding intermediate wall 333, which forms a receiving chamber 334 for receiving fluid, is arranged between the front piston opening 331 and the rear piston end 332. The receiving chamber 334 is divided by means of a separating element 335 into a first chamber 336 and into a second chamber 337. The separating element 335 is formed as a film. The chambers 336, 337 can each receive a fluid, with a first fluid being arranged in the first chamber 336 and a second fluid being arranged in the second chamber 337. In the storage position, the film separates the second chamber 337 from the first chamber 336 and thus from a supply space 342 formed in the intermediate piece 304 such that they do not have any flow communication.

The first fluid can in this respect, for example, be a medicine to be dispensed and the second fluid can be a flushing solution.

The intermediate piece 304 is movably arranged in the passage 322 along the axis A. A second needle seat 341 is formed in the intermediate piece 304, with the needle cannula 326 being firmly held in the second needle seat 341. The intermediate piece 304 is in two parts and comprises an outer element 345 and an inner element 344, wherein the second needle seat 341 is formed at the inner element 344. The inner element 344 further comprises an abutment 346 for the outer element 345, the abutment being formed as a peripheral shoulder. The abutment 346 is arranged in the direction of the front body opening 324 and acts as a stopper for the outer element 345 which can therefore stop a movement of the outer element 345. In addition, the inner element 344 is formed in the direction of the rear body opening 323 as a mandrel 348 to break open the separating element 335. The intermediate piece 304 furthermore has the supply space 342 which is formed in the intermediate piece 304 and which can in particular be of conical design. The second needle seat 341 and the supply space 342 are in flow communication so that the first fluid and the second fluid can flow via the supply space 342 into the needle cannula 326 when seals acting in the storage position are deactivated.

The piston 303, the hollow body 302 and the intermediate piece 304 are movable by means of a coaxial relative movement starting from a storage position into a release position. In the storage position present in Fig. 301, a first sealing region 343 is formed between the intermediate wall 333 and the outer element 345 of the intermediate piece 304 and a second sealing region forming further seals 347, 347a is formed between the inner and outer elements 344, 345, wherein, viewed in the axial direction, a seal 347 is provided in front of the supply space 342 and a seal 347a is provided behind the supply space 342. The first sealing region 343 comprises a groove at the intermediate wall 333 of the receiving chamber 334 and a dimple at the intermediate piece 304 or at the outer element 345. The second sealing region 347, 347a in each case comprises a further groove at the outer element 345 and a dimple at the inner element 344.

The first sealing region 343 prevents fluid from being able to flow out of the receiving chamber 334 into the passage 322 of the hollow body 302. The second sealing region prevents fluid from being able to flow into the supply space 342 by means of the seal 347 in the storage position, on the one hand, and from fluid being able to flow through between the inner element 344 and the outer element 345 into the passage 322 of the hollow body 302 by means of the seal 347a, on the other hand. The receiving chamber 334 and the supply space 342 are thus not in flow communication in the storage position. Neither the first fluid nor the second fluid therefore flow from the receiving chamber 334 into the supply space 342.

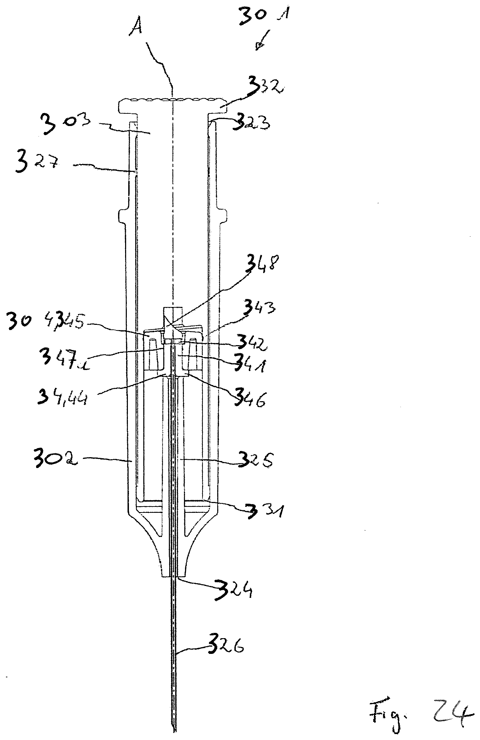

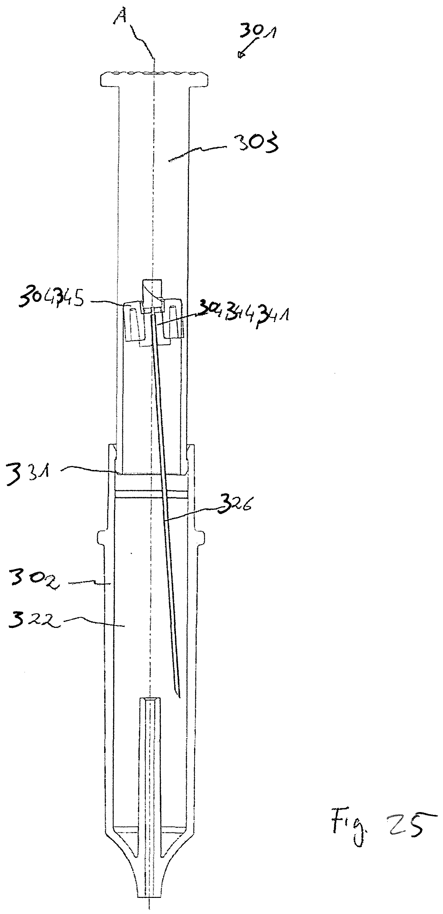

The receiving chamber 334 is bounded at the rear end by a sloping surface 355 which cooperates with the rear end of the intermediate piece 304, which will be looked at in more detail in the following in connection with Figs. 24 and 25.

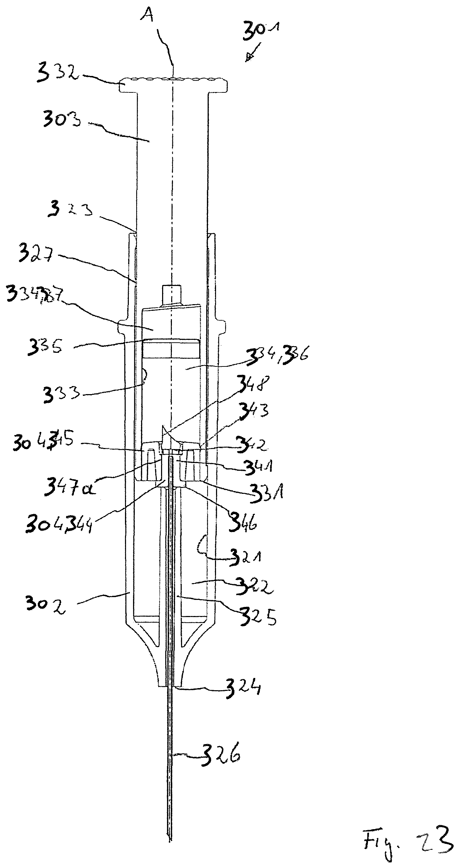

A schematic representation of the first embodiment in the release position is shown in Fig. 23. The release position corresponds to an activated position, i.e. the piston 303 has been moved along the axis A in the passage 322 in the direction of the front body opening 324 by means of a coaxial movement by means of a force transmission, for example by pressure onto the rear piston end 332. In this respect, a holding device 27 is provided in the form of a peripheral, inwardly projecting rib which holds the piston 303 at the hollow body surface 321 in that a friction force is generated which has to be overcome by means of the force transmission.

In the release position, the inner element 344 has been moved along the axis A in the direction of the front body opening 324 and is arranged at the first needle seat 325. In this respect, the inner element 344 lies on the first needle seat 325 and the first and second needle seats 325, 341 together form an approximately continuous needle seat. In this respect, the needle cannula 326, which is arranged in the storage position completely in the first and second needle seats 325, 341 and in the hollow body 302, has been moved along the axis A through the front body opening 324 out of the hollow body 302 and is located partly outside the hollow body 302. The outer element 345 has likewise been moved along the axis A in the direction of the front body opening 324 and is arranged at the abutment 346, whereby the sealing effect of the seal 347 of the second sealing region has been deactivated so that, in the release position, the first chamber 336 of the receiving chamber 334 and the supply space 342 are in flow communication.

In the release position, the first fluid can flow from the first chamber 336 through the supply space 342 into the inner element 344 or into a needle seat opening formed therein and can then flow into the needle cannula 326.

On the subsequent transition into a flushing position (not shown) after emptying the first chamber 336, the mandrel 348 pierces the film, whereupon the second chamber 337 and the supply space 342 are in flow communication and a flushing procedure can be carried out by discharging the second fluid, that is by emptying the second chamber 337, through the needle cannula 326.

Fig. 24 shows a schematic representation of the first embodiment after discharging the first fluid and the second fluid. In this end position, the piston 303 contacts the intermediate piece 304 with the slope 355 formed at the rear end of the receiving chamber 334 while interposing the pierced film 335. The piston 303 is arranged approximately completely in the passage 322 of the hollow body 302, with a further movement of the piston 303 being prevented along the axis A in the direction of the front body opening 324. The separating element 335 has been broken open by means of the mandrel 348 and the second fluid has flowed from the second chamber 337 (Fig. 23) via the supply space 342 into the needle cannula 326. A recess into which the mandrel 348 projects is provided in an axial direction to the rear beyond the slope 355.

The slope 355 endeavors to locate the intermediate piece 304 together with the inner element 344 and the needle cannula 326 in a slanted position, which is, however, not possible in the position in accordance with Fig. 24 since the needle cannula 326 is still located in the first needle seat 325 and can thus not be deflected or pivoted.

Fig. 25 shows a schematic representation of the first embodiment in a draw-in position. In the draw-in position, the piston 303 has been withdrawn approximately completely out of the passage 322 of the hollow body 302, with the front piston opening 331 being arranged in the passage 322. The needle cannula 326 is held firmly at the intermediate piece 304 via the inner element 344 at the second needle seat 341 such that the needle cannula 326 is taken along on the backward movement of the piston 303. In this respect, the needle cannula 326 is completely pulled into the passage 322 and out of the first needle seat 325 and is thus released. The intermediate piece 304, pre-biased into a corresponding sloping position due to the slope 355, can adopt the sloping position by this release of the needle cannula 326 and can consequently locate the needle cannula 326 in a slanted position which can thus not accidentally or randomly thread back into the first needle seat 325 and is consequently so-to-say captured in the hollow body 302. The needle cannula 326 or the syringe 301 can therefore not be reused and there is no risk of injury.

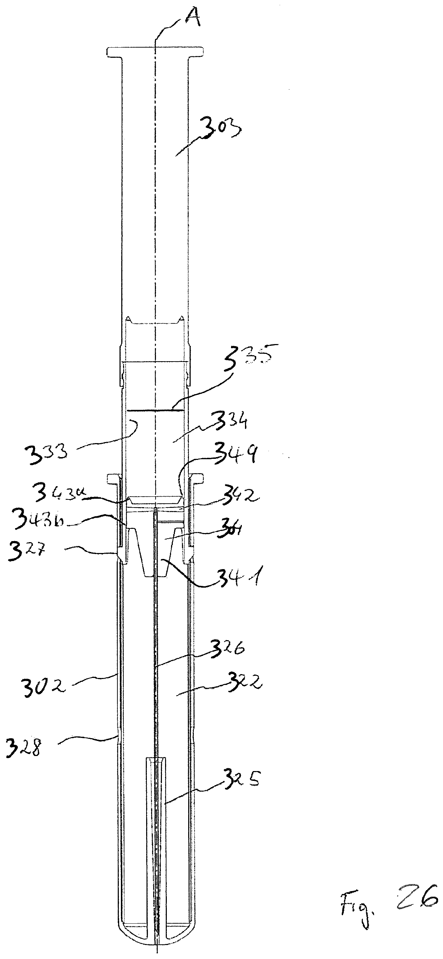

Fig. 26 shows a schematic representation of a second embodiment of a syringe in accordance with the invention in the storage position. The design of the syringe 301 in this respect has a number of common features with the syringe 301 from Figs. 22 to 25 so that substantially the differences will be looked at.

The intermediate piece 304 is arranged, analog to Figs. 22 to 25, movably in the passage 322 along the axis A. A second needle seat 341 is formed in the intermediate piece 304, with the needle cannula 326 being firmly held in the second needle seat 341. The intermediate piece 304 is in one part, unlike the embodiment in accordance with Figs. 22 to 25. The single-part intermediate piece 304 has a supply space 342 which is formed in the intermediate piece 304. In the storage position shown in Fig. 26, the first sealing region 343a is formed between the intermediate wall 333 and the intermediate piece 304 and forms a first seal at the rear side of the supply space 342. A second seal 343b is arranged at the front side of the supply space 342. The seal formed by the first sealing region 343a comprises a groove at the intermediate wall 333 of the receiving chamber 334 and a dimple at the intermediate piece 304. The first sealing region 343a prevents fluid from being able to flow into the supply space 342 in the storage position, whereas the second seal 343b prevents fluid from being able to flow from the supply space 342 into the passage 322 of the hollow body 302. The receiving chamber 334 and the supply space 342 are thus not in flow communication in the storage position, i.e. neither the first fluid nor the second fluid flows from the receiving chamber 334 into the supply space 342.

In the storage position, the piston 303 is held by a holding device 27 and can only be moved by means of a force transmission. In an active position, not shown, the piston 303 is held by means of an activating device 28 and can only be moved into the release position by means of a force transmission. The needle cannula 326 is moved out in the active position. The separating element 335 only shown schematically here is broken open in this second embodiment by means of a sharp peripheral edge 349 of the intermediate piece 304. A slope can be provided in accordance with the embodiment of Figs. 22 to 25 or another means can be provided for pivoting or deflecting the needle cannula 326.

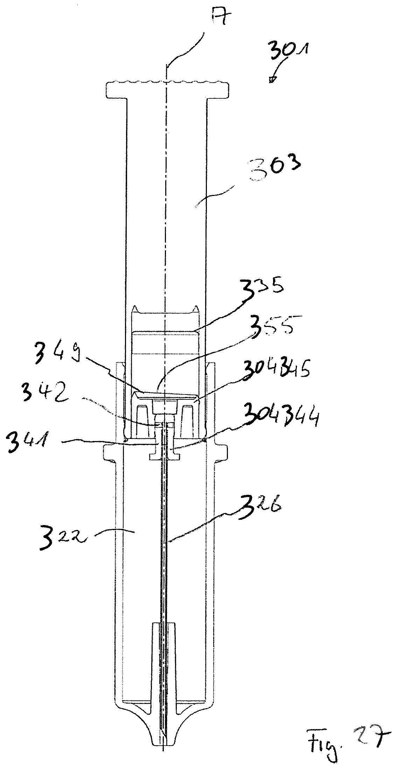

Fig. 27 shows a schematic representation of a third embodiment of a syringe in accordance with the invention in the storage position. The design of the syringe 301 in this respect has a number of common features with the syringe 301 from Figs. 22 to 25 so that substantially the differences will be looked at. The two-part intermediate piece 304 is movably arranged in the passage 322 along the axis A. A second needle seat 341 is formed in the intermediate piece 304, with the needle cannula 326 being firmly held in the second needle seat 341. A sharp peripheral edge 349 for breaking open the separating element 335 is formed at the outer element 345, in the direction of the rear piston end 332. The edge 349 does not have a peripherally constant height, but rather defines a plane 355 which is inclined with respect to the axis A and which, analog to the sloping surface 355 in the embodiment of Figs. 22 to 25, ensures a slanted position of the needle cannula 326 in the draw-in position.

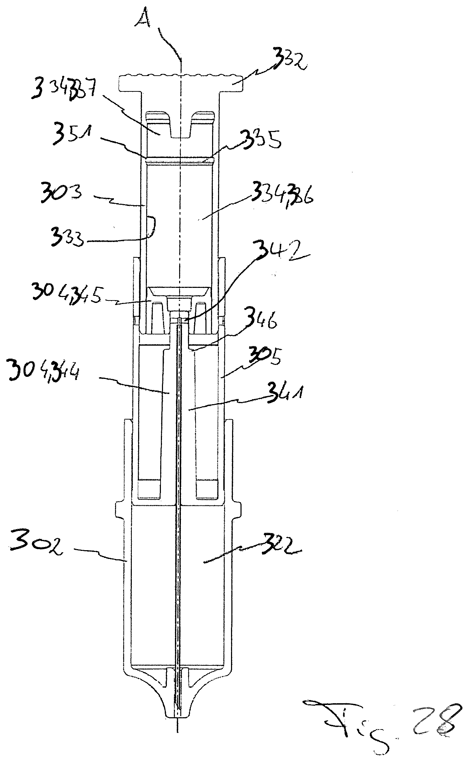

Fig. 28 shows a schematic representation of a fourth embodiment of a syringe in accordance with the invention in the storage position. The design of the syringe 301 substantially corresponds to the syringe 301 from Figs. 22 to 25 so that substantially the differences will be looked at. The intermediate piece 304 is in two parts, with the inner element 344 being configured such that the second needle seat 341 is hollow cylindrical and has two different outer diameters and so forms the abutment 346 for the outer element 345. In addition, the inner element 344 is formed as a further hollow body 305 in which the piston 303 is arranged. The inner element 344, which is configured in one piece overall, consequently comprises the radially inwardly disposed needle seat 341 and an outer part which defines the hollow body 305, which is arranged concentrically to the needle seat 341 and which receives the piston 303. The inner element 344 has no mandrel 348.

The further hollow body 305 is arranged in the passage 322 of the hollow body 302. The coaxial relative movement of the piston 303 thus takes place along the axis A in the further hollow body 305 of the inner element 344 which in turn moves along the axis A in the hollow body 302.

The separating element 335 is configured as a plug and is arranged in the receiving chamber 334 movably along the axis A. The separating element 335 can be displaced by means of the outer element 345 of the intermediate piece 304 to empty the second chamber 337. Provision can alternatively be made that the separating element 335, which is e.g. formed as a film, is broken open by means of the outer element 345, in particular by means of a sharp edge formed at the outer element 345.

In addition, a sealing region 351 which acts in the storage position or the release position is configured and arranged between the intermediate wall 333 and the separating element 335 such that the second chamber 337 and the supply space 342 do not have any flow communication, just as the second chamber 337 and the first chamber 336 have no flow communication. The sealing region 351 comprises a groove in the intermediate wall 333 and a dimple complementary thereto in the separating element 335. In the flushing position, the sealing region 351 is deactivated and the second chamber 337 and the supply space 342 are in flow communication.

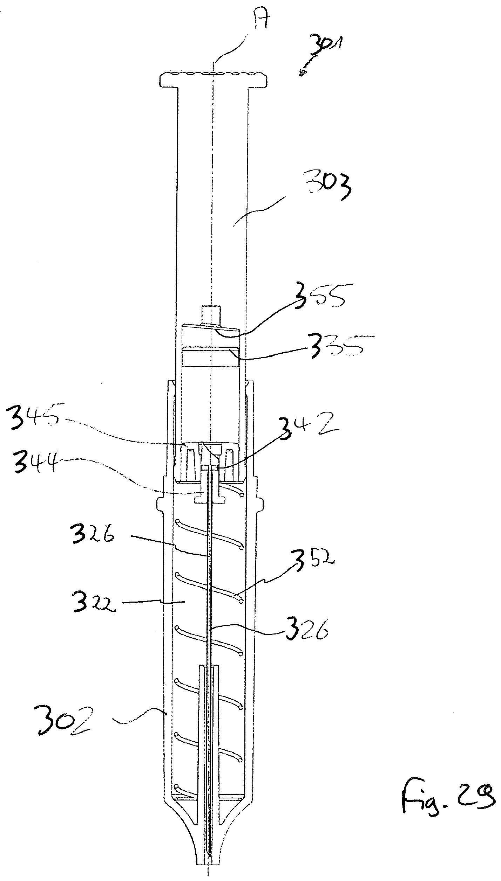

Fig. 29 shows a schematic representation of a fifth embodiment of a syringe 301 in accordance with the invention in the storage position. The design of the syringe 301 substantially corresponds to the syringe 301 from Figs. 22 to 25 so that substantially the differences will be looked at. To ensure that, on the transition into the draw-in position, the needle cannula 326 is drawn completely into the passage 322, a spring 352 is arranged in the passage 322 against whose restoring force the piston 303 has to be pressed to the front, starting from the storage position. The spring 352 consequently effects or assists the transition into the draw-in position in accordance with the proper use of the syringe 301.

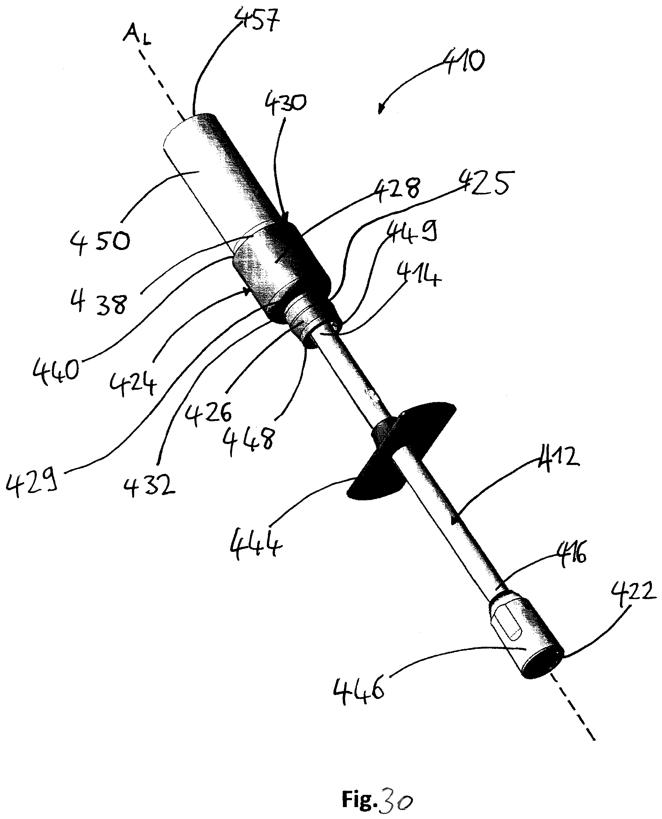

Fig. 30 shows a discharger 410 comprising a discharge section 412 defining a longitudinal axis A_{L}, an intermediate section 424 and a container 450. The discharge section 412 has a proximal end 414 and a distal end 416 and is provided with wing-like projections 444 being approximately situated in the middle between the proximal end 414 and the distal end 416. The projections 444 are in the shape of a finger flange and, for example, are integrally formed with the discharge section 412 by injection molding. On its distal end 416 the discharge section 412 comprises a distal end portion 446 defining an outlet opening 422. The distal end portion 446 can, for example, be an individual part fixed (e.g. welded) to the discharge section 412, or it can be integrally formed with the discharge section 412, e.g. by injection molding. In this embodiment, the distal end portion 446 is designed as a spray head, preferably for a large area application. However, the distal end portion 446 can be adapted to the type and site of application and can also comprise a needle (cannula), a brush, a sponge, a pipette or the like.

The intermediate section 424 comprises a proximal portion 428 and a distal portion 426 being formed in one piece and being interconnected by a breakable section 432. The breakable section 432 is located at a distal end 429 of the proximal portion 428 and has the shape of a tearable film or a web. The breakable section 432 is made of the same material as the proximal portion 428 and the distal portion 426.

The distal portion 426 of the intermediate section 424 has a receiving end 448 with an opening 449 for receiving the proximal end 414 of the discharge section 412. The proximal end 414 is merely plugged into the distal portion 426 without further securing the connection by welding or gluing.

The proximal portion 428 of the intermediate section 424 also has a receiving end 438 with an insertion opening 440 and defines a receiving space 430 which is adapted to be loaded with a separate container 450. The container has a proximal end 457 and is plugged into the receiving space 430 by sliding the container 450 through the insertion opening 440.

In total, the discharger 410 consists of three individual parts, namely the discharge section 412, the intermediate section 424 and the container 450. In general, the distal end portion 446 can be a further individual part, too.

The distal portion 426 of the intermediate section 424 comprises a sealing lip 425 disposed circumferentially around the distal portion 426. The sealing lip 425 is defined by a circumferential area of the distal portion 426 being larger in diameter than the remaining distal portion 426.

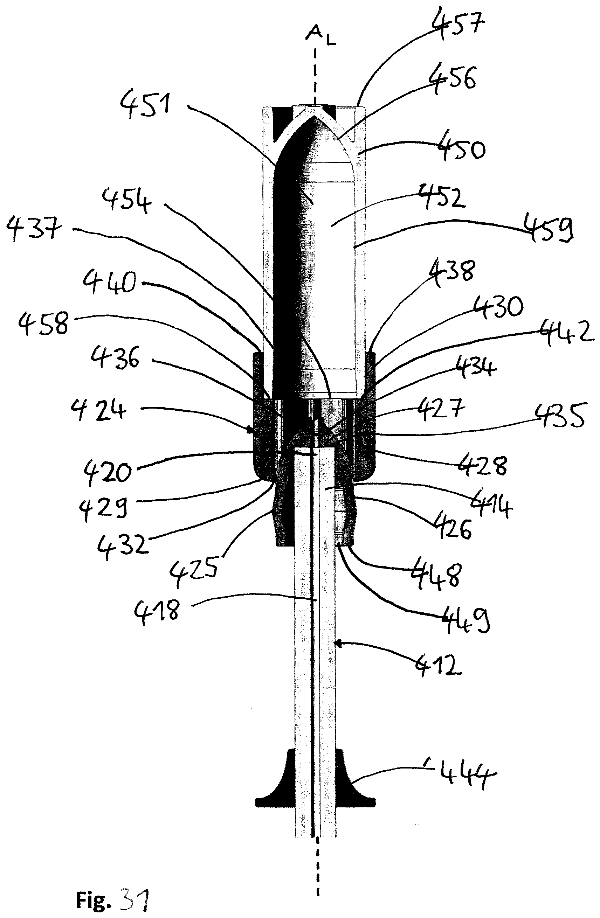

Fig. 31 shows a cross-sectional view of the discharger 410, wherein for the purpose of improved visualization, the distal end 416 as well as the distal end portion 446 of the discharge section 412 have been omitted.

Fig. 31 reveals that the distal portion 426 of the intermediate section 424 further comprises activation means 434 being enclosed by the proximal portion 428 of the intermediate section 424. The activation means 434 are in the shape of a piercing-tip having a dome-shaped contour 435 being essentially complementary to an inner wall portion 456 of the container 450. The activation means 434 further comprise a flow channel 436 providing a flow connection to an inlet opening 420 of a discharge passage 418 centrally running inside the discharge section 412 along the longitudinal axis A_{L}. The flow channel 436 is centrally located within the dome-shaped activation means 434 and further comprises two separated deformable ridge-like protrusions 437 located adjacent to an entry opening of the flow channel 436. The flow channel 436 extends along the longitudinal axis A_{L}.

Fig. 31 moreover reveals that the container 450 comprises a fluid reservoir 451 holding an amount of fluid 452 to be discharged. The container 450 further comprises a distal end face 458 being formed by a breakable seal 454. The inner wall portion 456 of an inner wall 459 of the container 450 is rotationally symmetric and approximately complementary to the dome-shaped contour 435 of the activation means 434.

In addition, it can be seen that the receiving space 430 is defined by an area between the receiving end 438 and a transition portion 442 serving as a stop for the container 450 when being loaded into the receiving space 430. Only roughly one seventh of the total length of the container 450 is received in the receiving space 430. The rest of the container 450 protrudes from the receiving end 438 of the proximal portion 428.

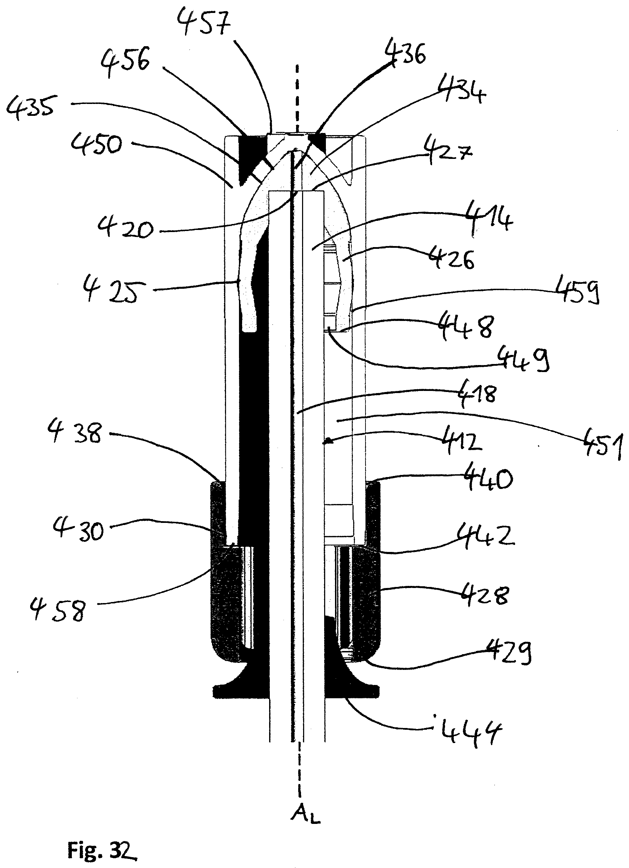

Fig. 32 shows the discharger 410 with the proximal portion 428 in its final discharge position, i.e. the proximal portion 428 has been fully moved towards the distal end 416 (cf. Fig. 30) of the discharge section 412. In this position, the breakable section 432 (cf. Figs. 1 and 2) is broken and the distal portion 426 of the intermediate section 424 is located in the fluid reservoir 451 of the container 450. The seal 454 has been pierced and the fluid 452 has been displaced by the activation means 434. The deformable ridge-like protrusions 437 (cf. Fig. 31) have been compressed such that the activation means 434 and the inner wall portion 456 of the container 450 establish a form-fitting connection.

It can be further seen, that the sealing lip 425 of the distal portion 426 tightly fits to the inner wall 459 of the container 450, thereby preventing fluid leakage of the container 450 past the receiving end 448 of the distal portion 426. The complementary shapes of the inner wall portion 456 of the container 450 and the contour 435 of the activation means 434 ensure that virtually no residual amounts of fluid 452 remain within the container 450.

For operating the discharger 410, i.e. to discharge the fluid 452 inside the container 450 through the discharge passage 418 out of the outlet opening 422, the proximal portion 428 together with the container 450 protruding from the receiving end 438 needs to be pushed towards the distal end 416.

When the proximal portion 428 is in an initial position as shown in Figs. 1 and 2, the breakable section 432 is still intact and the activation means 434 are still positioned spaced apart from the seal 454 of the container 450. In other words, the activation means 434 do not yet protrude into the fluid reservoir 451 of the container 450.

When applying an axial force to the proximal portion 428 of the intermediate section 424 by actuating the proximal end 457 of the container 450 like a push button which can be pushed by the user with a thumb while counteracting this actuation by holding the projections 444 with two fingers behind these projections 444, in a first step, the breakable section 432 breaks and the proximal portion 428 is transferred into its starting position. In the starting position the proximal portion 428 is moveable relative to the distal portion 426 along the longitudinal axis A_{L} towards the distal end 416 of the discharge section 412. In a second step, for discharging the fluid 452 the user has to deliberately push the container 450 towards the distal end 416.

In general, the movement of the proximal portion 428 between the initial position and the starting position and further towards the final discharge position preferably can be adapted to be a flowing movement, such that the fluid can be discharged uniformly.

While the proximal portion 428 together with the container 450 moves towards the distal end 416, the deformable ridge-like protrusions 437 initially punctuate the seal 454 while the dome-shaped activation means 434 then pierces the seal 454. The activation means 434 enter into the fluid reservoir 451 of the container 450, thereby urging the fluid 452 out of the container 450 through the flow channel 436 being connected to the discharge passage 418. Due to the complementary shapes of the activation means 434 and the inner wall portion 456 of the container 450 the fluid 452 can only escape through the flow channel 436. Since the distal portion 426 sealingly fits into the fluid reservoir 451 by means of the sealing lip 425, leakage of fluid past the distal portion 426 is prohibited at any time.

In general, the discharger 410 may be fabricated from any suitable material. Preferably, the material can be plastic. The material may be selected from the group comprising polypropylene, cyclic olefin polymer, polyethylene, polyamide, polybutylene terephthalate and polymethyl methacrylate. Alternatively, the material may be glass, metal or an alloy.

It is particularly preferred that the discharger 410, i.e. each of the container 450, the intermediate section 424 and the discharge section 412, may be fabricated by injection molding as injection-molded part.

With respect to one common classification applied in the medical, dental or veterinary field or the health care sector, the discharger as provided by the present disclosure belongs to the group consisting of systems without projection cap.

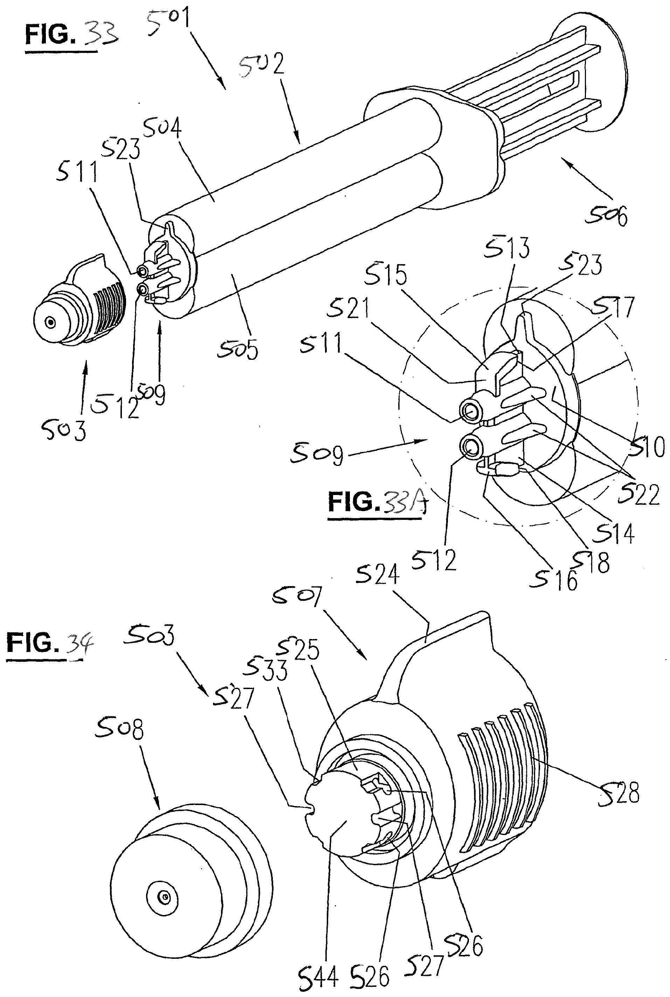

Dispensing device 501according to Fig. 33 includes includes a double syringe 502 and a mixing and spray head 503 that is attachable thereto. In a known manner, the double syringe comprises two storage containers 504 and 505 having equal volumes or equal cross-sectional areas, respectively, or cross-sectional areas whose ratio is different from 1:1. The two components are dispensed by means of a double plunger 506.

Mixing and spray head 503, hereinafter called "head" for the sake of simplicity, is essentially com-posed of three parts, namely a transfer housing 507 and a mixing and spray piece 4508 that may be connected to the transfer housing by snap means, welding, cementing, a screw connection or by means of a bayonet coupling.

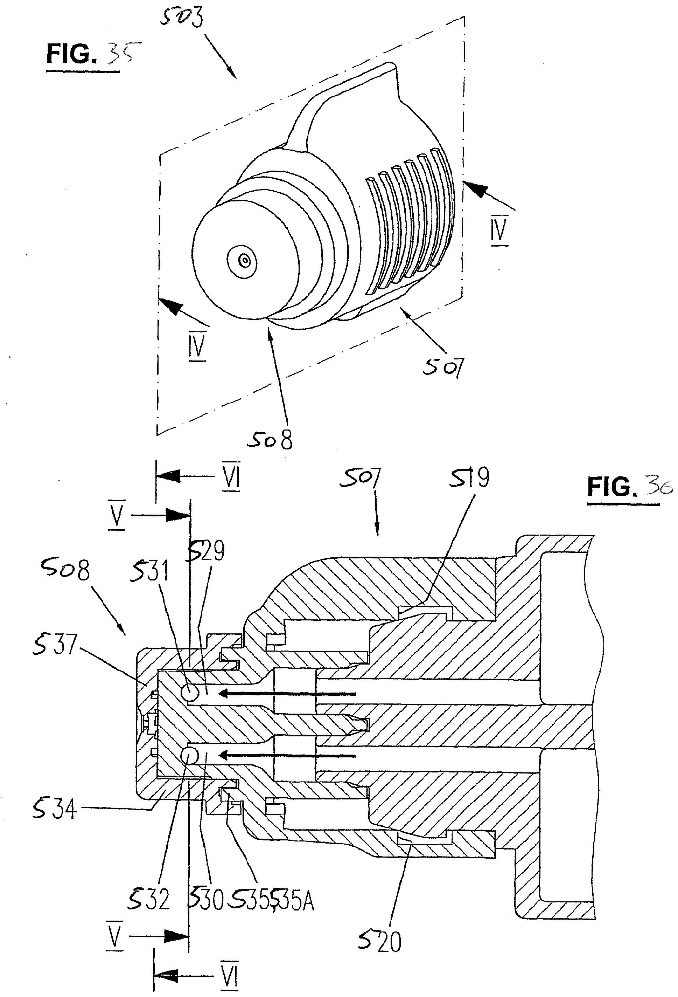

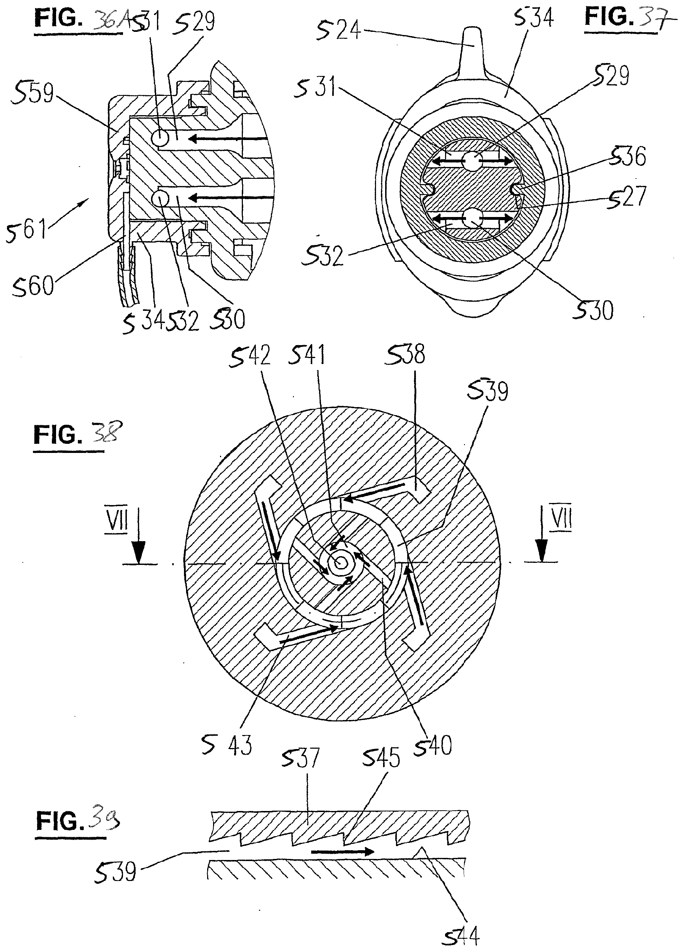

Basically, the head may be coupled to the dispensing appliance in different ways, for example by bayonet coupling means or by a screw connection. Advantageously, however, the head is simply plugged onto the dispensing appliance and easily removable therefrom by a connection as it is described in Swiss patent application no. 00453/06 to the applicant of the present technology. To this end, fastening area 509 on the outlet side of the" dispensing appliance has an outlet flange 510 on which the two individual outlets 511 and 512 are arranged. Each outlet 511 and 512 has an essentially diametrically arranged, outwardly directed ridge 513 respectively 514 that tapers from outlet flange 510 toward the outlet. Each ridge has a saddle portion 515 respectively 516 that ends at a distance from the outlet flange and is followed there by a snap nose 517 respectively 518, the snap noses engaging in recesses 519 and 520 in transfer housing 7, see Fig. 36.

The two saddle portions 515 and 516 have the same configuration, but saddle portion 515 has an end portion 521 on the outlet side while saddle portion 516 has none. The presence or absence of end portion 521 constitutes a coding means ensuring that the head can only be attached in an unequivocal orientation. Furthermore, on both sides of their connecting plane, the two outlets are provided with clamping guides 522. Additionally, outlet flange 510 has a visual coding nose 523 that serves as an orientation aid for the head, the latter having a corresponding orientation nose 524.

In Fig. 34, a head 503 consisting of two component parts is illustrated where mixing and spray piece 508 is fastened to transfer housing 507 while the end of the latter forms rear chamber bot-tom wall 544 of the mixing and swirl chambers. At its outlet side end, transfer housing 507 has a distributor socket 525 in which four distributor outlets 526 and two positioning grooves 527 are arranged. In the area of corrugation 528, the housing is compressible in order to disengage snap noses 517 and 518 from recesses 519 and 520, thereby allowing to withdraw the head.

As follows from Fig. 36, two longitudinal bores 529 and 530 are arranged in the distributor socket which communicate with transversal bores 530 and 532, these transversal bores leading to distributor outlets 526. The coding and orientation noses allow plugging the head onto the dispensing appliance in such a manner that longitudinal bores 529 and 530 are in line with outlets 511 and 512 of the dispensing appliance.

As appears in Fig. 354, longitudinal feed passages 533 extend from ends 526 of the transversal bores. In Fig. 36 it is shown that coupling portion 534 of mixing and spray piece 4508 has a circumferential groove 535 for receiving a corresponding collar 535A on the transfer housing. Housing portion 534 further comprises two positioning cams 536 which engage in corresponding positioning grooves 527. Closure 537 of mixing and spray piece 508 has four longitudinal channels 538 opening into an annular mixing chamber 539 from where four feed passages 540 lead to a concentric swirl chamber 541 from which spray outlet 542 leads to the exterior.

In Fig. 36A, an embodiment variant is illustrated in which a compressed air inlet 560 is arranged on end plate 450 of mixing and spray piece 561 in order to achieve a better spray action in certain cases. The other parts of the head are the same.

In Fig. 38 it is visible that feed passages 540 and feed ducts 543 do not form the same angle with respect to the mixing chamber and are offset from one another. Moreover, the feed passages and the feed ducts and the swirl and mixing chambers, respectively, do not have to be arranged in the same plane with respect to the longitudinal axis of the head.

In Fig. 39 it is apparent, that bottom wall 544 of the mixing chamber is plane while at the top, i.e. in closure 537, serrated mixing elements 45 are arranged which produce turbulences in order to mix the two components before they reach the swirl chamber from where they are sprayed through the spray outlet.

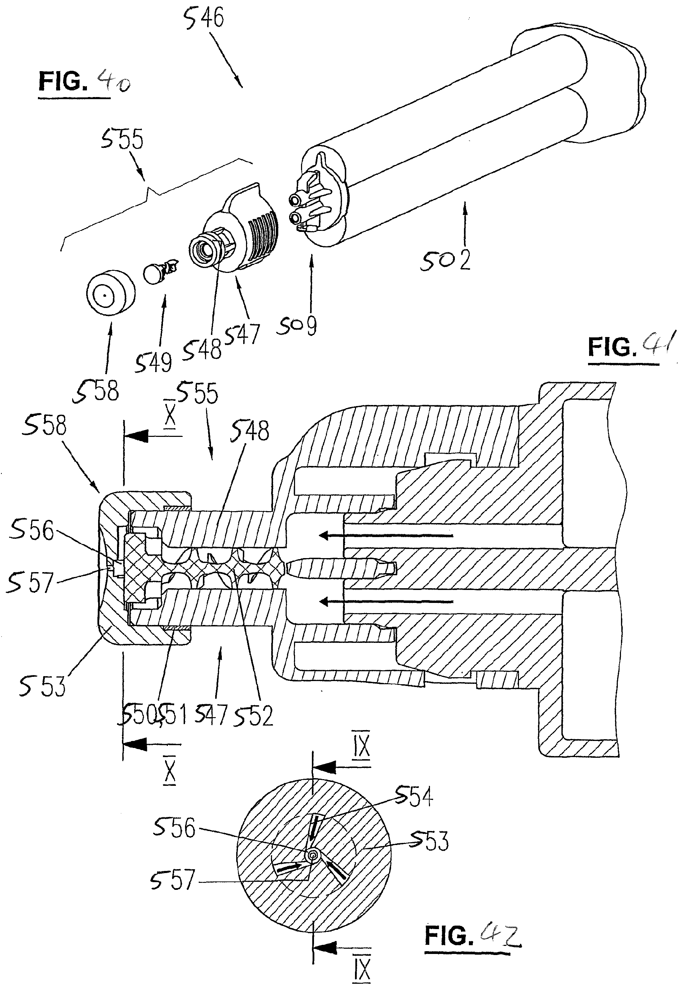

As mentioned in the introduction. the present technology essentially aims to provide a mixing assembly before the spray assembly, more particularly before the swirl chamber, in order to achieve an efficient mixture of the components prior to the actual spraying procedure. Instead of providing an annular, concentric mixing chamber having serrated mixing elements, it is also possible according to Figures 40 to 42 to provide a mixing assembly comprising a static mixing element as it is known per se. Dispensing device 546 according to Fig. 40 includes the same dispensing appliance 502 and the same fastening area as in the previous example. The fastening area of transfer housing 547 is the same as in the previous exemplary embodiment whereas mixer socket 548 is configured for receiving a mixing assembly 549 in its interior and fastening means 450, 551 on its exterior in order to attach mixing and spray piece 558 to transfer housing 5547, thereby forming mixing and spray head 555.

Mixing assembly 549 with mixing element 552 is fastened in the interior of closure 553 of mixing and spray piece 558 and forms a unit with the latter. Fastening means 550, 551 for the attachment of the mixing and spray piece to transfer housing 547 may be snap, bayonet, or screw connections, or it may be fastened by ultrasonic welding or cementing.

As follows from Fig. 42, closure 553 of the head has feed passages 554 arranged therein, e.g. three of them, which lead to a swirl chamber 556 in the same manner as in the previous example from where spray outlet 557 leads to the exterior.

Based on the two exemplary embodiments of a mixing chamber or of a mixing assembly, respectively, other possibilities of mixing assemblies can be designed by one skilled in the art by which the components, whose number may also be greater than two, are efficiently mixed shortly before the swirl chamber prior to being sprayed through the swirl chamber and the spray outlet.

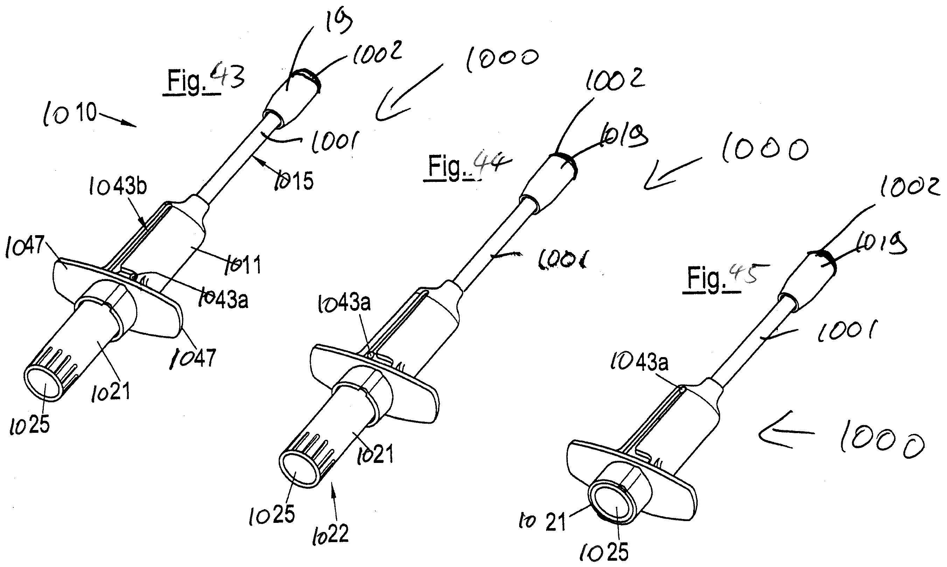

Figs. 43 to 55 show respective applicators 1100 for nasal decolonization using a fluid F. The applicator 1100 comprising an application tip 1019 adapted for insertion into a nasal cavity. The application tip 1019 is configured to store and release said fluid F. The applicator 1100 further comprises a support 1101 to which the application tip 1019 is connected or at which the application tip 1019 is integrally formed.

The application tip 1019 is formed from at least one of an open-cell foam, a plastic, a flockfibres can be of diff polymers such as polyester, cotton, polyester, rayon, calcium alginate, foam, knitted polyester, a medical grade plastic, a thermoplastic elastomer, low density polyethylene (LDPE), acrylonitrile butadiene styrene (ABS), polypropylene (PP), polyethylene (PE), high-density polyethylene (HDPE), polycarbonate (PC), polyurethane (PU), polyetheretherketone (PEEK), and polyvinyl alcohol (PVA).

An end 1102 of the application tip 1019 at least partly has at least one of a conical outer shape, an at least substantially conical outer shape, a truncated cone shape, an at least substantially truncated cone shape, a rounded shape, an at least substantially rounded shape, a spherical shape, an at least substantially spherical shape, an oval shape, an at least substantially oval shape, rounded edges and combinations of the foregoing, i.e. a shape which increases in size between the end 1102 and a middle portion of the application tip 1019.

Figs. 43 to 48, show a discharge system as an applicator 1100 comprising a dispenser 1010into which a separate container 1025 having a generally cylindrical outer shape can be loaded.

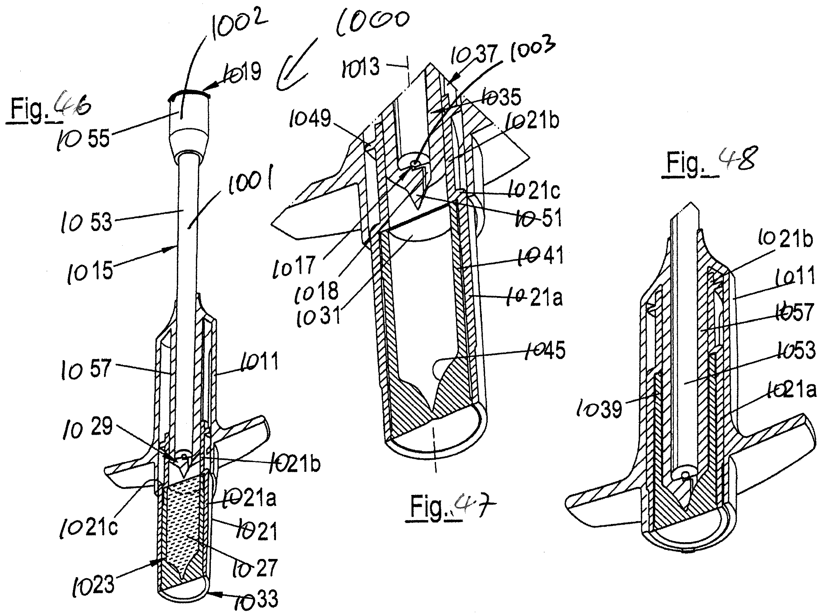

Although other applications are possible, in the embodiment shown the dispenser 1010 is intended for use in the medical, dental or veterinary field, and indeed for discharging a predetermined amount of fluid 1027 (Fig. 46) which is contained inside the container 1025. The fluid to be discharged is a liquid which includes one or more medical, dental or veterinary agents.

The dispenser and the container are designed as disposable articles intended for one-time use only.

The container 1025 is made as a capsule which has a generally cylindrical outer shape. The container 1025 comprises a wall portion 1041 (Fig. 47) with an integral proximal end portion providing a proximal end face, and with an opening at the distal end, this opening being closed by a breakable seal 1031 in the form of a foil. The wall portion 1041 and the seal 1031 confine a fluid space of the container which is at least partly filled with the fluid 1027. The amount of the fluid is for example 0.e5 ml or 2 ml.

In a ready-to-use state as shown in Fig. 43, the container 1025 has been loaded into a carriage 1021 of the dispenser 1010. To be loaded into the carriage 1025, the container 1025 is slid through a proximal insertion opening 1033 of the carriage 1021.

The carriage 1021 is received in a proximal end region of a housing 1011, 1011 which is provided with wing-like protrusions 1027 extending in opposite directions like in a common disposable syringe. Moreover, a slot 1043b is formed in the housing 1011, 1011 which cooperates with a pin 1043a formed at the carriage 1021 so as to form securing means which are explained later in more detail.

The dispenser 1010also comprises a discharge device 1015 from which Figs. 42 to 1045 show a tube 1053 (Fig. 46) extending from within the housing 1011, 1011 towards a thickened distal end portion 1055 (Fig. 46). The end portion 1055 can be integral with the tube 1053 or made as a separate component connected in a suitable manner to the tube 1053.

The dispenser 1010is adapted to discharge the fluid 1027 inside the container 1025 through the discharge device 1015 simply by pushing the carriage 1021 including the container 1025 into the housing 1011,. This process is described elsewhere in connection with Figs. 46 to 48.

The securing means 1043a, 1043b prevent the carriage 1021 from being pushed into the housing 1011, 1011 unintentionally. In an initial condition shown in Fig. 43, the pin 1043a of the carriage 1021 is situated at the end of a circumferential portion of the slot 1043b (Fig. 44). This condition prohibits a movement of the carriage 1021 along the longitudinal axis 1013 (Fig. 47) of the dispenser 1010.

In order to discharge the fluid, the user must deliberately rotate the carriage 1021 with respect to the housing 1011, 1011 so as to align the pin 1043a with an axial portion of the slot 1043b (Fig. 44). In order to facilitate the rotation of the carriage 1021, the outer wall of the carriage 1021 is provided with longitudinal grooves 1022.

In Fig. 44, the carriage 1021 is in a starting position and ready to be pushed into the housing 1011,.

Fig. 45 shows a condition in which the carriage 1021 is in a final discharge position in which the carriage 1021 and thus the loaded container 1025 are fully received within the housing 1011, the proximal end faces of the carriage 1021 and the container 1025 being flush with a proximal end face of the housing 1011.

From Figs. 43 to 45 it can be seen that the carriage 1021 and the container 1025 together can be actuated like a push button which is to be pressed by a user with her or his thumb while counteracting this pressing actuation by holding the housing with two fingers behind the projections 1047.

As shown in Fig. 46, the discharge device 1015 comprises a sleeve portion 1057 formed integrally with the housing 1011, 1011 and being arranged inside the housing 1011,. Further, the discharge device 1015 comprises the tube 1053 mentioned above which is fitted into the sleeve portion 1057.

A proximal end portion 1035 (Fig. 47) of the discharge device 1015 is fitted into a distal cylindrical portion 1021b of the carriage 1021 through a distal opening 1037 thereof. The proximal end portion 1035 comprises an inlet opening 1017 at the proximal end of the tube 1053, an inlet passage 1018 formed at the proximal end of the sleeve portion 1057 and activation means 1029 (Fig. 46) comprising a tip 1051 (Fig. 47) formed by a correspondingly tapering of the proximal end of the sleeve portion 1057. In the initial condition shown in Fig. 46, the tip 1051 is still positioned spaced apart from the seal 1031 of the container 1025, i.e. the activation means 1029 comprising the tip 1051 do not yet protrude into a proximal cylindrical portion 1021a of the carriage 1021 which defines a receiving space 1023 of the carriage 1021 for the container 1025.

An axial portion of the inlet passage 1018 extends offset from the longitudinal axis 1013 and leads to a radial portion of the inlet passage 1018 which leads into the inlet opening 1017 of the tube 1053.

A discharge passage extending through the tube 1053 connects the inlet opening 1017 with an application tip 1019 (Fig. 46) provided at the distal end of the discharge device 1015.

As can be seen especially in Figs. 46 and 48, the housing 1011, 1011 generally has a cylindrical outer shape with a constant inner diameter which is equal to the outer diameter of the proximal cylindrical portion 1021a of the carriage 1021. In order to conform also the distal cylindrical portion 1021b, the outer diameter of which being smaller than that of the proximal cylindrical portion 1021a, to the inner diameter of the housing 1011,, a flange 1049 is formed integrally with the distal cylindrical portion 1021b.

Moreover, the thickness of the wall portion 1041 of the container 1025 corresponds to the difference between the inner diameters of the cylindrical portions 1021a, 1021b of the carriage 1021. Consequently, the container 1025 reduces the inner diameter of the proximal cylindrical portion 1021a to that of the distal cylindrical portion 1021b, thereby establishing an inner space of the carriage 1021 having a constant inner diameter which corresponds to the outer diameter of the proximal end portion 1035 of the discharge device 1015.

This dimensioning of the individual components of the discharge system provides for a mechanically reliable mutual guiding of the individual components when the carriage 1021 including the container 1025 is pushed into the housing. Specifically, the carriage 1021 is guided by the inner wall of the housing 1011, 1011 through its flange 1049 and its proximal cylindrical portion 1021a. In addition, the carriage 1021 is guided by the discharge device 1015.

Moreover, the design of the individual components as explained above minimizes the required outer dimensions of the dispenser 1010, in particular the outer diameter of the housing 1011,. As can be seen in Fig. 48, there is no unused space inside the housing 1011,. When the carriage 1021 including the container 1025 is in the final discharge position, all components are concentrically packed within the housing 1011,.

A transition portion 1021c between the proximal cylindrical portion 1021a and the distal cylindrical portion 1021b of the carriage 1021 is formed as a circumferential shoulder which acts as an axial stop for the container 1025 when being loaded into the carriage 1021. The axial distance between the proximal end face of the proximal cylindrical portion 1021a and the transition portion 1021c corresponds to the axial length of the container 1025.

The outer shape of the tapered proximal end of the discharge device 1015 corresponds to a proximal end section 1045 of the inner wall of the container 1025 so that almost no free space remains inside the container 1025 when the carriage 1021 including the container 1025 is in its final discharged position as shown in Fig. 48. The complementary shapes of the discharge device 1015 and the container 1025 thus ensure that virtually no residual amounts of fluid remain within the container 1025.

Since in many countries pertinent regulations prohibit a simple throwing away of medical, dental or veterinary substances, the lack of substantial residual amounts of fluid as provided by the discharge system as described herein simplifies the disposal of used containers and dispensers in accordance with the respective national regulations.

In operation, in the initial state as shown in Fig. 43 the securing means 1043a, 1043b prevent the carriage 1021 from being unintentionally pushed into the housing 1011,.

For discharging the fluid included in the container 1025, which has previously been loaded into the carriage 1021, the user has to rotate the carriage 1021 in order to bring the pin 1043a into alignment with the axial portion of the slot 1043b (Fig. 44).

The carriage 1021 including the container 1025 can now be pushed into the housing 1011,, and indeed from the starting position shown in Fig. 44 into the final discharge position shown in Fig. 45.

When the carriage 1021 is being moved along the longitudinal axis 1013 into the housing 1011,, the seal 1031 of the container 1025 is broken by the tip 1051 of the activation means 1029 provided at the proximal end of the discharge device 1015. While the carriage 1021 continues its movement into the housing 1011,, the proximal end portion 1035 of the discharge device 1015 enters into the fluid space of the container 1025, thereby urging the fluid 1027 out of the container 1025. Since the proximal end portion 1035 is sealingly fitted into the distal cylindrical portion 1021b of the carriage 1021, the fluid can only escape through the inlet passage 1018 and thus through the discharge passage of the tube 1053 of the discharge device 1015.

Consequently, while the carriage 1021 is still on its way into the final discharge position, the fluid begins to flow through the inlet passage 1018 and the inlet opening 1017 and through the discharge passage so as to be dispensed from the application tip 1019.

Since the proximal end portion 1035 of the discharge device 1015 sealingly fits into the fluid space of the container 1025 as well, leakage of fluid past the discharge device 1015 is prohibited at any time.

Generally, the dispenser and/or the container may be fabricated from any suitable material. In one embodiment, the material is plastic. The material may be selected from the group comprising PP, COC, PE, PA, PBT and PMMA. Alternatively, the material may be glass, metal or an alloy.

From the above description, the skilled person will easily appreciate that the discharge system as described herein ensures a simple and reliable single-hand operation for discharging a predetermined amount of fluid such as a liquid containing at least one medical, dental or veterinary agent. Moreover, this discharge system allows for a long-term storage due to the usage of a separate fluid container so that it is no longer necessary to keep the fluid within the dispenser itself. Rather, the fluid can be stored over a long period of time within the container which can be closed by a breakable seal in a manner such that the fluid is very well protected from environmental influences.

With respect to one common classification applied in the medical, dental or veterinary field or the health care sector, the dispenser and the discharge system as provided for by the present disclosure belong to the group consisting of systems without protection cap.

The activation means 1029 of the dispenser 1010can be positioned inside the housing 1011, 1011 so as to protrude into the receiving space 1023 when the carriage 1021 is being moved from the starting position towards the final discharge position.

The activation means 1029 can be provided with means for breaking a seal 1031, in particular for piercing, penetrating, puncturing and/or perforating the seal 1031.

The carriage 1021 may have a proximal end having an insertion opening 1033, the receiving space 1023 being accessible through the insertion opening, and the carriage may be adapted to be loaded with the container 1025 by sliding the container through the insertion opening into the receiving space.

The discharge device 1015 may be adapted to guide the carriage 1021 along the longitudinal axis when the carriage is being moved from the starting position towards the final discharge position.

The discharge device 1015 may have a proximal end portion 1035 generally facing towards the receiving space 1023, the activation means 1029 and the inlet opening 1017 being formed at or being integrated into the proximal end region.

The discharge device 1015 may have a proximal end portion 1035 including the inlet opening 1017, and wherein the carriage 1021 has a distal opening 1037, the discharge device extending with its proximal end portion through the distal opening into the carriage, in particular wherein the proximal end portion 1035 of the discharge device 1015 may be sealingly fitted into the carriage 1021 so as to prevent at least substantially fluid leakage through the distal opening 1037 of the carriage.

The proximal end portion 1035 of the discharge device 1015 may have an outer diameter which is smaller than an inner diameter of the carriage 1021 in the area of the receiving space 1023 so as to provide a reception space 1039 for a wall portion 1041 of the container 1025 disposed around the proximal end portion of the discharge device when the carriage is being moved towards the final discharge position.

The carriage 1021 may generally have a multiple-cylinder shape at least comprising a proximal cylindrical portion 1021a and a distal cylindrical portion 1021b, the proximal cylindrical portion defining the receiving space 1023 for the container 1025, in particular wherein the proximal cylindrical portion 1021a of the carriage 1021 has an outer diameter which is greater than an outer diameter of the distal cylindrical portion 1021b of the carriage 1021.

The proximal cylindrical portion 1021a of the carriage 1021 may have an inner diameter which is greater than an inner diameter of the distal cylindrical portion 1021b of the carriage 1021.

Inside the carriage 1021, a transition portion 1021c may be provided between the proximal cylindrical portion 1021a and the distal cylindrical portion 1021b, the transition portion 1021c defining a distal end of the receiving space 1023 and serving as a stop for the container 1025 being loaded into the receiving space. The dispenser 1010may further comprise securing means 1043 adapted to prevent the carriage 1021 from being unintentionally moved from the starting position along the longitudinal axis 1013, in particular wherein the securing means 1043 may comprise a pin/slot-arrangement having at least one pin 1043a formed at the carriage 1021 and at least one slot 1043b formed in a wall 1011,a of the housing 1011,, the pin 1043a being guided by the slot 1043b, in particular wherein the slot 1043b may comprise a portion extending parallel to the longitudinal axis 1013 and a portion extending in a plane running not parallel to the longitudinal axis 1013.

The dispenser may be used for discharging a liquid including at least one medical, dental or veterinary agent, wherein in particular the amount of the liquid lies in the range of 0.2 to 2 ml and preferably is approximately 0.5 ml.

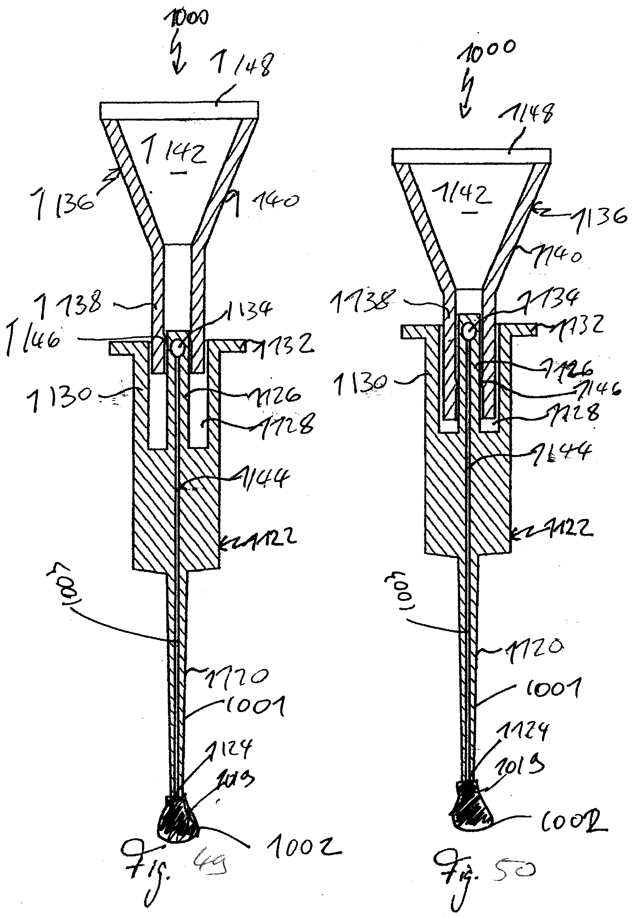

Figs. 49 and 50 show an applicator device 1120 which is used, for example, for the dispensing of a free-flowing fluid and comprises a carrier body 1122, to which the application tip 1019 is connected. The application device 1120 has a discharge opening 1124 for the supply of the fluid to the application tip 1019.

On the side disposed opposite to the application tip 1019, the carrier body 1122 has a cylindrical, piston-like pin 1126 which is surrounded by a ring-shaped recess 1128, which is in turn bordered on the outside by a peripheral wall 1130. A collar 1132 that acts as a gripping aid is in turn molded onto the peripheral wall 1130.

In the terminal area opposite from the application tip 1019, the pin 1126 is penetrated by a transverse channel 1134 that extends in the radial direction. From the transverse channel 1134, an axial channel 1144 which lies in the axis of the pin 1126 in turn branches off and runs to the discharge opening 1124 of the application device 14.

The applicator device 1120 further comprises a reservoir device 1136 in which the free-flowing substance can be stored. The reservoir device 1136 comprises a tube-shaped guide segment 1138 and a receptacle segment 1140 that is adjacent to the side farthest from the applicator device 1120. The receptacle segment 1140 defines a receptacle chamber 1142 for the free-flowing substance. The guide segment 1138, on its inside, has a ring-shaped edge seal 1146, which is located so that it can slide on the peripheral surface of the cylindrical pin 1126. The receptacle segment 36 is realized in the shape of a tube and has a sealing seam 1148 in its terminal area farther from the pin 1126. The reservoir device 1136 is fabricated from an elastically deformable material so that the receptacle segment 1140 can be compressed manually.

In the position of the reservoir device 1136 illustrated in FIG. 49, the edge seal 1146 is located in a terminal area of the peripheral surface of the pin 1126, so that the flow of the fluid between the receptacle chamber 1142 and the transverse channel 1134 is blocked. For the activation of the applicator device 1120, the reservoir device 1136 is moved into its open dispensing position which is illustrated in FIG. 50. During this process, the edge seal 1146 passes over the transverse channel 1134 so that a flow connection between the receptacle chamber 1142 and the transverse channel 1134 is created via an annular gap between the cylindrical pin 1126 and the guide segment 1138 of the reservoir device 1136. As the result of manual pressure which is applied to the side of the receptacle segment 1140, the free-flowing substance contained in the receptacle chamber 1142 can be displaced out of the receptacle chamber 1142 and transported via the transverse channel 1134 and the axial channel 1144 to the discharge opening 1124 of the application device 1120 and applied.

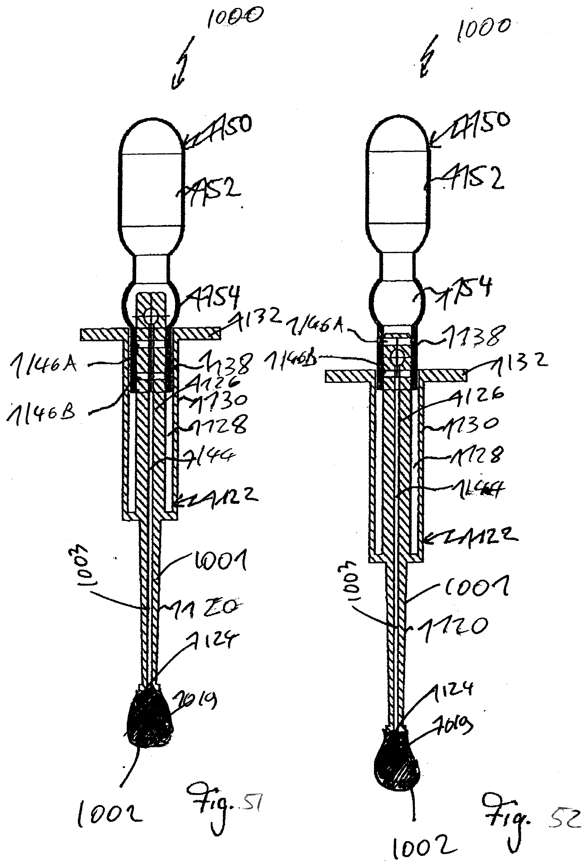

FIGS. 51 and 52 show an alternative realization of an applicator device 1100, which comprises a reservoir device 1150 which, instead of the reservoir device 1136 illustrated in FIG. 49, can be placed on the carrier body 1122.

The reservoir device 1150 comprises the tubular guide segment 1138 which on the inside has two ring-shaped edge seals 1146A and 1146B which interact with the cylindrical pin 1126 of the carrier body 1122. On the end farther from the edge seals 1146A and 1146B, a bubble-like receptacle segment 1152 is adjacent to the guide segment 1138, which receptacle segment 1152 contains the fluid to be applied and is elastically compressible.

In a middle area of the guide segment 1138, there is also a widened area 1154, which in the activation position of the reservoir device 1150 is located on the cylindrical pin of the carrier body 12 at the level of its transverse channel 1134. Advantageously, the widened area 1154 improves the flow behavior of the fluid and acts as a stop during the activation of the applicator device 10'. The function of the reservoir device 1150 and its interaction with the carrier body 1018 correspond to the realization illustrated in FIGS. 49 and 50.

The elastically deformable area of the receptacle segment 1152 of the application device 1120 may be in the form of a bubble.

The elastically deformable area of the receptacle segment 1152 of the application device 1120 may be in the form of a small tube.

The elastically deformable area of the receptacle segment 1152 of the application device 1120 may be in the form of a collapsible tube.

The reservoir device of the application device 1120 may include a guide segment having a widened area which during activation of the applicator device lies at the level of the transverse channel of the pin.

The reservoir device of the application device 1120 may include at least two receptacle bodies, one for each component of a multiple-component system.

The at least two receptacle bodies can be telescoped to create a flow connection, whereby at least one of the receptacle bodies has, on its inside, an edge seal which interacts with a peripheral surface of the other receptacle body.

The pin 1126 has at least one annular groove which interacts with the edge seal 1146 and defines a deactivation position and/or an activation position of the receptacle device.

The pin 1126 may be cylindrical.

The applicator device 1100 may comprise a carrier body 1122 which is provided with an application device 1120 and which, on the end farther from the application device 20 has a cylindrical pin 1126 which is penetrated by a transverse channel 26, from which an axial channel 28 branches off, which leads to the application device 1120, whereby on the cylindrical pin 1126 a reservoir device 1036, 1150 can be displaced which on its inside has a ring-shaped edge seal 1146 which interacts in a sealed manner with the cylindrical pin 1126, whereby the applicator device is activated by the displacement of the reservoir device 1136, 1150 on the cylindrical pin 1126 toward the application device 1120 to open a flow connection between the reservoir device 1036, 1150 and the transverse channel 1134. The reservoir device 1036, 1150 has a receptacle segment 1038, 1140, 1152 that is elastically deformable at least in portions so that, when a flow connection between the transverse channel 1036 and the receptacle segment 1038, 1140, 1152 exists in the activation position of the applicator device, the free-flowing substance is discharged via the application device 1120 by manual compression of the elastically deformable area of the receptacle segment 1138, 1140, 1152.

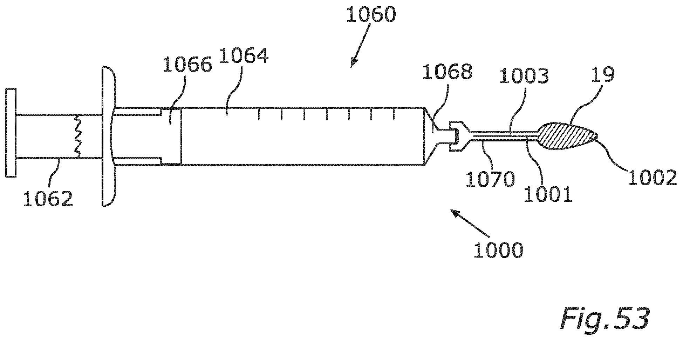

Fig. 53 shows the applicator 1100 having a syringe 1060 as the dispenser. The syringe 1060 comprises a plunger 1062 and a barrel 1064 in which the fluid can be stored. The plunger 1062 has a piston 1066 at its end disposed opposite of the end into which the plunger 1062 is insertable. The piston 1066 acts on the fluid during a dispensing of the fluid from the barrel 1064 via the application tip 1019 when a force is exerted on the plunger 1062 in the direction of the barrel 1064.

The syringe in the present example has a needle hub 1068 via which a rod 1070 carrying the application tip 1019 can be connected to the syringe 1060. It should be noted that the rod 1070 could also be integrally formed with the barrel 1064.

The applicator 1100 may thus further comprise a dispenser 1010, 1060, 1120, wherein the support 1101 is a rod comprising a passage 1003 and the passage 1003 is connected to an outlet of the dispenser 1010, 1060, 1120 for dispensing liquids from the container of the dispenser to the application tip 1019 for a supply of the fluid to the application tip 1019.

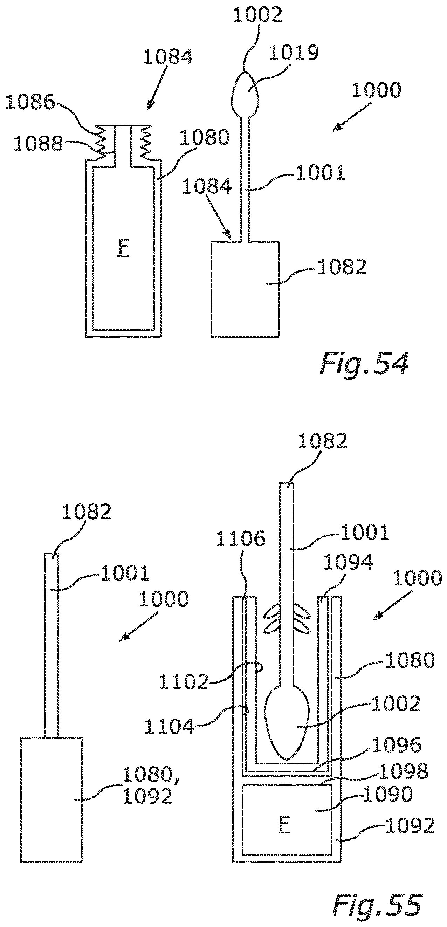

Fig. 54 shows a further type of applicator 1100. The applicator 1100 further comprises a container 1080, optionally filed with said fluid F, with the application tip 1019 being stored in the container 1080 and optionally in said fluid F prior to use of the application tip 1019.

The applicator 1100 comprises a handle 1082, with the handle 1082 being a part of a closure 1084 for the container 1080. The closure 1084 is a screw closure via which the handle 1082 can be releasably screwed to the container 1080. For this purpose, the handle 1082 comprises an internal thread that mates with an external thread 1086 present at a neck 1088 of the container 1080. The application tip 1019 and the rod are insertable into and removable from the container 1080. It should be noted that also other types of closure means can be employed, such as a bayonet type closure and the like.

The handle 1082 is a part of the support 1101. The handle 1082 can be integrally formed with the support 1101 or can be connected thereto after manufacture of the respective part.

The applicator 1100 of Fig. 54 can also be termed a lip-gloss type applicator 1100.

Fig. 55 shows a further type of applicator 1100. The container 1080 comprises two pieces of which one has a fluid reservoir for the fluid F. The two pieces of the container 1080 are an outer container 1092 and an inner container 1094.

In use of the applicator 1100 of Fig. 55 the handle 1082 is moved in the direction of the container 1080. This causes the application tip 1019 to penetrate a wall 1096 of the inner container 1094 which is directly adjacent to a wall 1098 of the outer container 1092 and the wall 1098 of the outer container 1092 to be pierced such that the application tip 1019 can enter the fluid reservoir 1090 and be wetted with the fluid in the reservoir 1090.

Rather than having two separate walls 1096, 1098 only the inner container 1094 or the outer container 1092 may have a respective wall that is pierced by the application tip 1019.

The support may comprise one or more seals 1100 which seal between the inner surface 1102 of the inner container 1094 and the support 1101.

Also no inner container 1092 need be present if the wall 1098 of the outer container 1092 is attached directly to the inner surface 1104 of the outer container 1092 or container 1080. In this instance the application tip would be stored in the space between the wall 1098 and an open end 1106 of the container 1080.

The walls 1096, 1098 act as a membrane 1108 which seals the fluid F in the fluid reservoir 1090.

The application tip 1019 shown in the foregoing may have a length selected in the range of 0.2 to 5 cm, preferably in the range of 0.4 to 3 cm, especially in the range of 0.5 to 2 cm.

The application tip 1019 shown in the foregoing may have a maximum outer diameter selected in the range of 0.2 to 3 cm, preferably in the range of 0.3 to 2 cm, especially in the range of 0.5 to 1.5 cm.

The larger the size of the application tip 1019 is, this may be used for a nasal cavity of e.g. a horse or a cow and the smaller dimensions for a cat or dog, whereas the middle ranges can be used for adult humans.

On use of the applicator 1100 the application tip 1019 thereof is inserted into the nasal cavity of a patient (not shown). The wetted application tip 1019 is then held in place and the fluid F stored therein is released by moving, e.g. rotating and pushing the application tip 1019 relative to the nasal cavity in order to coat this with the fluid F. Depending on the applicator 1100, the application tip 1019 already comprises the fluid F thereon and is inserted wet into the nasal cavity. If the application tip 1019 does not comprise the fluid F yet, as an applicator 1100 of one of the examples having a dispenser is used, then the application tip 1019 is inserted into the nasal cavity, where it is then wetted with the fluid F using the dispenser, thereafter the fluid F is distributed over the wall of the nasal cavity.

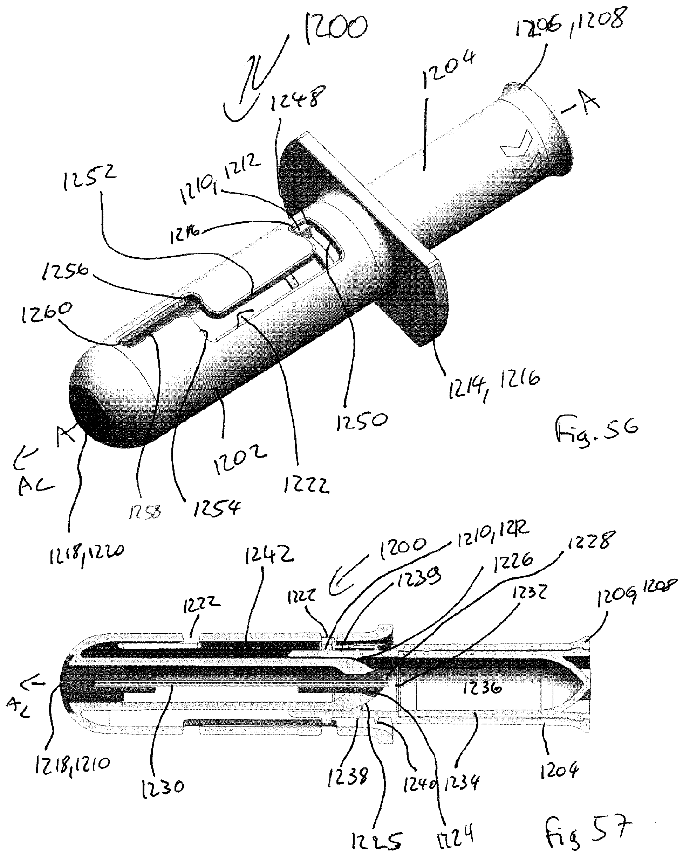

Fig. 56 shows yet a further perspective view of a further kind of discharger 1200. The discharger 1200 is formed by at least two parts, an outer housing component 1202 and an inner housing component 1204.

The inner housing component 1204 has a base 1206 via which the discharger 1200 can be gripped at one end 1208 thereof. The inner housing component 1204 further comprises a pin 1210 at a second end 1212 thereof. The first and second ends 1208, 1212 of the inner housing component 1204 are oppositely disposed from one another.

The outer housing component 1202 comprises a grip plate 1214 at a first end 1216 thereof. The outer housing component 1202 further comprises an outlet opening 1218 at a second end 1220 thereof. The first and second ends 1214, 1220 of the outer housing component 1202 are oppositely disposed from one another.

The first end 1208 of the inner housing component 1204 is similarly oppositely disposed of the second end 1220 of the outer housing component 1202.

The outlet opening 1218 is arranged at the longitudinal axis A_{L}. The second end 1220 of the outer housing component 1202 is configured to be inserted into respective nostrils of a patient. The outlet opening 1218 is consequently configured to spray the material stored therein into the respective nostril.

The outlet opening 1218 may be integrally formed with the outer housing component 1202 or be formed by an insert (not shown) insertable into the second end 1220 and connectable to a passage 1230 of the outer housing component (see Fig. 57), e.g. via a snap fit or press fit connection.

The outer housing component 1202 further comprises a guide passage 1222 in which the pin 1210 of the inner housing component 1204 is guided during a dispensing of the material stored in the discharger 1200.

The guide passage 1222 not only comprises a store 1246, but also comprises a first branch 1248, a second branch 1250, a third branch 1252, a fourth branch 1254, a receptacle 1256, a fifth branch 1258 and an end stop 1260.

The outer housing component further comprises a piercing tip 1224 as shown in Fig. 57. The piercing tip 1224 can be configured in the same way as the previously discussed piercing tips. Fig. 57 shows a schematic sectional drawing through the discharger 1200 of Fig. 56 along the sectional line A:A.

The inner housing component 1204 comprises a sealing lip 1222 configured to interact with an outer surface 1225 of the piercing tip 1224 in order to avoid material stored in the discharger from passing past the piercing tip 1224.

The piercing tip 1224 comprises an inlet 1228 connecting the passage 1230 to the outlet opening 1218. If the piercing tip 1224 is moved towards a reservoir 1236 in a manner as previously described the material stored in the reservoir 1236 is sprayed via the outlet opening 1218.

In the present example the reservoir 1236 is formed by a separate barrel 1234 that can be configured in the same way as those previously described. The barrel 1234 is sealed off via a membrane 1232 in order to safely store the material therein.

Rather than using a barrel the inner housing component 1204 may also comprise an integrally formed reservoir 1236 that is then sealed off via the membrane 1232.

The inner housing component further comprises guide noses 1238, 1240 projecting from an outer surface 1239 thereof. The guide noses 1238, 1240 engage an inner surface 1242 of the outer housing component 1202 and ensure that the inner housing component 1204 is guiding within the outer housing component 1204.

Moreover, the pin 1210 is visible as it projects into the outer housing component 1202 and specifically into the guide passage 1222.

The discharger 1200 may comprise a child safety element (not shown). The child safety element may be formed by a lug (also not shown) in the guide passage 1222. The lug may comprise a pre-determined breaking point that tears off, if a certain force is applied on the lug.

The lug would then be configured to hold the pin 1210 in the store 1246 of the guide passage 1222 of the discharger 1200.

On an initial activation of the discharger 1200, the inner housing component 1204 is moved away from the outer housing component 1202 by applying a force sufficient to break the pre-determined breaking point of the child safety element.

Following this the pin 1210 is moved into the first branch 1248. The pin is limited from moving axially too far by the second branch 1250. In order to move the inner housing component 1204 for a dispensing purpose the inner housing component 1204 has to be rotated relative to the outer housing component by guiding the pin in the second branch 1250.

The inner housing component can be rotated until the pin 1210 reaches the third branch 1252, following which no rotation of the inner housing component 1204 is possible and a user can axially move the inner housing component 1204 into the outer housing component 1202.

Thereby the piercing tip 1224 pierces the membrane 1232 and permits a dispensing of material while the inner housing component 1204 moves into the outer housing component 1202 for a length of the third branch 1252 until the pin 1210 reaches the fourth branch 1254.

Once the pin 1210 arrives at an end of the third branch 1250 the inner housing component 1204 cannot be moved axialy further into the outer housing component 1202 and in order to move the inner housing component 1204 further this has to be rotated relative to the outer housing component for a length of the fourth branch 1252.

The fourth branch 1252 ends in the fifth branch which comprises the receptacle 1256, and the end stop 1260 at oppositely disposed ends thereof. The pin 1210 can move into the receptacle as a pressure is released on the inner housing component 1204, e.g. if the discharger is moved from one nostril into the other and can then move towards the end stop 1260 when pressure is applied again.

During this movement from the third branch 1252 into the fifth branch 1258 a user can move the discharger 1200 from one nostril to another in order to apply the material stored therein at the different nostrils.

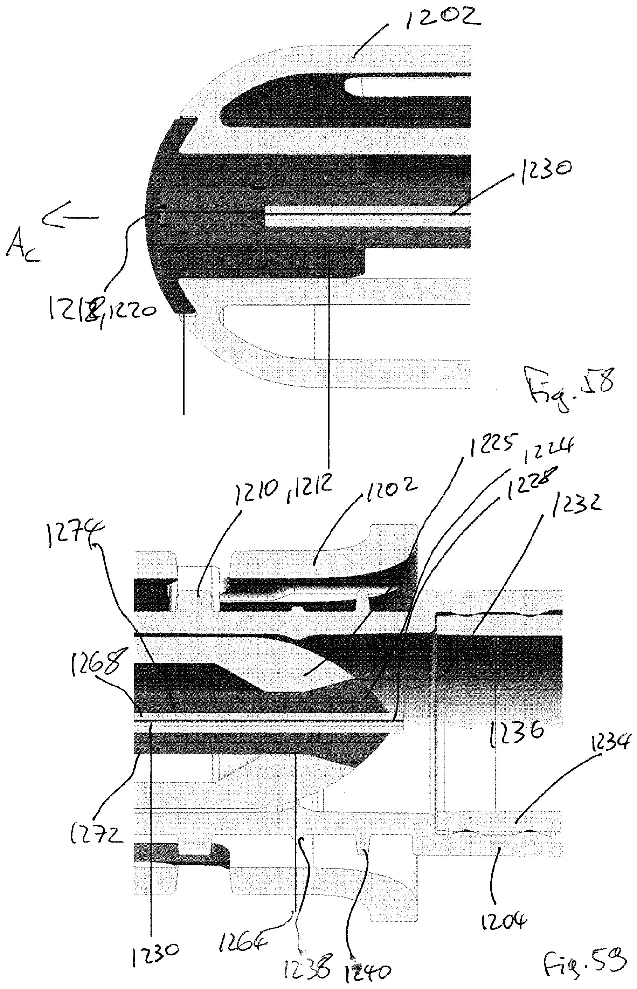

Fig. 58 shows a view of the second end 1220 of the outer component 1202. As shown the outlet opening 1218 is formed by a plastic insert 1270. This plastic insert is connected to the outer housing component 1202 by a fixed connection such as a laser weld 1266.

The insert 1270 further comprises a sealing surface 1262 sealing against an outer wall 1272 of the passage 1230. The passage 1230 is either integrally formed with the outer housing component 1202 or can be formed separate therefrom as shown in the present example.

As indicated in the present example the passage 1230 can be formed in a metal insert 1268. The metal insert is journaled at the second end 1220 via the sealing surface and at the first end 1216 of the outer housing component 1202 via a further sealing surface 1264 as indicated in Fig. 59.

The outlet opening 1218 may also be integrally formed with the outer housing component 1202.

In this connection it should be noted that the metal insert 1268 may comprise the piercing tip 1224 or the piercing tip 1224 may be injection molded around the metal insert 1268.

### List of reference numerals:

- 1: discharger
- 2: dispensing element
- 3: housing
- 4: container
- 5: distal end
- 6: proximal end
- 7: outlet
- 8: distal region
- 9: proximal region
- 10: static piston
- 10': central section of 10
- 11: wing-like projections
- 12: compartment
- 13: membrane
- 13': plane comprising 13
- 13": seal
- 13‴: front end
- 14: outer wall
- 14': end of 14
- 14": outer surface of 14
- 15: cut-out
- 16: slot
- 17, 17': longitudinal section
- 18: connection section
- 19, 19': pin
- 20: Luer lock
- 21: end
- 22: recess
- 23: inlet
- 23': end remote from inlet
- 24,: piercing tip
- 24', 24": end of 24
- 25: sealing lip
- 25': sealing element
- 26: sealing lip
- 26': sealing element
- 27: sealing lip
- 27': sealing element
- 28: cylindrical wall
- 28': outer surface of 28
- 29: sealing lip
- 29': sealing element
- 30: inner surface of 28
- 31: piercing tip receiving end
- 31': end of 12
- 31": point
- 32: inner wall
- 32': end of 32
- 33: groove
- 33': opening of 33
- 34: nose
- 35: ring
- 36: outer surface of 32
- 37: inner surface of 12
- 38: space
- 39: end
- 40: web
- 40': end of 33
- 41: wall
- 42: web
- 43: base
- 110: discharger
- 111: fluid
- 112: housing
- 113: sleeve portion
- 114: proximal end
- 115: proximal end portion
- 116: distal end
- 117: thickened distal end portion
- 118: discharge section
- 120: discharge passage
- 121: distal cylindrical portion
- 122: inlet opening
- 124: outlet opening
- 126: carriage
- 127: flange
- 128: receiving space
- 129: shoulder
- 130: piercing tip
- 131: outer surface
- 132: flow channel
- 133a, 33b: surface line
- 134: container
- 136: apex
- 138: inner contour
- 139: ridge
- 140: protrusion
- 141: fluid reservoir
- 142: seal
- 143: thorn-like protrusion
- 144: wing-like projections
- 146: pin
- 147: slot
- 148: securing means
- 149a: circumferential portion
- 149b: longitudinal portion
- 210: discharger
- 211: housing
- 213: longitudinal axis
- 215: discharge device
- 217: inlet opening
- 218: inlet passage
- 219: outlet opening
- 221: carriage
- 221a: proximal cylindrical portion
- 221b: distal cylindrical portion
- 221c: transition portion
- 222: grooves
- 223: receiving space
- 225: container
- 227: fluid
- 229: activation means
- 231: seal
- 233: insertion opening of the carriage
- 235: proximal end portion of the discharge device
- 237: distal opening of the carriage
- 239: reception space
- 241: wall portion
- 243a: pin
- 243b: slot
- 245: proximal end section of the inner wall
- 247: projection
- 249: flange
- 251: tip
- 253: tube
- 255: distal end portion of the discharge device
- 257: sleeve portion of the discharge device
- 301: syringe
- 302: hollow body
- 303: piston
- 304: intermediate piece
- 305: hollow body
- 321: hollow body surface
- 322: passage
- 323: rear body opening
- 324: front body opening
- 325: first needle seat
- 326: needle cannula
- 331: front piston opening
- 332: rear piston end
- 333: intermediate wall
- 334: receiving chamber
- 335: separating element, film
- 336: first chamber
- 337: second chamber
- 341: second needle seat
- 342: supply space
- 343: first sealing region
- 343a: first sealing region
- 344: inner element
- 345: outer element
- 346: abutment
- 347: seal
- 347a: seal
- 348: mandrel
- 349: edge
- 351: sealing region
- 352: spring
- 355: pivot means or deflection means, sloping surface, inclined plane
- 410: discharger
- 412: discharge section
- 414: proximal end
- 416: distal end
- 417: inlet opening
- 418: discharge passage
- 420: inlet opening
- 422: outlet opening
- 424: intermediate section
- 425: sealing lip
- 426: distal portion
- 427: stop portion
- 428: proximal portion
- 429: distal end
- 430: receiving space
- 432: breakable section
- 434: activation means
- 435: dome-shaped contour
- 436: flow channel
- 437: protrusions
- 438: receiving end
- 440: insertion opening
- 442: transition portion
- 444: projections
- 446: distal end portion
- 448: receiving end
- 449: opening
- 450: container
- 451: fluid reservoir
- 452: fluid
- 454: seal
- 456: inner wall portion
- 458: distal end face
- 457: proximal end
- 459: inner wall
- 1010: dispenser
- 1011: housing
- 1013: longitudinal axis
- 1015: discharge device
- 1017: inlet opening
- 1018: inlet passage
- 1019: application tip
- 1021: carriage
- 1021a: proximal cylindrical portion
- 1021b: distal cylindrical portion
- 1021c: transition portion
- 1022: grooves
- 1023: receiving space
- 1025: container
- 1027: fluid
- 1029: activation means
- 1031: seal
- 1033: insertion opening of the carriage
- 1035: proximal end portion of the discharge device
- 1037: distal opening of the carriage
- 1039: reception space
- 1041: wall portion
- 1043a: pin
- 1043b: slot
- 1045: proximal end section of the inner wall
- 1047: projection
- 1049: flange
- 1051: tip
- 1053: tube
- 1055: distal end portion of the discharge device
- 1057: sleeve portion of the discharge device
- 1060: syringe
- 1062: plunger
- 1064: barrel
- 1066: piston
- 1068: needle hub
- 1070: rod
- 1080: container
- 1082: handle
- 1084: closure
- 1086: external thread
- 1088: neck
- 1090: fluid reservoir
- 1092: outer container
- 1094: inner container
- 1096: wall
- 1098: wall
- 1100: seal
- 1102: inner surface of 1094
- 1104: inner surface of 1080
- 1106: open end of 1080
- 1108: membrane
- 1120: application device
- 1122: carriage
- 1124: discharge opening
- 1126: pin
- 1128: recess
- 1130: wall
- 1132: collar
- 1134: channel
- 1136: reservoir device
- 1138: segment
- 1140: segment
- 1142: chamber
- 1144: channel
- 1146, 1146A, 1146B: seal, seal, seal
- 1148: seam
- 1150: reservoir device
- 1152: segment
- 1154: area
- 1100: applicator
- 1101: support
- 1102: end
- 1103: passage
- 1200: discharger
- 1202: outer housing component
- 1204: inner housing component
- 1206: base
- 1208: first end of 1204
- 1210: pin
- 1212: second end of 1204
- 1214: grip plate
- 1216: first end of 1202
- 1218: outlet opening
- 1220: second end of 120
- 1222: guide passage
- 1224: piercing tip
- 1225: outer surface of 1224
- 1226: sealing lip
- 1228: inlet
- 1230: passage
- 1232: membrane
- 1234: barrel
- 1236: reservoir
- 1238: guide nose
- 1239: outer surface of 1204
- 1240: guide nose
- 1242: inner surface of 1202
- 1246: store
- 1248: first branch
- 1250: second branch
- 1252: third branch
- 1254: fourth branch
- 1256: receptacle
- 1258: fifth branch
- 1260: end stop
- 1262: sealing surface
- 1264: sealing surface
- 1266: laser weld
- 1268: metal insert
- 1270: plastic insert
- 1272: wall of 1230

- A₁: longitudinal first axis
- A₂: longitudinal second axis
- A_{L}: longitudinal axis
- A: longitudinal axis
- B: arrow
- F: fluid
- L: length

## Claims

1. A discharger (1) that comprises a dispensing element (2) and a housing (3), the housing (3) having a static piston (10) connected to the dispensing element (2) at least in a fluid conducting manner and with the static piston (10) being arranged within the housing (3); the discharger (1) further comprising a container (4) containing a fluid (F), the container (4) being moveable relative to the static piston (10) and the housing (3), with the static piston (10) comprising a piercing tip (24) adapted to pierce a membrane (13) or seal of the container (4) when the container (4) is moved towards the housing (3), with the static piston (10) further comprising at least one sealing element (25', 26', 27') provided in the region of the piercing tip (24), with the at least one sealing element (25', 26', 27') providing a seal between the piercing tip (24) and an inner surface (37) of the container (4) when the moveable container (4) is moved relative to the housing (3) into a position in which the membrane (13) or seal is pierced and in which the at least one sealing element (25', 26', 27') engages the inner surface (37) to prevent the fluid (F) from passing between the at least one sealing element (25', 26', 27') and the inner surface (37) and into a part of the housing (3), wherein the Fluid (F) is a medical fluid, especially a medical fluid comprising lipid nanoparticles.

2. A discharger in accordance with claim 1, wherein the at least one sealing element (25', 26', 27') is arranged at an end (24') of the piercing tip (24) remote from the other end (24") of the piercing tip (24) comprising an inlet (23) to the dispensing element (2).

3. A discharger in accordance with claim 2, wherein the membrane (13) or seal is configured to be pierced by said other end of the piercing tip (24) comprising the inlet (23).

4. A discharger in accordance with at least one of the preceding claims, wherein the at least one sealing element is a sealing lip (25, 26, 27).

5. A discharger in accordance with at least one of the preceding claims, wherein the first sealing element (25') has a diameter that is smaller than a diameter of a second sealing element (26'), and if a third sealing element (27') is provided then the first sealing element (25') has a diameter that is smaller than a diameter of the third sealing element (27'), with the second and third sealing elements (26', 27') preferably having an at least substantially identical diameter.

6. A discharger in accordance with claim 5, wherein an external diameter of the first sealing element (25') is less than or equal to an internal diameter of the container (4) and an external diameter of the second sealing element (26') and optionally of the third sealing element (27') is larger than the internal diameter of the container (4).

7. A discharger in accordance with at least one of the preceding claims, wherein first, second and preferably third sealing lips (25, 26, 27) are provided that are all configured to engage the inner surface (37) to prevent the fluid (F) from passing into said part of the housing (3).

8. A discharger in accordance with claim 7, wherein the first, second and preferably third sealing lips (25, 26, 27) are arranged one after the other in parallel to one another along a longitudinal axis (A) of the discharger (1).

9. A discharger in accordance with at least one of the preceding claims 2 to 8, wherein a diameter of the piercing tip reduces from the at least one sealing element (25', 26', 27') to said other end (24") of the piercing tip (24) comprising the inlet (23).

10. A discharger in accordance with at least one of the preceding claims, wherein the container (4) comprises a compartment (12) in its interior in which the fluid (F) is initially stored prior to piercing the membrane (13) or seal, and wherein the inner surface (37) is present at a wall (32) of the compartment (12).

11. A discharger in accordance with claim 10, wherein the container (4) further comprises an outer wall (14) arranged in parallel to the wall (32) of the compartment (12) and a guide groove (33) present between the outer wall (14) and the wall (32) of the compartment (12), with said guide groove (33) receiving at least some of the housing (3).

12. A discharger in accordance with claim 10 or claim 11, wherein the compartment (12) comprises an end (31') that is disposed opposite to the membrane (13) or seal and that is configured substantially complementary to the shape of the piercing tip (24).

13. A discharger in accordance with at least one of the preceding claims, wherein the discharger comprises a child safety lock.

14. A discharaer in accordance with claim 13, wherein the child safety lock is formed between the container (4) and the housing (3).

15. A discharger in accordance with at least one of the preceding claims, wherein the at least one sealing element (25', 26', 27') does not contact the housing (3) in a storage state of the discharger (1) and with the at least one sealing element (25', 26', 27') only contacting the inner surface (37) in a discharging state or discharged state of the discharger (1).

16. A discharger in accordance with at least one of the preceding claims, wherein an inner surface (30) of a cylindrical wall (28) of the housing (3) comprises a further inwardly projecting sealing lip (29) that is configured to engage a surface (36) of the container (4) different from the inner surface (37).

17. A method of discharging a fluid (F) from a discharger (1), in particular in accordance with at least one of the claims 1 to 16, the method comprising the steps of:
- guiding a container (4) in a direction along a longitudinal axis (A) of the discharger (1) towards a piercing tip (24) of a static piston (10) accommodated within a housing (3) of the discharger (1),
- piercing a membrane (13) or seal present at the container (4) by means of the piercing tip (24) causing the membrane (13) or seal to be pierced, in particular initially at a center of the membrane (13) or seal,
- guiding the container (4) further towards the static piston (10) and bringing an inner surface (37) of the container (4) into engagement with at least one sealing element (25', 26', 27') arranged at the piercing tip (24) of the static piston (10) to prevent the fluid (F) from passing between the at least one sealing element (25', 26', 27') and the inner surface (37) and thereby into a part of the housing (3), and
- discharging the fluid (F) via an outlet (7) of the discharger (1), wherein the Fluid (F) is a medical fluid, especially a medical fluid comprising lipid nanoparticles.

## Patentansprüche

1. Ausgabevorrichtung (1), die ein Abgabeelement (2) und ein Gehäuse (3) umfasst, wobei das Gehäuse (3) einen stationären Kolben (10) aufweist, der zumindest fluidleitend mit dem Abgabeelement (2) verbunden ist und wobei der stationäre Kolben (10) innerhalb des Gehäuses (3) angeordnet ist; wobei die Ausgabevorrichtung (1) ferner einen Behälter (4) umfasst, der ein Fluid (F) enthält, wobei der Behälter (4) relativ zu dem stationären Kolben (10) und dem Gehäuse (3) bewegbar ist, wobei der stationäre Kolben (10) eine Durchstechspitze (24) umfasst, die dazu ausgelegt ist, eine Membran (13) oder Dichtung des Behälters (4) zu durchstechen, wenn der Behälter (4) in Richtung auf das Gehäuse (3) bewegt wird, wobei der stationäre Kolben (10) ferner mindestens ein Dichtelement (25', 26', 27') umfasst, das im Bereich der Durchstechspitze (24) vorgesehen ist, wobei das mindestens eine Dichtelement (25', 26', 27') eine Abdichtung zwischen der Durchstechspitze (24) und einer Innenfläche (37) des Behälters (4) bereitstellt, wenn der bewegbare Behälter (4) relativ zu dem Gehäuse (3) in eine Position bewegt wird, in der die Membran (13) oder Dichtung durchstochen ist und in der das mindestens eine Dichtelement (25', 26', 27') mit der Innenfläche (37) in Eingriff steht, um zu verhindern, dass das Fluid (F) zwischen dem mindestens einen Dichtelement (25', 26', 27') und der Innenfläche (37) und in einen Teil des Gehäuses (3) hindurchtritt, wobei das Fluid (F) ein medizinisches Fluid, insbesondere ein medizinisches Fluid, das Lipidnanopartikel umfasst, ist.

2. Ausgabevorrichtung nach Anspruch 1, wobei das mindestens eine Dichtelement (25', 26', 27') an einem Ende (24') der Durchstechspitze (24) angeordnet ist, das von dem anderen Ende (24") der Durchstechspitze (24), das einen Einlass (23) zu dem Abgabeelement (2) umfasst, entfernt angeordnet ist.

3. Ausgabevorrichtung nach Anspruch 2, wobei die Membran (13) oder Dichtung eingerichtet ist, durch das andere Ende der Durchstechspitze (24), das den Einlass (23) umfasst, durchstochen zu werden.

4. Ausgabevorrichtung nach einem der vorstehenden Ansprüche, wobei das mindestens eine Dichtelement eine Dichtlippe (25, 26, 27) ist.

5. Ausgabevorrichtung nach einem der vorstehenden Ansprüche, wobei das erste Dichtelement (25') einen Durchmesser aufweist, der kleiner als ein Durchmesser eines zweiten Dichtelements (26') ist, und wenn ein drittes Dichtelement (27') vorgesehen ist, dann das erste Dichtelement (25') einen Durchmesser aufweist, der kleiner als ein Durchmesser des dritten Dichtelements (27') ist, wobei die zweiten und dritten Dichtelemente (26', 27') vorzugsweise mindestens im Wesentlichen identische Durchmesser aufweisen.

6. Ausgabevorrichtung nach Anspruch 5, wobei ein Außendurchmesser des ersten Dichtelements (25') kleiner als oder gleich einem Innendurchmesser des Behälters (4) ist und ein Außendurchmesser des zweiten Dichtelements (26') und gegebenenfalls des dritten Dichtelements (27') größer als der Innendurchmesser des Behälters (4) ist.

7. Ausgabevorrichtung nach einem der vorstehenden Ansprüche, wobei erste, zweite und vorzugsweise dritte Dichtlippen (25, 26, 27) vorgesehen sind, die alle eingerichtet sind, mit der Innenfläche (37) in Eingriff zu stehen, um zu verhindern, dass das Fluid (F) in den Teil des Gehäuses (3) hindurchtritt.

8. Ausgabevorrichtung nach Anspruch 7, wobei die erste, zweite und vorzugsweise dritte Dichtlippe (25, 26, 27) nacheinander parallel zueinander entlang einer Längsachse (A) der Ausgabevorrichtung (1) angeordnet sind.

9. Ausgabevorrichtung nach einem der vorstehenden Ansprüche 2 bis 8, wobei ein Durchmesser der Durchstechspitze von dem mindestens einen Dichtelement (25', 26', 27') bis zu dem anderen Ende (24") der Durchstechspitze (24), das den Einlass (23) umfasst, abnimmt.

10. Ausgabevorrichtung nach einem der vorstehenden Ansprüche, wobei der Behälter (4) eine Kammer (12) in seinem Inneren umfasst, in der das Fluid (F) vor dem Durchstechen der Membran (13) oder Dichtung anfänglich gespeichert ist, und wobei die Innenfläche (37) an einer Wand (32) der Kammer (12) vorhanden ist.

11. Ausgabevorrichtung nach Anspruch 10, wobei der Behälter (4) ferner eine Außenwand (14), die parallel zu der Wand (32) der Kammer (12) angeordnet ist, und eine Führungsnut (33), die zwischen der Außenwand (14) und der Wand (32) der Kammer (12) vorhanden ist, umfasst, wobei die Führungsnut (33) zumindest einen Teil des Gehäuses (3) aufnimmt.

12. Ausgabevorrichtung nach Anspruch 10 oder Anspruch 11, wobei die Kammer (12) ein Ende (31') umfasst, das der Membran (13) oder Dichtung gegenüberliegt und das eingerichtet ist, im Wesentlichen komplementär zu der Form der Durchstechspitze (24) zu sein.

13. Ausgabevorrichtung nach einem der vorstehenden Ansprüche, wobei die Ausgabevorrichtung eine Kindersicherung umfasst.

14. Ausgabevorrichtung nach Anspruch 13, wobei die Kindersicherung zwischen dem Behälter (4) und dem Gehäuse (3) ausgebildet ist.

15. Ausgabevorrichtung nach einem der vorstehenden Ansprüche, wobei das mindestens eine Dichtelement (25', 26', 27') das Gehäuse (3) in einem Lagerzustand der Ausgabevorrichtung (1) nicht berührt und wobei das mindestens eine Dichtelement (25', 26', 27') die Innenfläche (37) nur in einem Ausgabezustand oder einem ausgegebenen Zustand der Ausgabevorrichtung (1) berührt.

16. Ausgabevorrichtung nach einem der vorstehenden Ansprüche, wobei eine Innenfläche (30) einer zylindrischen Wand (28) des Gehäuses (3) eine weitere nach innen vorstehende Dichtlippe (29) umfasst, die eingerichtet ist, mit einer Oberfläche (36) des Behälters (4), die sich von der Innenfläche (37) unterscheidet, in Eingriff zu stehen.

17. Verfahren zum Ausgeben eines Fluids (F) aus einer Ausgabevorrichtung (1), insbesondere nach mindestens einem der Ansprüche 1 bis 16, wobei das Verfahren die Schritte umfasst:
- Führen eines Behälters (4) in einer Richtung entlang einer Längsachse (A) der Ausgabevorrichtung (1) in Richtung auf eine Durchstechspitze (24) eines stationären Kolbens (10), der innerhalb eines Gehäuses (3) der Ausgabevorrichtung (1) aufgenommen ist,
- Durchstechen einer am Behälter (4) vorhandenen Membran (13) oder Dichtung mittels der Durchstechspitze (24), wodurch die Membran (13) oder Dichtung, insbesondere zunächst in einer Mitte der Membran (13) oder Dichtung, durchstochen wird,
- weiteres Führen des Behälters (4) in Richtung auf den stationären Kolben (10) und Bringen einer Innenfläche (37) des Behälters (4) mit mindestens einem Dichtelement (25', 26', 27'), das an der Durchstechspitze (24) des stationären Kolbens (10) angeordnet ist, in Eingriff, um zu verhindern, dass das Fluid (F) zwischen dem mindestens einen Dichtelement (25', 26', 27') und der Innenfläche (37) und dadurch in einen Teil des Gehäuses (3) hindurchtritt, und
- Ausgeben des Fluids (F) über einen Auslass (7) der Ausgabevorrichtung (1), wobei das Fluid (F) ein medizinisches Fluid, insbesondere ein medizinisches Fluid, das Lipidnanopartikel umfasst, ist.

## Revendications

1. Dispositif d'écoulement (1) comprenant un élément de distribution (2) et un boîtier (3), le boîtier (3) présentant un piston statique (10) connecté à l'élément de distribution (2) au moins de manière à conduire un fluide et le piston statique (10) étant agencé à l'intérieur du boîtier (3) ; le dispositif d'écoulement(1) comprenant en outre un récipient (4) contenant un fluide (F), le récipient (4) étant mobile par rapport au piston statique (10) et au boîtier (3), le piston statique (10) comprenant une pointe de perçage (24) adaptée pour percer une membrane (13) ou un joint du récipient (4) lorsque le récipient (4) est déplacé vers le boîtier (3), le piston statique (10) comprenant en outre au moins un élément d'étanchéité (25', 26', 27') prévu dans la région de la pointe de perçage (24), ledit au moins un élément d'étanchéité (25', 26', 27') assurant une étanchéité entre la pointe de perçage (24) et une surface intérieure (37) du récipient (4) lorsque le récipient mobile (4) est déplacé par rapport au boîtier (3) dans une position dans laquelle la membrane (13) ou le joint est percé et dans laquelle ledit au moins un élément d'étanchéité (25', 26', 27') vient en prise avec la surface intérieure (37) pour empêcher le fluide (F) de passer entre ledit au moins un élément d'étanchéité (25', 26', 27') et la surface intérieure (37) et dans une partie du boîtier (3), dans lequel le fluide (F) est un fluide médical, en particulier un fluide médical comprenant des nanoparticules lipidiques.

2. Dispositif d'écoulement selon la revendication 1, dans lequel ledit au moins un élément d'étanchéité (25', 26', 27') est agencé à une extrémité (24') de la pointe de perçage (24) éloignée de l'autre extrémité (24") de la pointe de perçage (24) comprenant une entrée (23) vers l'élément de distribution (2).

3. Dispositif d'écoulement selon la revendication 2, dans lequel la membrane (13) ou le joint est configuré(e) pour être percé(e) par ladite autre extrémité de la pointe de perçage (24) comprenant l'entrée (23).

4. Dispositif d'écoulement selon l'une des revendications précédentes, dans lequel ledit au moins un élément d'étanchéité est une lèvre d'étanchéité (25, 26, 27).

5. Dispositif d'écoulement selon l'une des revendications précédentes, dans lequel le premier élément d'étanchéité (25') a un diamètre qui est inférieur à un diamètre d'un deuxième élément d'étanchéité (26'), et si un troisième élément d'étanchéité (27') est prévu, alors le premier élément d'étanchéité (25') a un diamètre qui est inférieur à un diamètre du troisième élément d'étanchéité (27'), les deuxième et troisième éléments d'étanchéité (26', 27') ayant de préférence un diamètre au moins sensiblement identique.

6. Dispositif d'écoulement selon la revendication 5, dans lequel un diamètre extérieur du premier élément d'étanchéité (25') est inférieur ou égal à un diamètre intérieur du récipient (4) et un diamètre extérieur du deuxième élément d'étanchéité (26') et éventuellement du troisième élément d'étanchéité (27') est supérieur au diamètre intérieur du récipient (4).

7. Dispositif d'écoulement selon l'une au moins des revendications précédentes, dans lequel des première, deuxième et de préférence troisième lèvres d'étanchéité (25, 26, 27) sont prévues qui sont toutes configurées pour venir en prise avec la surface intérieure (37) afin d'empêcher le fluide (F) de passer dans ladite partie du boîtier (3).

8. Dispositif d'écoulement selon la revendication 7, dans lequel les première, deuxième et de préférence troisième lèvres d'étanchéité (25, 26, 27) sont agencées l'une après l'autre parallèlement les unes aux autres le long d'un axe longitudinal (A) du dispositif d'écoulement (1).

9. Dispositif d'écoulement selon l'une au moins des revendications précédentes 2 à 8, dans lequel un diamètre de la pointe de perçage se réduit depuis ledit au moins un élément d'étanchéité (25', 26', 27') jusqu'à ladite autre extrémité (24") de la pointe de perçage (24) comprenant l'entrée (23).

10. Dispositif d'écoulement selon l'une au moins des revendications précédentes, dans lequel le récipient (4) comprend un compartiment (12) dans son intérieur dans lequel le fluide (F) est initialement stocké avant le perçage de la membrane (13) ou du joint, et dans lequel la surface intérieure (37) est présente au niveau d'une paroi (32) du compartiment (12).

11. Dispositif d'écoulement selon la revendication 10, dans lequel le récipient (4) comprend en outre une paroi extérieure (14) agencée parallèlement à la paroi (32) du compartiment (12) et une rainure de guidage (33) présente entre la paroi extérieure (14) et la paroi (32) du compartiment (12), ladite rainure de guidage (33) recevant au moins une partie du boîtier (3).

12. Dispositif d'écoulement selon la revendication 10 ou la revendication 11, dans lequel le compartiment (12) comprend une extrémité (31') qui est disposée à l'opposé de la membrane (13) ou du joint et qui est configurée de manière sensiblement complémentaire à la forme de la pointe de perçage (24).

13. Dispositif d'écoulement selon l'une au moins des revendications précédentes, dans lequel le dispositif d'écoulement comprend une sécurité enfant.

14. Dispositif d'écoulement selon la revendication 13, dans lequel la sécurité enfant est formée entre le récipient (4) et le boîtier (3).

15. Dispositif d'écoulement selon l'une au moins des revendications précédentes, dans lequel ledit au moins un élément d'étanchéité (25', 26', 27') ne vient pas en contact avec le boîtier (3) dans un état de stockage du dispositif d'écoulement (1) et ledit au moins un élément d'étanchéité (25', 26', 27') n'est en contact qu'avec la surface intérieure (37) dans un état d'écoulement ou un état écoulé du dispositif d'écoulement (1).

16. Dispositif d'écoulement selon l'une au moins des revendications précédentes, dans lequel une surface intérieure (30) d'une paroi cylindrique (28) du boîtier (3) comprend une lèvre d'étanchéité supplémentaire faisant saillie vers l'intérieur (29) qui est configurée pour venir en prise avec une surface (36) du récipient (4) différente de la surface intérieure (37).

17. Procédé d'écoulement d'un fluide (F) à partir d'un dispositif d'écoulement (1), en particulier selon l'une au moins des revendications 1 à 16, le procédé comprenant les étapes consistant à :
- guider un récipient (4) dans une direction le long d'un axe longitudinal (A) du dispositif d'écoulement (1) vers une pointe de perçage (24) d'un piston statique (10) logé à l'intérieur d'un boîtier (3) du dispositif d'écoulement (1),
- percer une membrane (13) ou un joint présent au niveau du récipient (4) au moyen de la pointe de perçage (24), ce qui provoque le perçage de la membrane (13) ou du joint, en particulier initialement au niveau d'un centre de la membrane (13) ou du joint,
- guider davantage le récipient (4) vers le piston statique (10) et mettre une surface intérieure (37) du récipient (4) en prise avec au moins un élément d'étanchéité (25', 26', 27') agencé au niveau de la pointe de perçage (24) du piston statique (10) pour empêcher le fluide (F) de passer entre ledit au moins un élément d'étanchéité (25', 26', 27') et la surface intérieure (37) et ainsi dans une partie du boîtier (3), et
- faire s'écouler le fluide (F) par l'intermédiaire d'une sortie (7) du dispositif d'écoulement (1), dans lequel le fluide (F) est un fluide médical, en particulier un fluide médical comprenant des nanoparticules lipidiques.
